# EUROPEAN PATENT SPECIFICATION

(11) **EP 3 651 785 B1**
(45) Date of publication and mention of the grant of the patent: **12.11.2025**
(21) Application number: 18832119.4
(22) Date of filing: 11.07.2018
(51) Int. Cl.: A61K 38/00, C07K 14/00, C07K 7/08

(54) **COMPOSITIONS AND METHODS FOR TREATING MYELIN DISORDERS**
ZUSAMMENSETZUNGEN UND VERFAHREN ZUR BEHANDLUNG VON MYELINERKRANKUNGEN
COMPOSITIONS ET MÉTHODES POUR TRAITER LES MALADIES DE LA MYÉLINE

(30) Priority: 11.07.2017 US 201762531251 P
(43) Date of publication of application: 20.05.2020
(73) Proprietor: Case Western Reserve University, Cleveland, OH 44106 (US)
(72) Inventor: SILVER, Jerry, Cleveland, Ohio 44106 (US); LUO, Fucheng, Cleveland, Ohio 44106 (US); YANG, Yan, Cleveland, Ohio 44106 (US)
(74) Representative: Maiwald GmbH
(86) International application number: PCT/US2018/041636
(87) International publication number: WO 2019/014342

(56) References cited:
- US-A1- 2009 281 084
- US-A1- 2015 366 949
- LI HENG ET AL: "Enhanced regeneration and functional recovery after spinal root avulsion by manipulation of the proteoglycan receptor PTP[sigma]", vol. 5, no. 1, 14 October 2015 (2015-10-14), XP055778445, Retrieved from the Internet <URL:http://www.nature.com/articles/srep14923> DOI: 10.1038/srep14923
- MOTAVAF MAHSA ET AL: "Attempts to Overcome Remyelination Failure: Toward Opening New Therapeutic Avenues for Multiple Sclerosis", CELLULAR AND MOLECULAR NEUROBIOLOGY, SPRINGER NEW YORK, US, vol. 37, no. 8, 21 February 2017 (2017-02-21), pages 1335 - 1348, XP036333800, ISSN: 0272-4340, [retrieved on 20170221], DOI: 10.1007/S10571-017-0472-6
- MICHAEL B. KEOUGH ET AL: "Remyelination Therapy for Multiple Sclerosis", NEUROTHERAPEUTICS, vol. 10, no. 1, 16 October 2012 (2012-10-16), US, pages 44 - 54, XP055258981, ISSN: 1933-7213, DOI: 10.1007/s13311-012-0152-7
- LUO FUCHENG ET AL: "Modulation of proteoglycan receptor PTP[sigma] enhances MMP-2 activity to promote recovery from multiple sclerosis", vol. 9, no. 1, 8 October 2018 (2018-10-08), XP055778453, Retrieved from the Internet <URL:http://www.nature.com/articles/s41467-018-06505-6> DOI: 10.1038/s41467-018-06505-6
- NIKNAM PARVIN ET AL: "Modulating proteoglycan receptor PTP[sigma] using intracellular sigma peptide improves remyelination and functional recovery in mice with demyelinated optic chiasm", MOLECULAR AND CELLULAR NEUROSCIENCES, SAN DIEGO, US, vol. 99, 2 July 2019 (2019-07-02), XP085811335, ISSN: 1044-7431, [retrieved on 20190702], DOI: 10.1016/J.MCN.2019.103391
- KIRKHAM ET AL.: "Neural stem cells from protein tyrosine phosphatase sigma knockout mice generate an altered neuronal phenotype in culture", BMC NEUROSCIENCE, vol. 7, no. 1, 19 June 2006 (2006-06-19), pages 50, XP055565020, Retrieved from the Internet <URL:https://doi.org/10.1186/1471-2202-7-50>

## Description

### RELATED APPLICATION

This application claims priority from U.S. Provisional Application Nos. 62/531,251, filed July 11, 2017.

### GOVERNMENT FUNDING

This invention was made with government support under the grant(s) NS077942 awarded by the National Institutes of Health. The United States government has certain rights in the invention.

### BACKGROUND

Multiple Sclerosis (MS) is a chronic autoimmune-mediated demyelinating disease of the central nervous system (CNS) characterized by robust inflammation that leads to dramatic loss of clusters of oligodendrocytes (OLs), demyelination, and irreversible neurologic disability. Although remyelination may spontaneously occur during the early phases of MS, it ultimately fails in regions that develop scar-like, proteoglycan laden plaques. However, the underlying mechanism of failed oligodendrocyte differentiation, maturation, and remyelination are still not well-understood. Recent studies have identified the regulatory effects of chondroitin sulfate proteoglycans (CSPGs) in OPC maturation and function.

CSPGs are structural extracellular matrix (ECM) molecules that consist of chains of sulfated glycosaminoglycans (GAGs) attached to a core protein. Dramatic up-regulation of CSPGs is a hallmark of CNS insult, such as spinal cord injury (SCI), epilepsy, Alzheimer's disease, stroke and MS. The inhibitory impact of CSPGs on axonal regeneration and their role in creating dystrophic endballs by entrapping regenerating axonal endings after spinal cord injury (SCI) has been well documented.

In MS, reactive astroglial-produced CSPGs, such as aggrecan and versican, have been detected in abundance within active demyelinating lesions. While permissive laminins promote the spreading, survival, and maturation of OLs, recent studies have shown that inhibitory CSPGs can out-compete these growth supportive ECM proteins and are able to strongly curtail mouse or human OPCs/OLs by causing a reduction in migration, morphological process extension, and diminished capacity to mature. CSPG inhibition, however, could be relieved by enzymatic degradation through Chondroitinase ABC to enhance OL maturation *in vitro. In vivo,* CSPGs increase temporally in Lysolecithin (LPC)-induced lesions and improved remyelination can likewise occur following CSPG-targeting treatments, such as proteoglycan synthesis inhibitors beta-d-xyloside or flurosamine. Li et al. SCIENTIFIC REORTS, vol. 5, no. 1, 14 October 2015 describes enhanced regeneration and functional recovery after spinal root avulsion by manipulation of the proteoglycan receptor PTPσ. Motavaf et al. CELLULAR AND MOLECULAR NEUROBIOLOGY, Springer New York, US, vol. 37, no.8, 21 February 2017, p. 1335-1348 describes attempts to overcome remyelination failure and aims at opening new therapeutic avenues for multiple sclerosis.

### SUMMARY

Embodiments described herein generally relate to agents for use in methods of enhancing oligodendrocyte progenitor cell (OPC) migration, differentiation, proliferation and/or maturation in subjects having or being at risk of a myelin related disorder.

The therapeutic agent includes a therapeutic peptide. The therapeutic peptide has an amino acid sequence that is at least about 65%, at least about 75%, at least about 85%, or at least about 95% identity to the amino acid sequences of SEQ ID NO:32 or SEQ ID NO: 33. For example, the therapeutic agent can include a therapeutic peptide that has an amino acid sequence selected from the group consisting of SEQ ID NOs: 1-25, 32, and 33.

In embodiments, the therapeutic agent can include a therapeutic peptide that has a sequence identity at least about 65%, at least about 70%, at least about 75%, at least about 80%, at least about 85%, at least about 90%, or at least about 95% homologous to the amino acid sequence of SEQ ID NO: 32. The therapeutic peptide can include, for example, a conservative substitution of an amino acid of at least one, two, three, or four of residue 4, 5, 6, 7, 9, 10, 12, or 13 of SEQ ID NO: 32. In one such embodiment, amino acid residue 4E is substituted with D or Q, the amino acid residue 5R is substituted with H, L, or K, the amino acid residue 6L is substituted with I, V, or M, the amino acid residue 7K is substituted with R or H, the amino acid residue 9N is substituted with E or D, the amino acid residue 10D is substituted with E or N, the amino acid residue 12L is substituted with I, V, or M, and/or the amino acid residue 13K is substituted with R or H.

In other embodiments, the therapeutic agent can include a therapeutic peptide that has a sequence identity at least about 65%, at least about 70%, at least about 75%, at least about 80%, at least about 85%, at least about 90%, or at least about 95% homologous to the amino acid sequence of SEQ ID NO: 33. The therapeutic peptide can include, for example, a conservative substitution of an amino acid of at least one, two, three, or four of residue 7, 8, 9, 10, 12, or 13 of SEQ ID NO: 33. In one such embodiment, amino acid residue 7E is substituted with D or Q, the amino acid residue 8R is substituted with H, L, or K, the amino acid residue 9L is substituted with I, V, or M, the amino acid residue 10K is substituted with R or H, the amino acid residue 12N is substituted with E or D, and/or the amino acid residue 13D is substituted with E or N.

In other embodiments, the therapeutic agent includes a transport moiety that is linked to the therapeutic peptide and facilitates uptake of the therapeutic peptides by the OPC. For example, the transport moiety can be an HIV Tat transport moiety or (PRR5) transport moiety. In one such embodiment, the transport moiety is linked to the therapeutic peptide by a peptide linker. In an embodiment, the therapeutic agent comprises an amino acid sequence selected from the group consisting of SEQ ID NOs:34-60

In some embodiments, the therapeutic agent is formulated for systemic administration to the subject being treated.

In some embodiments, the myelin related disorder comprises at least one of a myelinoclastic disorder, a leukodystrophic disorder, or a demyelinating diseases of the peripheral nervous system. In some embodiments, the myelin related disorder is multiple sclerosis

### BRIEF DESCRIPTION OF THE DRAWINGS

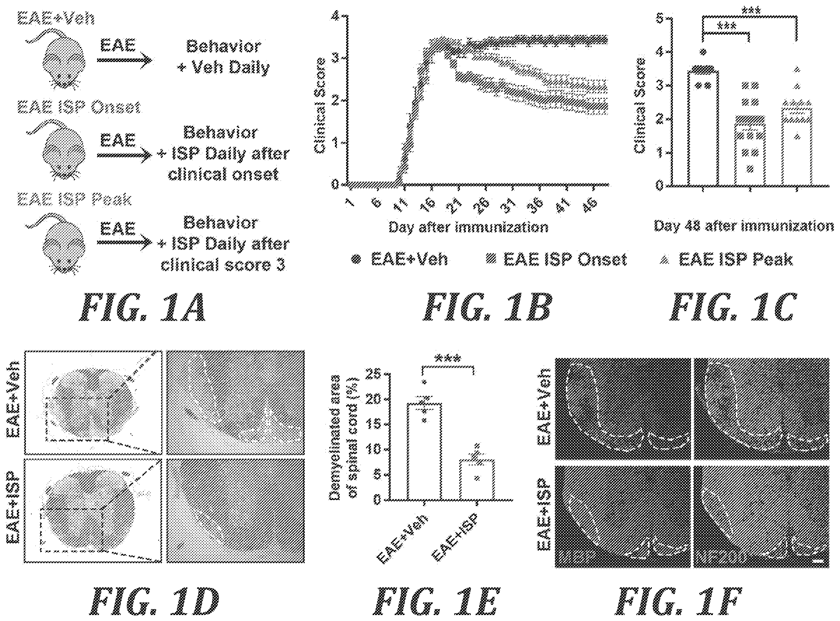

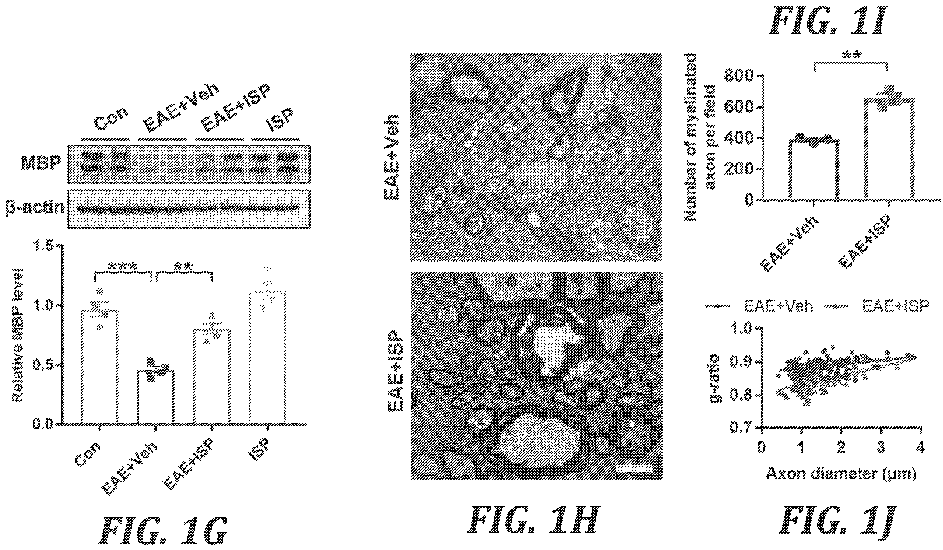

Figs. 1A-1J illustrate ISP promotes functional and histological recovery in EAE mouse model. Fig. 1A: Diagram of ISP administration to EAE mice at the beginning or the peak of sickness determined by clinical score. Fig. 1B: Clinical score of disease severity in MOC_{T35-55}-induced EAE mice treated with ISP or vehicle daily beginning at the onset or the peak of disease. Fig. 1C: Mean improvement in disease score per animal of EAE cohort in B. (n=9 (EAE vehicle group), 15 (EAE ISP onset group) and 12 (EAE ISP peak group), ANOVA F(2,33)=20.96; Tukey's multiple comparison test, *P*_{EAE+Veh versus EAE ISP onset} <0.0001 , *P*_{EAE+Veh versus EAE ISP peak} =0.0004). Figs 1D and 1E: Immunostains and graphs, respectively, showing Luxol fast blue (LFB) staining of myelin, demonstrating normal myelin integrity in ISP-treated EAE mice at 48 days post induction, in contrast to the marked loss of myelin present in the spinal cord of vehicle-treated control. Dashed lines demarcate lesion areas. Scale bar=100 µm. (n=5 mice/group, *P*=0.0002, t=6.647, df=8; Two-tailed unpaired Student's t test) Fig. 1F: Double immunostaining for MBP and neurofilament-200 (NF200) in the thoracic spinal cord of vehicle- and ISP-treated EAE mice at 48 days post induction. Dashed lines demarcate lesion areas. Scale bar=100 µm. Fig. 1G: Western blot analysis of MBP expression in spinal cord tissue of vehicle or ISP-treated control mice and EAE mice at 48 days post-induction. Data are normalized to β -actin protein expression. (n=4 mice/group, ANOVA F(3,12)=26.68; Tukey's multiple comparison test, *P*_{con versus EAE+Veh} = 0.0001, *P*_{EAE+Veh versus EAE+ISP} = 0.0037). Fig. 1H: Electron micrographs from ventral lumbar spinal cords of vehicle and ISP 1070 treated EAE mice 48 days following induction. Scale bar=2 µm. Fig. 1I: Number of myelinated axons in the spinal cord lesions of vehicle and ISP1072 treated EAE mice. (n=3 mice/group, *P*=0.0014, t=7.815, df=4; two-tailed unpaired Student's t test). Fig. 1J: Quantification of the g-ratios (axon diameter/fiber diameter) of myelinated fibers in the ventral lumbar spinal cords of vehicle and ISP-treated EAE mice. (EAE+Veh group, g-ratio= 0.8874 ±0.001901; EAE+ISP group, g1077 ratio=0.8423ISP group; n=134 remyelinated axons from 3 mice/group; *P*<0.0001, 1078 t=14.31, df=266; two-tailed unpaired Student's t test). The data are presented as mean±s.e.m. *P<0.05, **P<0.01, *** p < 0.001.

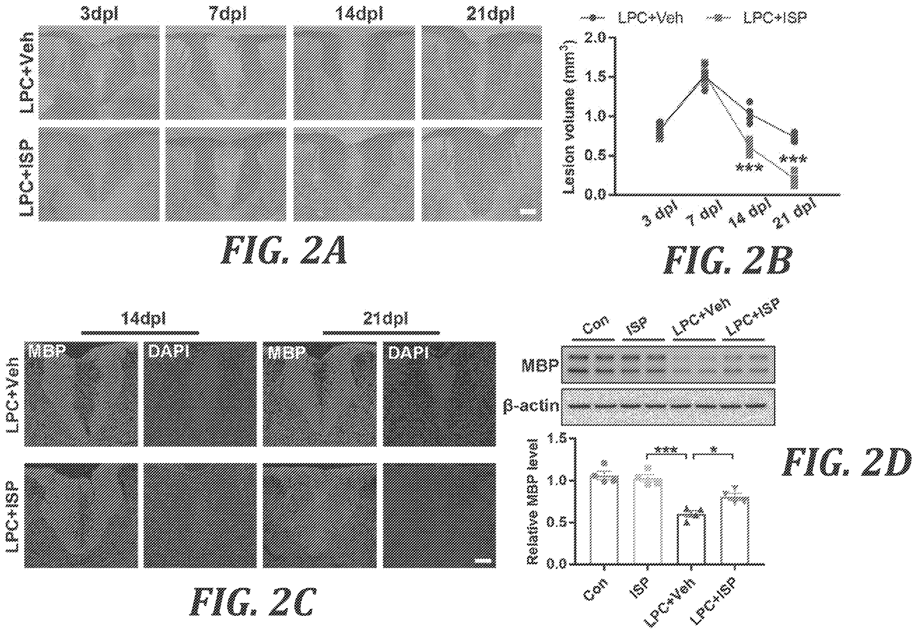

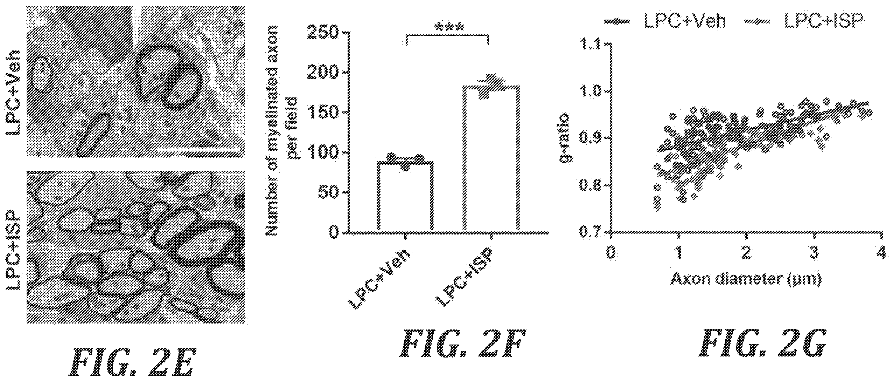

Figs. 2A-2G illustrate that ISP promotes remyelination in the spinal cord of lysolecithin (LPC)-demyelinated mice. Fig. 2A; Representative LFB-stained sections of LPC lesions from the spinal cords of vehicle or ISP treated mice. Dashed lines demarcate lesion areas. Scale bar=100 µm. Fig. 2B; Quantitative analysis of the volume of lesioned spinal cord in vehicle or ISP treated mice at 3, 7, 14 and 21dpl. (n=4 mice/group, ANOVA F(3,12)=16.41; Sidak's multiple comparison test, 14dpl: *P*_{LPC+Veh versus LPC+ISP} = 0.0003; 21dpl: *P*_{LPC+Veh versus LPC+ISP} <0.0001). Fig. 2C: Double immunostaining for MBP and DAPI in the spinal cord of vehicle- and ISP-treated mice at 14 and 21dpl. Dashed lines demarcate lesion areas. Scale bars=100 µm. Fig. 2D: Western blot analysis of MBP expression in spinal cord tissue of vehicle or ISP-treated mice at 14dpl. Data are normalized to β-actin protein expression. (n=4 mice/group, ANOVA F(3,12)=24.21; Tukey's multiple comparison test, *P*_{con versus LPC+Veh} < 0.0001, *P*_{LPC+Veh versus LPC+ISP} = 0.0276). Fig. 2E: Representative electron microscopy images of LPC lesions from the spinal cord of vehicle or ISP-treated mice at 14dpl. Scale bar=5 µm. Fig. 2E: Number of myelinated axons in LPC-induced lesions from vehicle or ISP1100 treated mice at 14dpl. (n=3 mice/group, *P*=0.0001, t=14.26, df=4; two-tailed unpaired Student's t test). Fig. 2G: The myelin g-ratio in the LPC-lesions of vehicle or ISP-treated mice at 14dpl (LPC+veh group, g-ratio= 0.9103 ±0.003583; LPC+ISP group, g-ratio=0.8741± 74103599; n=139 remyelinated axons from 3 mice/group; *P*<0.0001, t=7.142, df=276; two-tailed unpaired Student's t test). The data are presented as mean±s.e.m. *P<0.05, **P<0.01, *** p < 0.001.

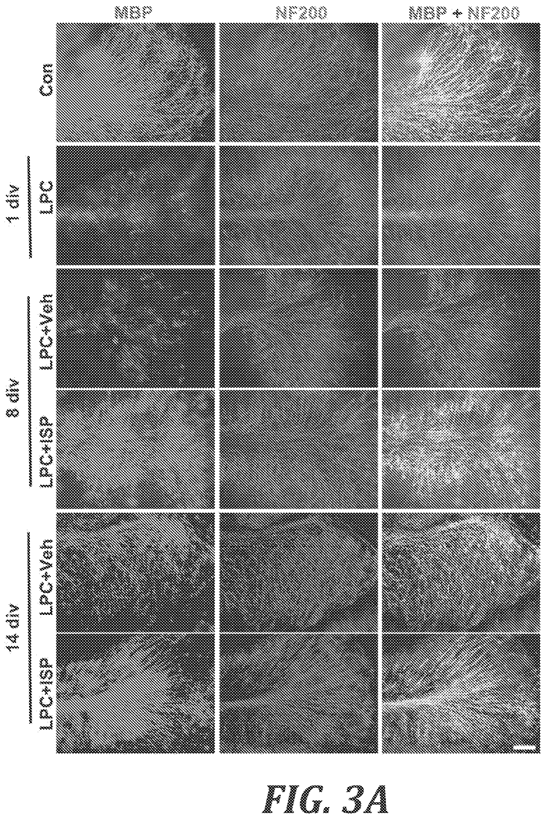

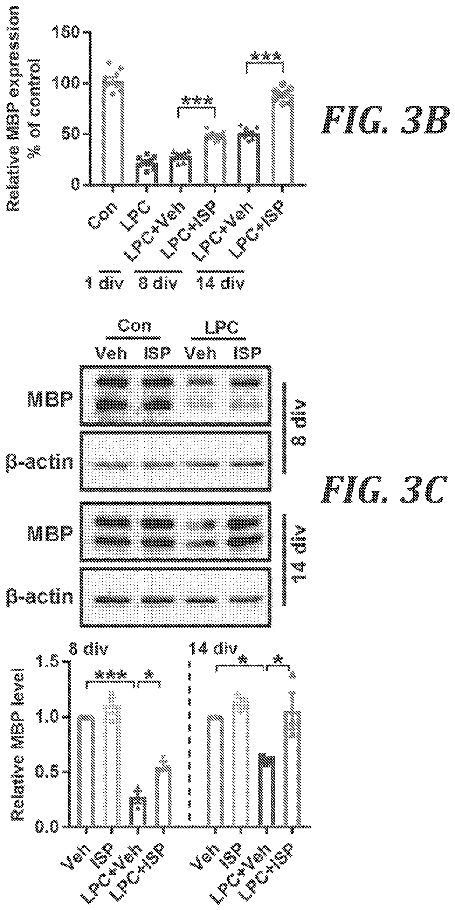

Figs. 3A-3C illustrate that ISP accelerates remyelination in LPC treated organotypic cerebellar cultures. Fig. 3A: Representative immunohistochemistry images of MBP and neurofilament-200 (NF200) show normal myelination in naïve (Con) sections, LPC-induced demyelination at 1 day *in vitro* (div), and increased remyelination after ISP treatment in LPC-demyelinated cerebellar slices at 8div and 14div. Scale bar=100 µm. Fig. 3B: Relative MBP immunoreactivity (i.e. co-localization of MBP and NF200) in cerebellar slices compared to no treatment (100% as control). (n=9 slices from 3 independent replicates per group, ANOVA F(5,48)=230.4, Tukey's multiple comparison test, 8dpl: *P*_{LPC+Veh versus LPC+ISP} < 0.0001, 14dpl: *P*_{LPC+Veh versus LPC+ISP} < 0.0001). Fig. 3C: Western blot analysis of MBP expression in vehicle or ISP-treated cerebellar slices at 8div and 14div. Data are normalized to β-actin protein expression. (n=3 independent replicates per group. 8div: ANOVA F(3,8)=58.89, Tukey's multiple comparison test, *P*_{Veh versus LPC+Veh} < 0.0001, *P*_{LPC+Veh versus LPC+ISP} = 0.0187; 14div: ANOVA F(3,8)=6.281, Tukey's multiple comparison test, *P*_{Veh versus LPC+Veh} < 0.048, *P*_{LPC+Veh versus LPC-ISP} = 0.025). The data are presented as mean±s.e.m. *P<0.05, **P<0.01, *** p < 0.001.

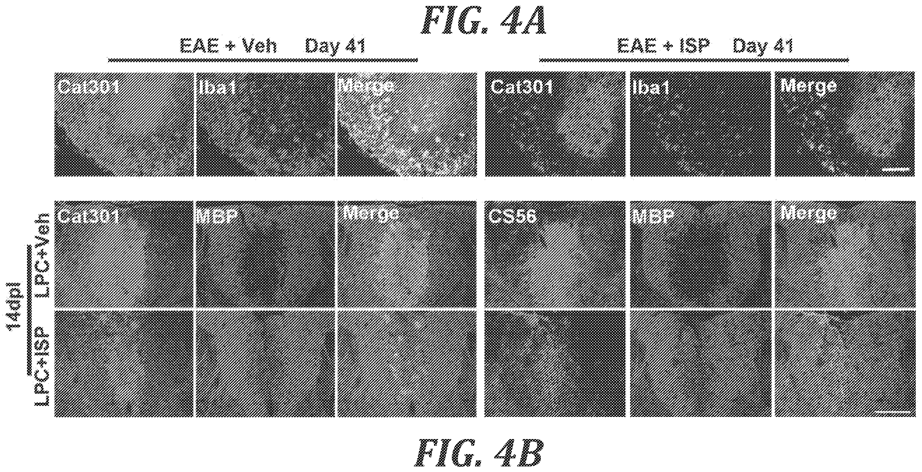

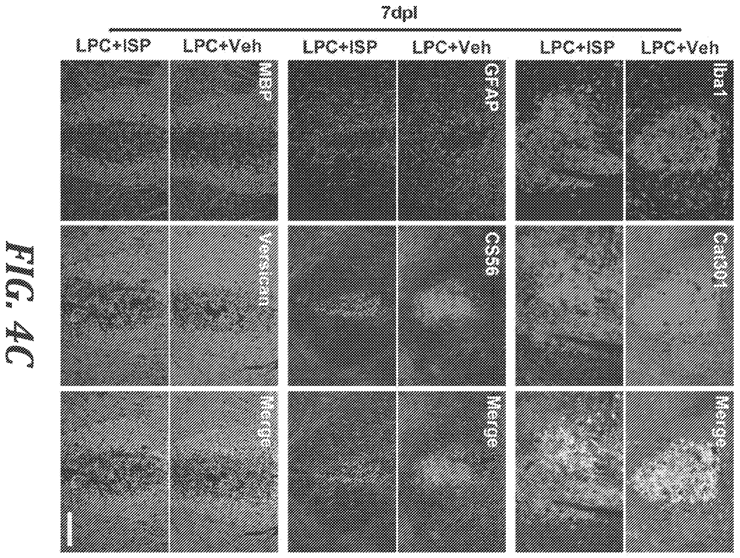

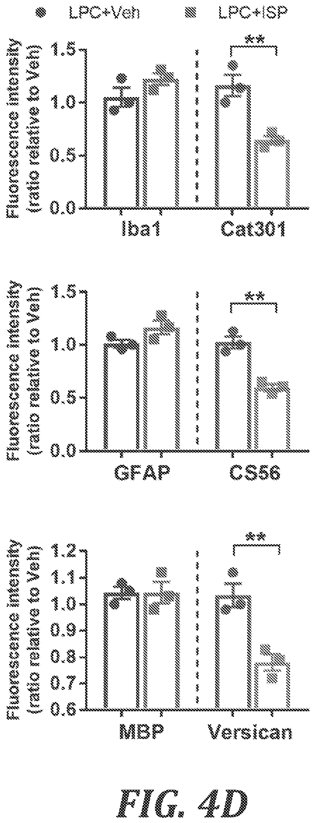

Figs. 4A-4D illustrate ISP decreases chondroitin sulfate proteoglycan (CSPG) load in both EAE and LPC models. Fig 4A: Representative immunohistochemistry images of Iba1 and Cat301 (aggrecan specific antibody) show decreased accumulation of CSPG and microglia/macrophages in the thoracic spinal cord of ISP-treated compared to vehicle treated mice EAE mice at 41 days post-induction. Scale bar=100 µm. Fig 4B: Representative immunohistochemistry images of MBP, Cat301 and CS56 (glycosaminoglycan specific antibody) showing decreased accumulation of CSPG after ISP treatment at 14dpl in LPC demyelination mice. Scale bar=100 µm. Fig 4C: Representative immunohistochemistry images of Iba1, GFAP, MBP, Cat301 and CS56 showing decreased accumulation of CSPG after ISP treatment at 7dpl in LPC demyelination mice. Scale bar=100 µm. Fig 4D: Relative quantification of immunofluorescence intensity of Iba1, GFAP, MBP, Cat301 and CS56 in the spinal cord of vehicle or ISP-treated LPC mice at 7dpl. (n=3 mice/group, Iba1: *P*=0.1848, t=1.6, df=4; Cat301: *P*=0.0092, t=4.719, df=4; GFAP: *P*=0.1111, t=2.039, df=4; CS56: *P*=0.0028, t=6.55, df=4; MBP: *P*>0.9999, t=0, df=4; Versican: *P*=0.0095, t=4.669, df=4; two-tailed unpaired Student's t test). The data are presented as mean±s.e.m. **P<0.01.

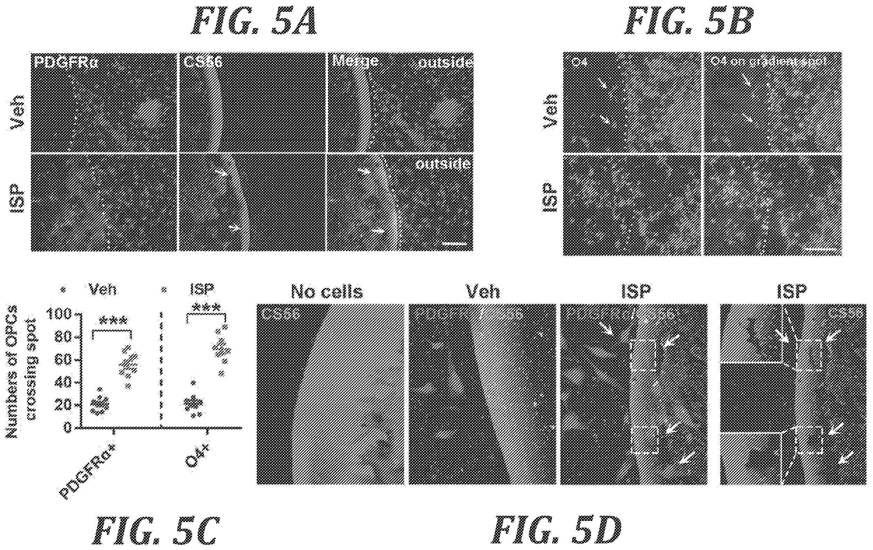

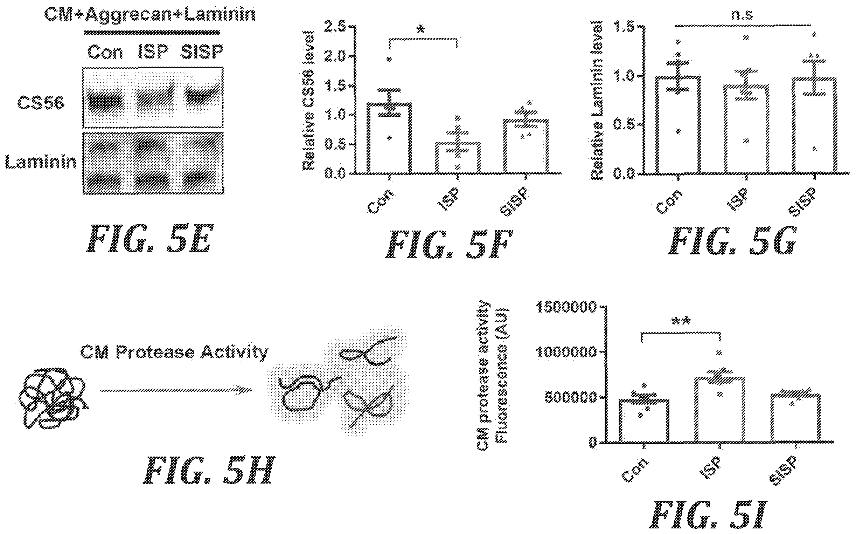

Figs. 5A-5I illustrate that ISP increases CSPG-degrading protease activity. Figs. 5A and 5B: CSPG gradient crossing assay shows that ISP treatment promotes the crossing of PDGFRα+ or O4+OPCs through the gradient of CSPG. Scale bar=100 µm. Fig. 5C: Quantification of immunostaining for the amount of PDGFRα+ or O4+ OPCs crossing the CSPG barrier after vehicle or ISP treatment. (n=9 spots from 3 independent replicates. PDGFRα: *P*<0.0001, t=7.99, df=16; O4: *P*<0.0001, t=9.419, df=16, two-tailed unpaired Student's t test). The data are presented as mean±s.e.m. Fig. 5D: Representative immunostain images of CS56 and PDGFRα+ OPCs on CSPG barrier depicting CSPG degradation after ISP treatment as they cross the barrier to leave CS56 "shadows" (inset and arrows). Fig. 5E: To investigate protease activity, OPC conditioned media (CM) was treated with vehicle control or 2.5uM ISP or scrambled-ISP (SISP) and incubated with aggrecan (20ug/mL) or laminin (10ug/mL), then analyzed through western blots. Fig. 5F: Quantification of glycosaminoglycan moiety through CS56 immunoblotting reveals significant CS56 degradation following ISP treatment (One-Way ANOVA, Dunnett's posthoc test, P=0.0432, F(2,12)=4.131, N=5 western blots. Fig. 5G: Quantification of laminin immuoblotting shows no significant changes (One- Way ANOVA, Tukey's posthoc test, P=0.9024, F(2, 15)=0.1034), N=6 western blots). Fig. 5H: Quenched casein in EnzChek protease activity assay fluoresces once it becomes cleaved. Fig. 5I: Quantification of EnzChek protease activity assay reveals significant protease activity (One-Way ANOVA Dunnett's posthoc test, P=0.0015, F(2,18)=9.534, N=26 from 7 replicates) in OPC CM treated with 2.5 µM ISP over control. Graphs indicate scatterplot representations of western blots or mean of each replicate with standard error means. *P<0.05, **P<0.01, *** p < 0.001. n.s., not significant.

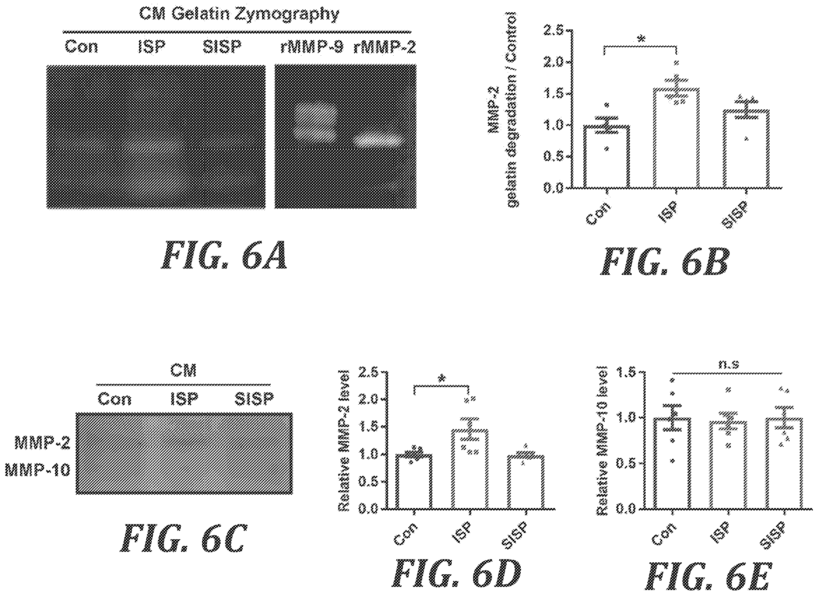

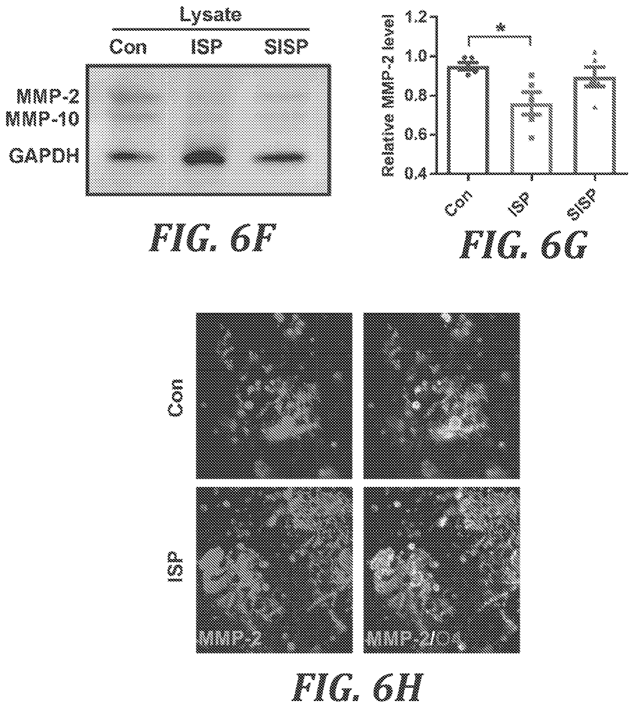

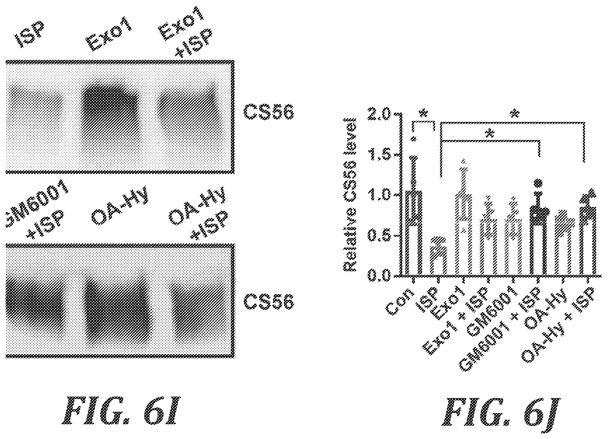

Figs. 6A-6I illustrate that ISP increases MMP-2 secretion and activity to further characterize protease activity, in Fig. 6A cultured OPCs were treated with vehicle control or 2.5 µM ISP or SISP, concentrated, then loaded onto gelatin SDS/PAGE gels for zymography analysis. 25 ng of recombinant MMP-9 or MMP-2 served as positive controls. Fig. 6B: Quantification of active MMP-2 lanes of gelatin zymography reveals significant MMP-2 activity following ISP treatment over control (One-Way ANOVA, Dunnett's posthoc test, P=0.0161, F(2,12)=5.937, N=5 zymograms). Fig. 6C: Enhanced MMP-2 expression following ISP treatment was confirmed in western blots of concentrated OPC conditioned media (CM). Fig. 6D: Quantification of MMP-2 immoblotting was significantly enhanced following ISP treatment over control (One-Way ANOVA, Dunnett's posthoc test, P=0.0150, F(2,15)=5.63, N=6 western blots). In contrast, in Fig. 6E MMP-10 immunoblotting was not significant among treatments (One-Way ANOVA, Tukey's posthoc test, P=0.9619, F(2,15)=0.03899, N=6 western blots). Fig 6I: to explore whether ISP induces secretion of proteases to degrade CSPGs, cultured OPCs were treated with the following drugs with or without 2.5uM ISP: exocytosis inhibitor Exo1 10ug/mL), general metalloprotease inhibitor GM6001 (25uM), or specific MMP-2 inhibitor (OA-Hy, Calbiochem, 100nM). Collected CM was incubated with aggrecan (20ug/mL) and immunoblotted with CS56. Fig. 6J: Quantification of CS56 reveals significant ISP-induced degradation of CSPGs over control, Exo1+ISP, GM6001+ISP, and OA-Hy + ISP (One-Way ANOVA, Tukey's posthoc test, P=0.0010, F(7,32)=4.749 N=5 western blots). Fig. 6H; Immunostaining of O4+ (red) OPCs showing MMP-2 concentrated in OPC soma and processes. Graphs indicate scatterplot representations of western blots or mean of each replicate with standard error means. *P<0.05. n.s., not significant.

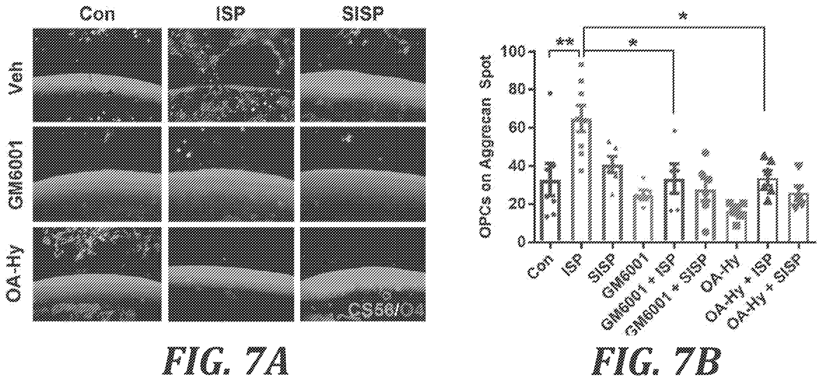

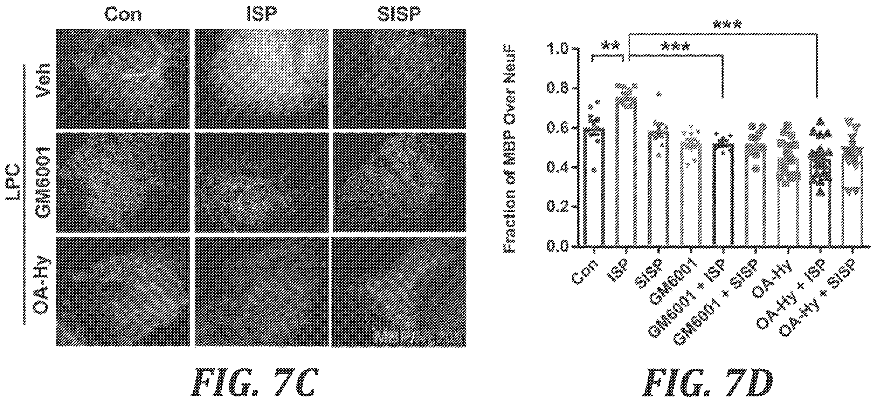

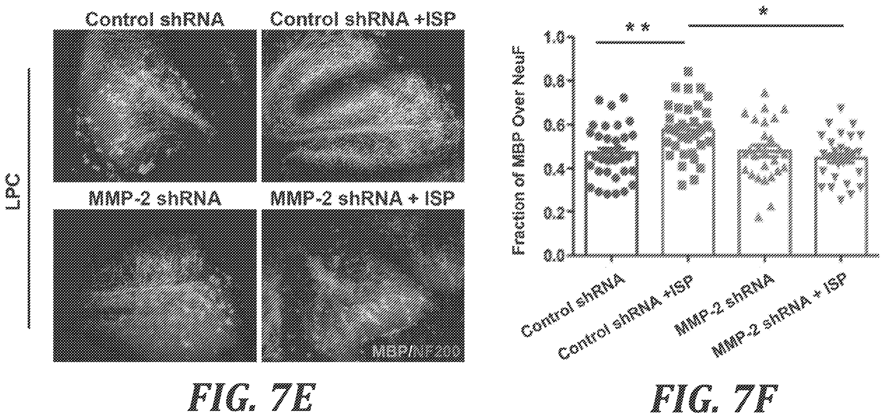

Figs. 7A-7F illustrates that ISP-induced MMP-2 activity increases OPC migration and remyelination through CSPG disinhibition. To ask whether ISP-induced protease activity is involved in OPC migration and remyelination, in Fig. 7A cultured OPCs were plated onto coverslips with CSPG spot gradients and treated with vehicle control, 2.5 µM ISP or SISP, 25uM GM6001 +/- ISP or SISP, or 100 nM MMP-2 inhibitor (OA-Hy) +/- ISP or SISP. The amount of O4+ OPCs crossing the CSPG barrier was counted and, in Fig. 7B, quantified and displayed graphically. ISP treatment (N=37 spots, 5 replicates) significantly induced greater O4₊OPC migration past the CSPG barrier compared to control (One-Way ANOVA, Tukey's posthoc test, P=0.0002, F(11,48)=5.013), GM6001+ISP (N=20 spots), or OA-Hy + ISP (N=20 spots) treatments. N(Spots)=31 Control, 26 SISP, 18 GM6001, 20 GM6001+SISP, 18 OA-Hy, 19 OA-Hy+SISP) Fig. 7C: To test whether remyelination was affected by protease inhibition, P7-9 cerebellar slices were all treated with LPC for 18 hours, then treated with control vehicle, 2.5 µM ISP or SISP, 25uM GM6001+/- ISP, or 100nM OA-Hy +/- ISP for 9 days before staining for neurofilament (NF200) or MBP. Fig. 7D: Remyelination was quantified through MBP and neurofilament colocalization. ISP treatment (N=35 images from 4 replicates with up to 13 sections total) significantly increased MBP-neurofilament colocalization over control (One-Way ANOVA, Tukey's posthoc test, P=0.0001, F (8, 88) = 13.61), GM6001 + ISP (N=28), and OA-Hy + ISP (N=23) groups. N(images)=17 Control, 22 SISP, 48 GM6001, 20 GM6001+SISP, 22 OA-Hy, 22 OA-Hy +SISP Graphs indicate scatterplot representations of mean of each replicate with standard error means. *P<0.05, **P<0.01, *** p < 0.001. Figs. 7E and 7F: Cerebellar slice cultures were treated with lentiviral constructs for 48 hours before LPC treatment. Vehicle or ISP (2.5 µM) treatment followed for 6 days *in vitro.* MBP immunofluorescence (green) was then quantified with NF200 (red). One-Way ANOVA, Tukey's posthoc test. P=0.0005. F(3, 116)=6.395. N=around 30 images from 10 slices). Graphs indicate scatterplot representations of mean of each replicate with standard error means.

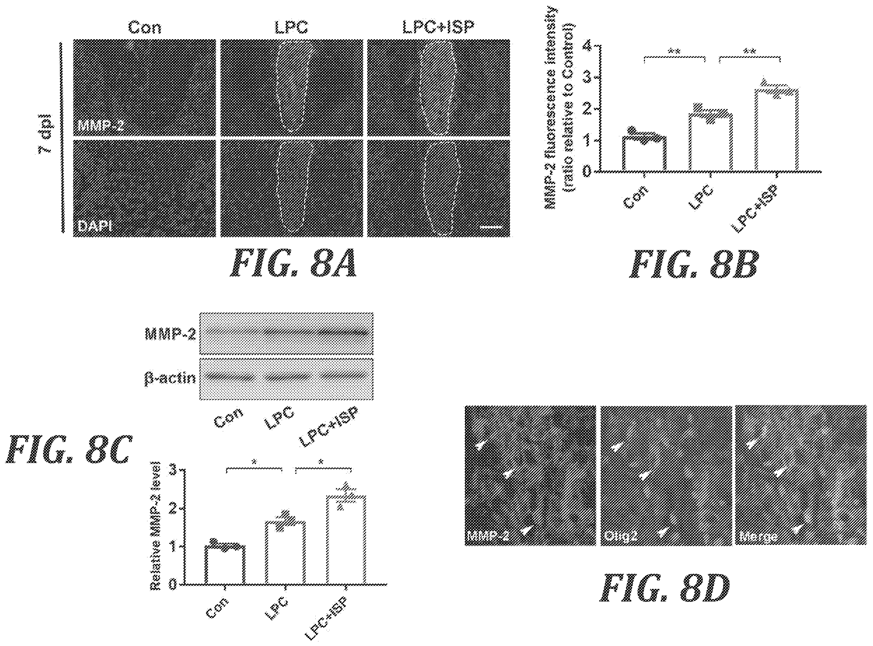

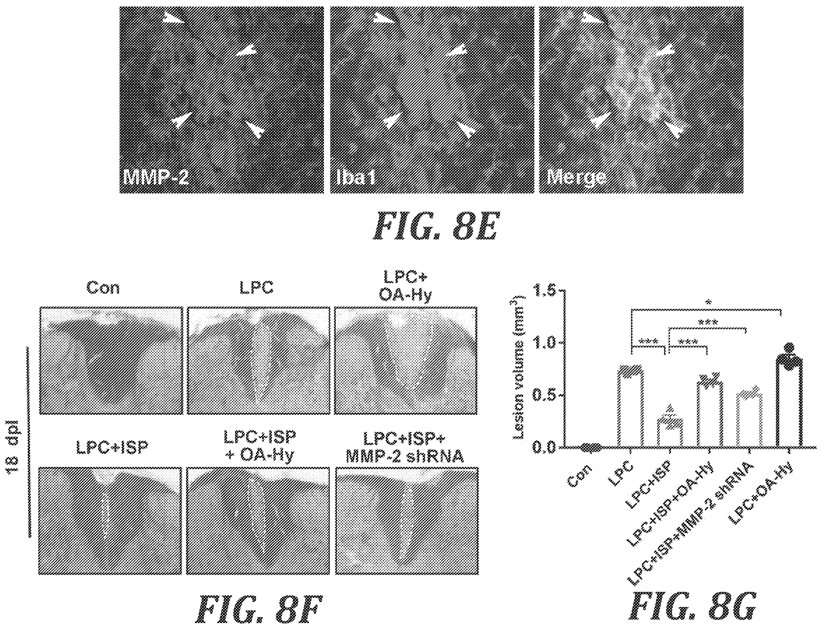

Figs. 8A-8F illustrate THAT ISP promotes myelin repair through increasing MMP-2 expression in LPC-induced demyelination model of mice. Fig. 8A: Representative images from immunohistochemistry of MMP-2 and DAPI show increased levels of MMP-2 in the spinal cord of ISP-treated mice at 7 days post LPC injection (dpl) compared to naive or LPC vehicle control cords. Dashed lines demarcate lesion areas. Scare bar=100 µm. Fig. 8B: Relative quantification of immunofluorescence intensity of MMP-2 in the spinal cord of ISP-treated mice at 7dpl (n=3 mice/group, ANOVA F(2,6)=48.12, Tukey's multiple comparison test, *P*_{con versus LPC} = 0.0075, *P*_{LPC versus LPC+ISP} = 0.0056). Fig. 8C: Western blot analysis of MMP-2 expression in spinal cord tissue of naïve, vehicle, or ISP-treated mice at 7dpl. Data normalized to β -actin protein expression. (n=3 mice/group, ANOVA F(2,6)=33.14; Tukey's multiple comparison test, *P*_{con versus LPC} = 0.0168, *P*_{LPC versus LPC+ISP} = 0.0143). Fig. 8D: Representative immunohistochemistry images show Olig2+ OPCs express MMP-2 in the spinal cord of ISP-treated mice at 14dpl. White arrows indicate the colocalization of MMP-2 and Olig2. Fig. 8E: Representative immunohistochemistry images show Iba1+cells (microglia/macrophage) express MMP-2 in the spinal cord of ISP-treated mice at 14dpl. White arrows indicate the colocalization of MMP-2 and Iba1. Scare bar=100 µm. Fig. 8F: Representative eriochrome cyanine (myelin) staining of LPC lesions from the spinal cords of naïve, vehicle, ISP, MMP-2 inhibitor (OA-Hy), or MMP-2 shRNA treated mice. Dashed lines demarcate lesion areas. Scale bar=100 µm. Fig. 8G: Quantitative analysis of the volume of lesioned spinal cord in vehicle, ISP, MMP-2 inhibitor (OA-Hy), or MMP-2 shRNA treated mice at 18dpl. (n=4 mice/group, ANOVA F(5,18)=169.7; Tukey's multiple comparison test, *P*_{LPC versus LPC+ISP} < 0.0001; *P*_{LPC+ISP} versus _{LPC+ISP+OA-Hy} <0.0001; *P*_{LPC+ISP} versus LPC+ISP+MMP-2 shRNA <0.0001; *P*_{LPC} versus _{LPC+OA-Hy} =0.0279). The data are presented as mean±s.e.m. *P<0.05, *** p < 0.001.

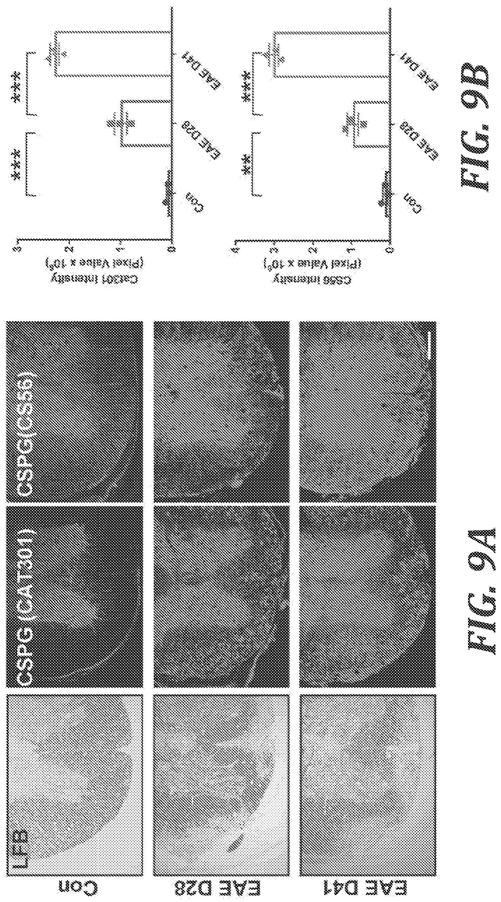

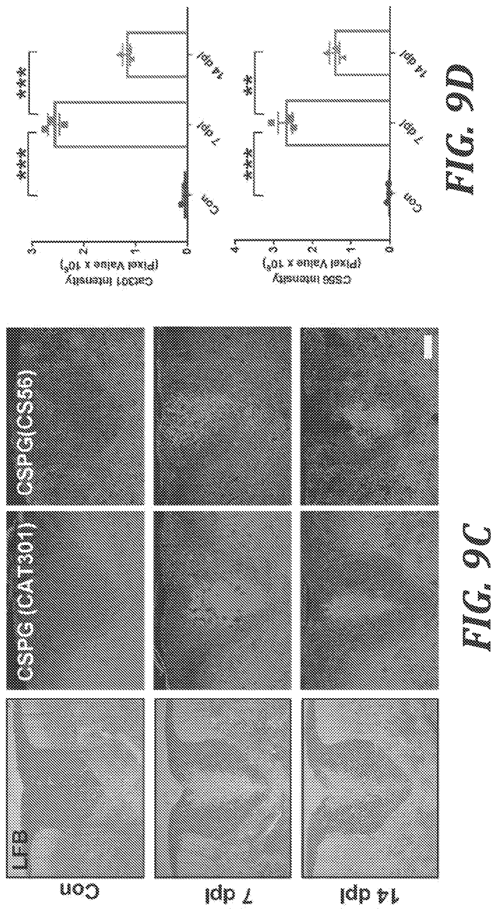

Figs. 9A-9D illustrate THAT increased CSPG load in mouse models of MS. Figs. 9A and 9B: Representative LFB-stained sections and immunohistochemistry images of Cat301 and CS56 show CSPG accumulation in the thoracic spinal cord of EAE mice at 28 and 48 days post-induction. Scale bar=100 µm. Quantification of pixel intensities of Cat301 (aggrecan CSPG) and CS56 (glycosaminoglycan moieties of CSPGs) depicted. (Cat301: n=3 mice/group, ANOVA F(2,6)=163.9, Tukey's multiple comparison test, *P*_{con versus EAE D28} =0.0007, *P*_{EAE D28} versus _{EAE D41} = 0.0001; CS56: n=3 mice/group, ANOVA F(2,6)=168.7, Tukey's multiple comparison test, *P*_{con} versus EAE D28 =0.0052, *P*_{EAE D28 versus EAE D41 <} 0.0001). Figs. 9C and 9D: Representative LFB-stained sections and immunohistochemistry images of Cat301 and CS56 show CSPG accumulation in the lesion site after LPC demyelination at 7dpl and 14dpl. Scale bar=100 µm. Quantification of pixel intensities of Cat301 and CS56 depicted (Cat301: n=3 mice/group, ANOVA F(2,6)=269.7, Tukey's multiple comparison test, *P*_{con versus 7 dp1}< 0.0001, *P*_{7dpl versus 14 dpl} < 0.0001; CS56: n=3 mice/group, ANOVA F(2,6)=105, Tukey's multiple comparison test, *P*_{con versus 7} dpl < 0.0001, *P*_{7dpl versus 14 dpl} = 0.0011).

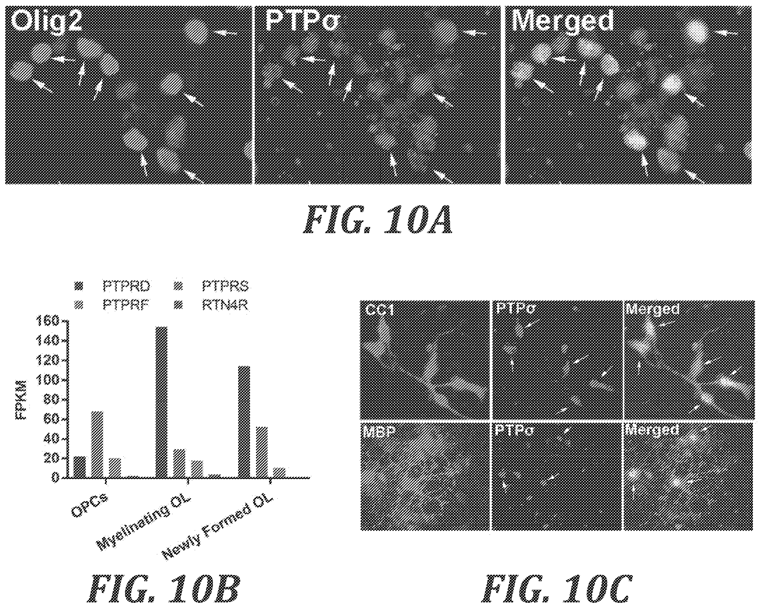

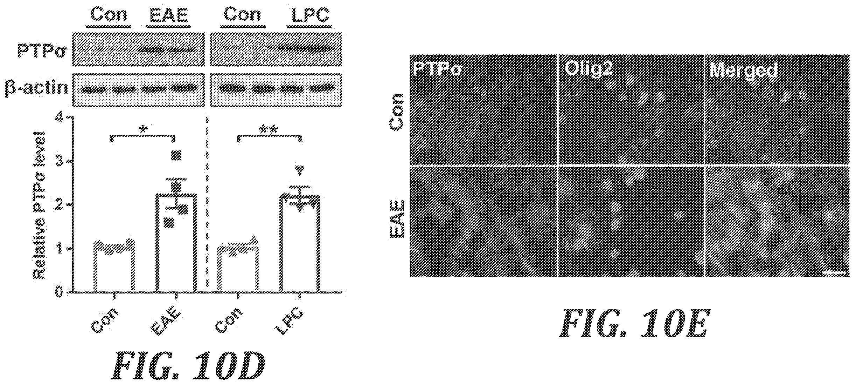

Figs. 10A-10E illustrate that PTPσ expression is enhanced following EAE and LPC. Fig. 10A: Representative immunocytochemistry images of PTPσ and Olig2 show PTPσ expression on oligodendrocyte progenitor cells grown *in vitro.* Fig. 10B: Graphical representation of cell-specific PTPRD (PTPδ), PTPRS (PTPα), PTPRF (LAR) and RTN4R (Nogo) mRNA levels obtained from a publicly available RNA-sequencing transcriptome database (web .stanford.edu/group/barres lab/). FPKM represents fragments per kilobase of transcript sequence per million mapped fragments. Fig. 10C: Representative immunohistochemistry images of PTPσ, CC1, and MBP show PTPα expression in CC1₊ or MBP+ cells in OPCs culture *in vitro.* Fig. 10D: Western blot analysis of PTPσ expression in the thoracic spinal cord of EAE or LPC-induced demyelination mice. Data are normalized to β-actin protein expression (n=4 mice per group. *P*_{con versus EAE}=0.012, t=3.558, df=6; *P*_{con versus LPC}=0.0012, t=5.775, df=6; two-tailed unpaired Student's t test). Fig. 10E: Representative immunohistochemistry images of PTPσ and Olig2 show increased expression of PTPσ in Olig2+ cells in the spinal cord of EAE mice at 28 days post-induction. Scale bar=50 µm. The data are presented as mean±s.e.m. *P<0.05, **P<0.01.

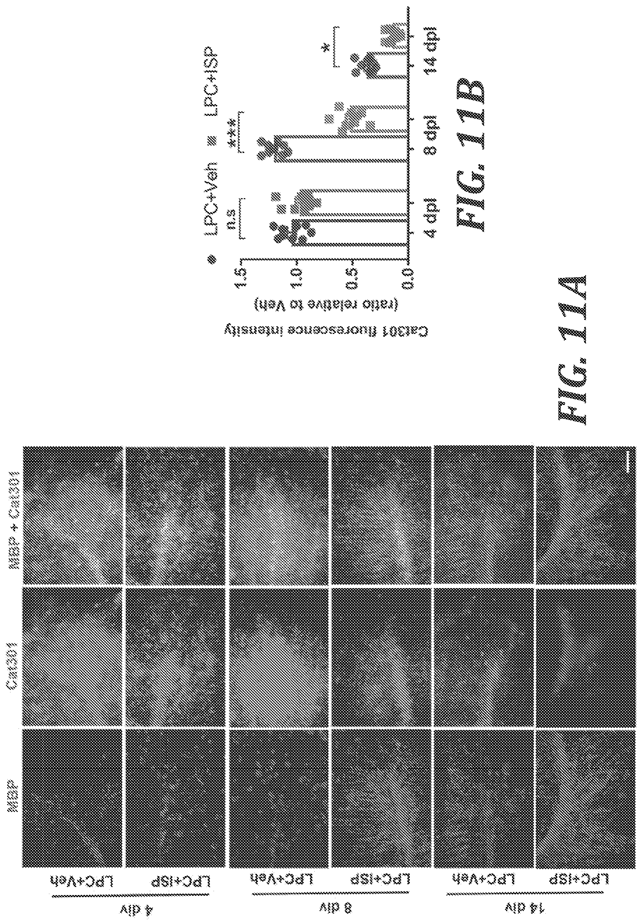

Figs. 11Aand 11B illustrate ISP increases CSPG clearing as remyelination occurs. Fig. 11A: Representative immunohistochemistry images of MBP and Cat301 show reduced abundance of CSPG after ISP treatment in LPC-demyelinated cerebellar slices at 8dpl and 14dpl. Scale bar=100 µm. Fig. 11B: Relative quantification of immunofluorescence intensity of Cat301 after Vehicle or ISP treatment in LPC demyelinated cerebellar slices at 4dpl, 8dpl and 14dpl (n=9 slices from 3 independent replicates per group, two-way ANOVA F(2,6)=30.78, Sidak's multiple comparison test, 8dpl: *P*_{LPC+Veh versus LPC+ISP} < 0.0001, 14dpl: *P*_{LPC+Veh versus LPC+ISP} = 0.0325). ). *P<0.05, *** p < 0.001, n.s: no significance.

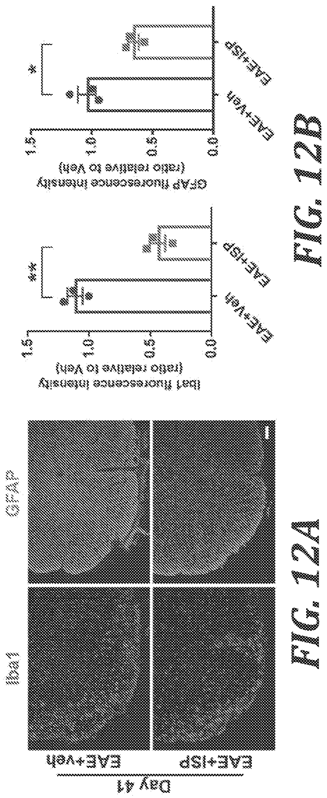

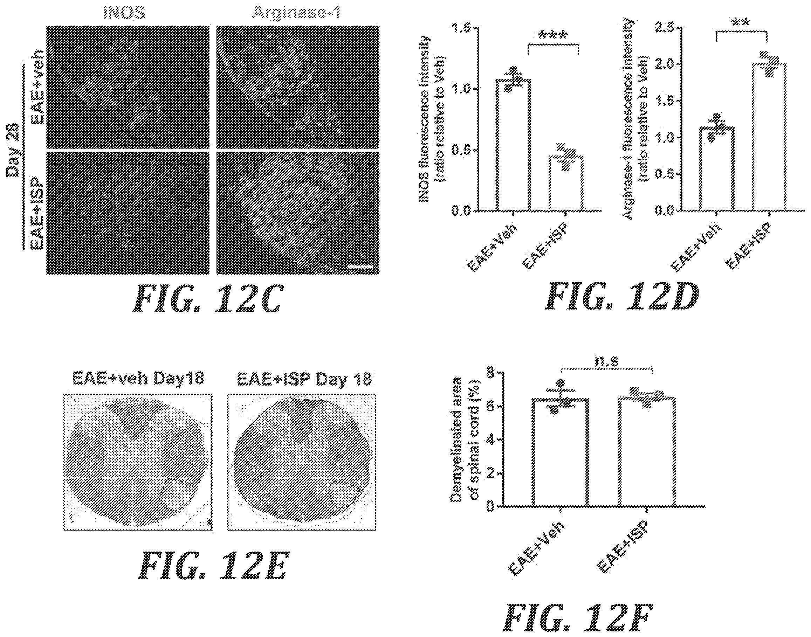

Figs. 12A-12F illustrate that ISP modulates inflammation in EAE models of mice. Fig. 12A: Representative immunohistochemistry images of Iba1 and GFAP show decreased activation of microglia and astrocytes respectively in the spinal cord of ISP-treated mice at day 41 following EAE induction. Scale bar= 100 µm. Fig. 12B: Relative quantification of immunofluorescence intensity of Iba1 and GFAP in the spinal cord of ISP-treated mice at day 41 (n= 3 mice/group, Iba1: *P*=0.0016, t=7.608, df=4; GFAP: *P*=0.0113, t=4.448, df=4. two-tailed unpaired Student's t test). Fig. 12C: Representative immunohistochemistry images of iNOS (M1 microglia marker) and Arginase-1 (M2 microglia marker) show increased M2 microglia and decreased M1 microglia in the spinal cord of ISP-treated mice at day 28. Scale bar= 100 µm. Fig. 12D: Quantification of relative immunofluorescence of iNOS and Arginase-1 in the spinal cord of ISP-treated mice at day 41 (n= 3 mice/group, NOS: *P*=0.0008, t=9.114, df=4; Arginase-1: *P*=0.0014, t=7.824, df=4. two-tailed unpaired Student's t test). Fig. 12E: Representative Luxol fast blue staining images show no differences in demyelination area of Vehicle or ISP-treated mice at day 18. Fig. 12F: Quantification of demyelination area in the spinal cord of Vehicle or ISP treated mice at day 18 indicates similar levels of demyelination in EAE model (n=3 mice/group, *P*=0.8559, t=0.1936, df=4, two-tailed unpaired Student's t test). *P<0.05, *** p < 0.001, n.s: no significance.

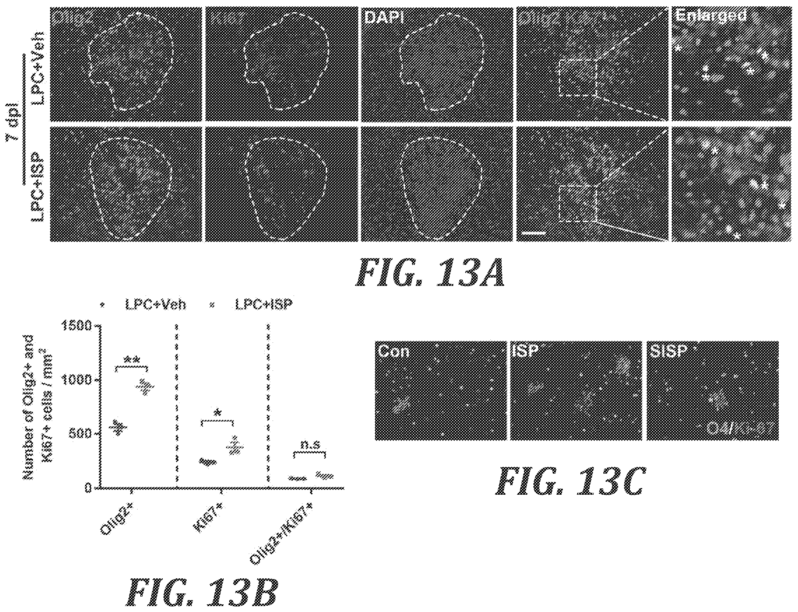

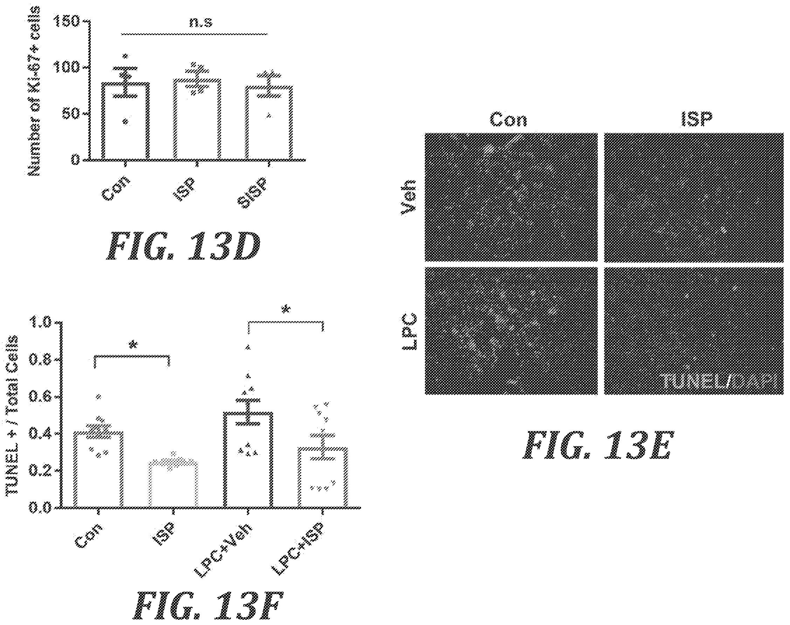

Figs. 13A-13F illustrate that ISP promotes OPC recruitment and survival on CSPGs. Fig. 13A: Representative images of Ki67 immunostaining showing proliferating OPCs (Olig2+) in the spinal cord of vehicle- and ISP-treated mice at 7dpl. White arrows show Olig2+/Ki67+cells. Scale bar=100 µm. Fig. 13B: Quantification of immunostaining showing Olig2+ cells/mm₂, Ki67+ cells/mm₂ and Olig2+/Ki67+ cells/mm₂ in the spinal cord of vehicle- and ISP-treated mice at 7dpl. (n=3 mice/group, Olig2: *P*=0.0012, t=1.6, df=4; Ki67: *P*=0.0449, t=2.883, df=4; Olig2+/Ki67+: *P*=0.056, t=2.666, df=4. two-tailed unpaired Student's t test). The data are presented as mean±s.e.m. Fig. 13C: Animals treated with vehicle or ISP for 7div following spinal cord LPC injections. Sections were stained with Olig2, Ki67, and DAPI B) Quantification of Olig2, Ki67, and colocalized Olig2 and Ki67 in LPC sections. C) OPCs were cultured on aggrecan/laminin-precoated coverslips, treated with vehicle control, 2.5 µM ISP or SISP, and stained with O4 and Ki67. Fig. 13D: Ki67 was not significantly changed among the groups (One-Way ANOVA, Tukey's posthoc test, P=0.8998, F(2,9)=0.1068, N=12 images each with 4 replicates and 3 repeats each). Fig. 13E: OPCs were cultured on aggrecan/laminin and treated with control or 2.5 µMISP for 2div before incubation with vehicle or LPC (1 µg/mL) for 2 hours before DAPI and TUNEL staining. Fig. 13F: Quantification of TUNEL+ over DAPI+ cells reveal significant survival of ISP-treated (N=40 images, 4 replicates) OPCs over control (One-Way ANOVA, Tukey's posthoc test, P=0.0021, F(3,36)=5.96). ISP-treatment enhanced survival of LPC-treated cells. (N(images)=36 control, 28 each LPC-treated group). Graphs indicate scatterplot representations of mean of each replicate with standard error means. *P<0.05, **P<0.01, *** p < 0.001.

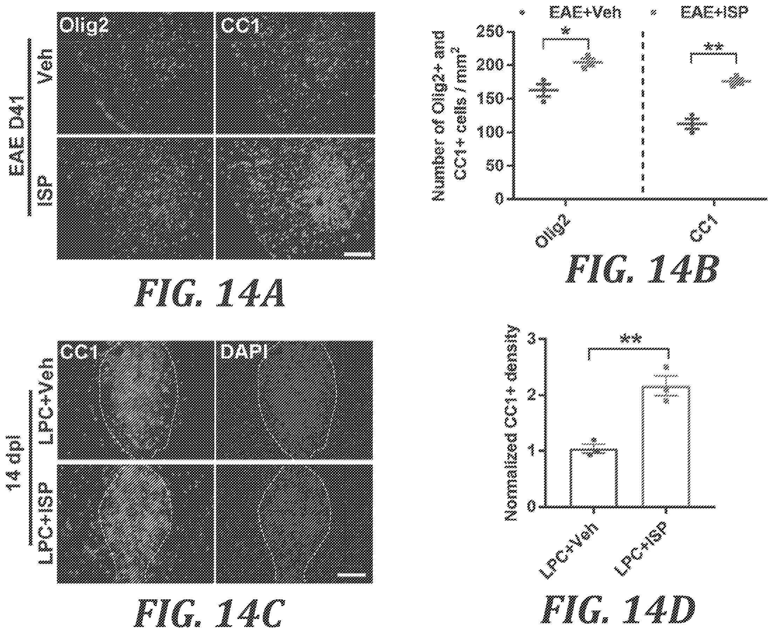

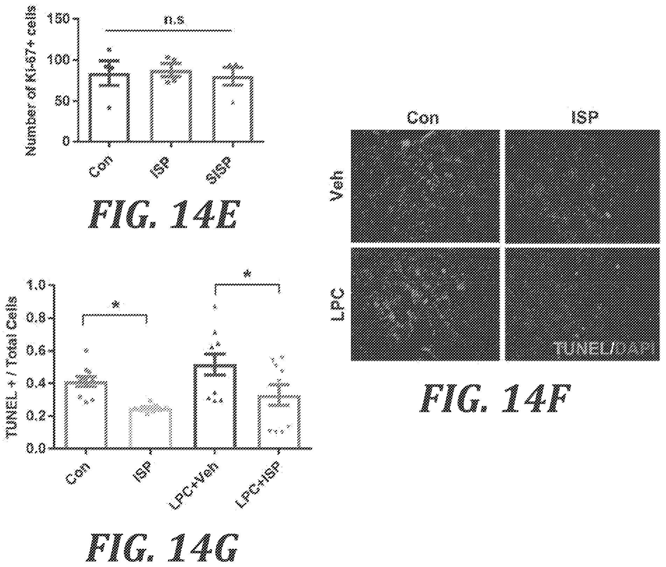

Figs. 14A-14G that ISP enhances OPC process outgrowth and maturation. Fig. 14A: Representative immunohistochemistry images of Olig2 and CC1 in the thoracic spinal cord of vehicle or ISP-treated EAE mice at 41 days post-induction. Scale bar=50 µm. Fig. 14b: Quantification of immunostaining for Olig2+ cells/mm₂ and CC1+ cells/mm₂ in the thoracic spinal cord of vehicle or ISP-treated EAE mice at 41 days post induction. (n=3 mice/group, Olig2: *P*=0.0189, t=3.811, df=4; CC1: *P*=0.0019, t=7.259, df=4. two-tailed unpaired Student's t test). Fig. 14c: Representative immunohistochemical images of DAPI and CC1 in the spinal cord of vehicle or ISP-treated LPC mice at 14dpl. Dashed lines demarcate lesion areas. Scale bar=100 µm. Fig. 14d: Quantification of immunostaining for normalized CC1+oligodendrocytes density at 14dpl. (n=3 mice/group, *P*=0.0044, t=5.789, df=4. two-tailed unpaired Student's t test). Fig. 14e: Representative immunohistochemical images of the maturation of OPCs after plating onto poly-l-lysine or CSPGs in the presence of ISP or vehicle. Scale bar=100 µm. Fig. 14f: Quantification of the relative proportion of maturing OLs after OPCs plating onto poly-l-lysine (control) or CSPGs in the presence of ISP or vehicle. (n= 3 independent replicates, O4: ANOVA F(2,6)=1.321, P=0.3347; MBP: ANOVA F(2,6)=30.99, Tukey's multiple comparison test, *P*_{Con versus CSPGs+Veh} = 0.0005, *P*_{CSPGs+Veh versus CSPG+ISP} =0.0175). Fig. 14g: Comparison of the size of MBP+footprints after OPCs plating onto poly-l lysine (control) or CSPGs in the presence of ISP or vehicle at 6 days. (n= 3 independent replicates, ANOVA F(2,6)=40.96, Tukey's multiple comparison test, *P*_{con versus CSPGs+Veh} = 0.0003, *P*_{CSPGs+Veh versus CSPGs+ISP} =0.0052). The data are presented as mean±s.e.m. *P<0.05, **P<0.01, *** p < 0.001. n.s., not significant.

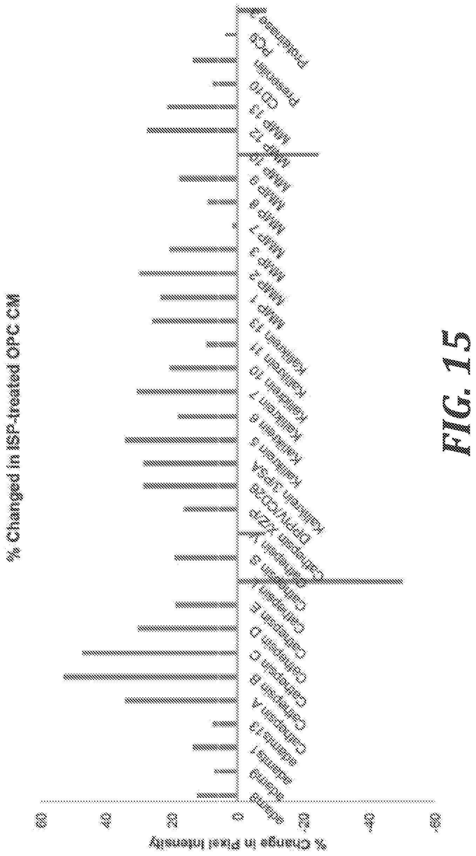

Fig. 15 graphically illustrates results of a protease assay. To begin screening which proteases may be upregulated by ISP treatment, cultured OPCs were treated with vehicle control or 2.5 µM ISP for 4 days *in vitro.* Conditioned media was then incubated with qualitative protease array (R & D Systems) and developed. % Change in pixel intensities of ISP-treated vs. control OPC CM was then calculated.

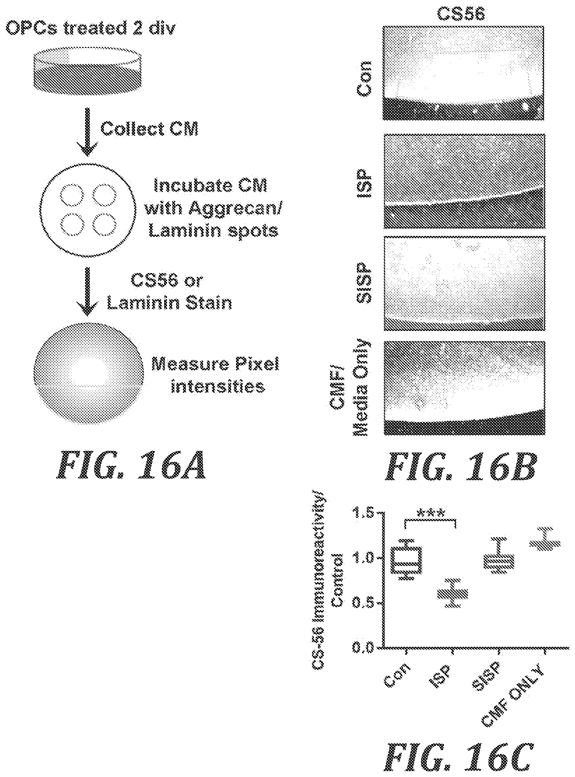

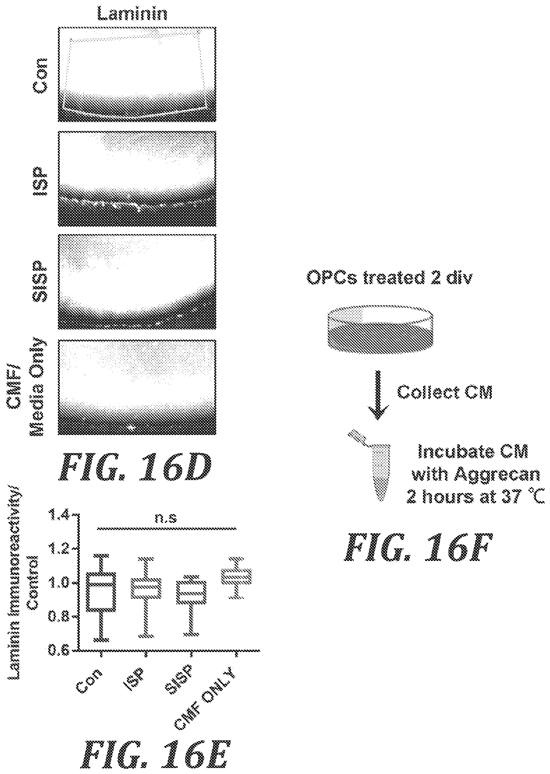

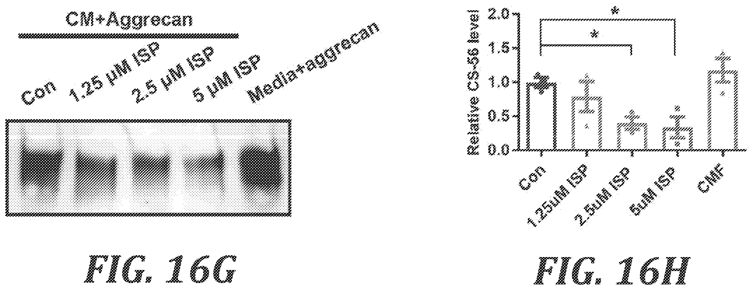

Figs. 16A-16H illustrates that ISP enhances CS56 degradation in a dose dependent manner. ISP-treated OPC CM is capable of degrading CS56 spots. Fig. 16A: OPCs treated with vehicle control, 2.5 µM ISP or SISP was incubated for 2 days *in vitro* before CM was collected and incubated with aggrecan/laminin spots. An additional subset of spots was incubated for the same duration with media only. Spots were immunostained with CS56 or laminin and the pixel intensities of the spot rim were recorded. Fig. 16B: Representative images of CS56-stained spots with yellow region of interest indicating measured portion of the spot. Fig. 16C: Quantification of CS56- immunostained spot indicates ISP-treated OPC CM significantly degrades CSPGs over control (One-Way ANOVA, Dunnett's posthoc test, P=0.0001, F(5,72)=45.19). N(images, 5 replicates)=69 Control, 104 ISP, 95 ISP, 31 Media only) Fig. 16D: Representative images of laminin-stained spots. Fig. 16E: Quantification of laminin-stained spots indicate no significant changes between groups (One-Way ANOVA, Tukey's posthoc test, P=0.0818, F(3,85)=2.312), N(images, 5 replicates)=133 Control, 134 ISP, 114 SISP, 34 Media Only) Fig. 16F: To confirm CS56 degradation, OPCs were treated with varying doses of ISP or vehicle control and incubated with a fixed concentration of aggrecan (20 µg/mL) for 2 hours before western blot analysis. Fig. 16G: Western blot analysis of CS56 and subsequent quantification (Fig.16H) of CS56 band indicate significant ISP-treated CS56 degradation over control at 2.5 and 5 µM doses (One-Way ANOVA, Tukey's posthoc test, P=0.0049, F(5,12)=6.112, N=3 western blots). Graphs indicate scatterplot representations of western blots or mean of each replicate with standard error means.

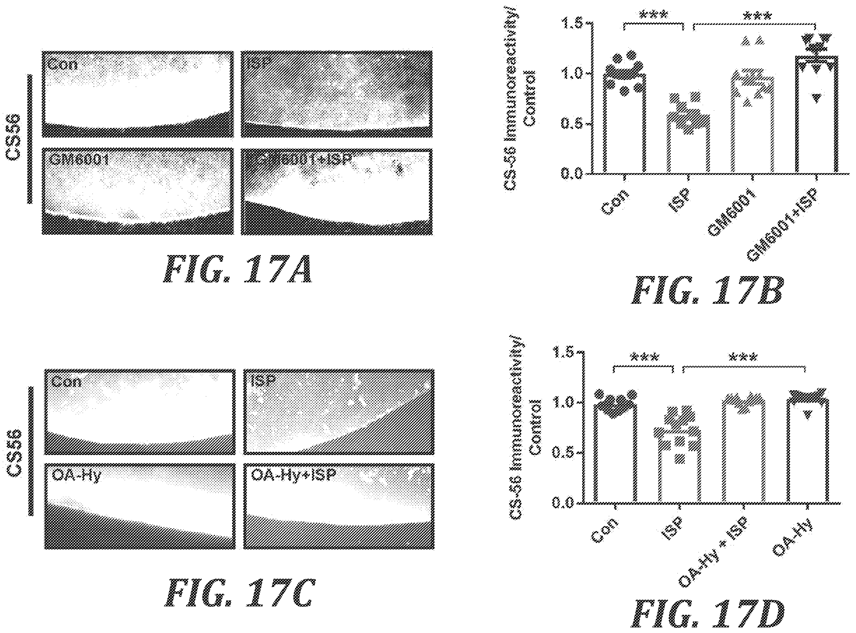

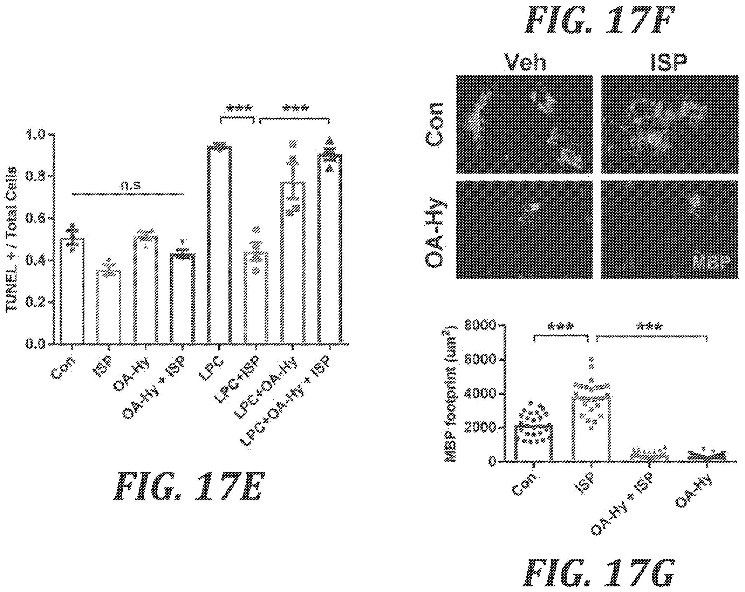

Figs. 17A-17G illustrate that protease inhibitors attenuate ISP-induced CSPG degradation and subsequent CSPG-OPC disinhibition. To assess functional effects of protease inhibitors on OPCs, Figs. 17A and 17B illustrate results of an assay where CS56-immunostained spots were incubated with vehicle control, 2.5 µM ISP, or 25 µM GM6001 +/- ISP treated OPC CM. Quantified CS56 immunoreactivity reveals significant ISP-induced CS56 degradation over control and GM6001+ISP (One54 Way ANOVA, Tukey's posthoc test, P=0.0001, F (3, 37) = 21.43), N(images, 3 replicates)=24 Control, 17 ISP, 19 GM6001, 20 GM6001+ISP). Similarly, Figs. 17C and 17D illustrate aggrecan spots treated with vehicle control, 2.5 µM ISP, or 100nM MMP-2 inhibitor (OA-Hy) +/- ISP revealed ISP-induced degradation over vehicle control and MMP-2 inhibitor (OA-Hy) + ISP (One-Way ANOVA, Tukey's posthoc test, P=0.001, F (3, 44)=31.50). N=24 images each). Fig. 17E: To assess whether the MMP-2 inhibitor affected apoptosis, OPCs cultured on aggrecan/laminin precoated coverslips were treated with ISP, 100nM MMP-2 inhibitor (OA-Hy) +/- ISP for 2 days *in vitro.* A subset of treated OPCs was additionally challenged with LPC 1ug/mL) incubation for 2 hours before TUNEL/DAPI staining. MMP-2 inhibitor (OA-Hy) did not significantly increase TUNEL+ cells/total cells. However, MMP-2 inhibitor (OA-Hy) even with ISP treatment increased apoptosis following LPC treatment over LPC-ISP treatment (One-Way ANOVA, Tukey's posthoc test, P=0.0001, F(7,21)=29.66), N=2 replicates, 2 wells each). Fig. 17F: 100nM MMP-2 inhibitor (OA-Hy) treatment of OPCs cultured on aggrecan/laminin additionally decreased MBP footprint significantly over ISP treatment alone (One-Way ANOVA, P=0.0001, F(3,112)=228.3), N(cells, 5 replicates total)=128 Control, 126 ISP, 191 OA-Hy, 222 OA-Hy+ISP). Graphs indicate scatterplot representations of mean of each replicate with standard error means.

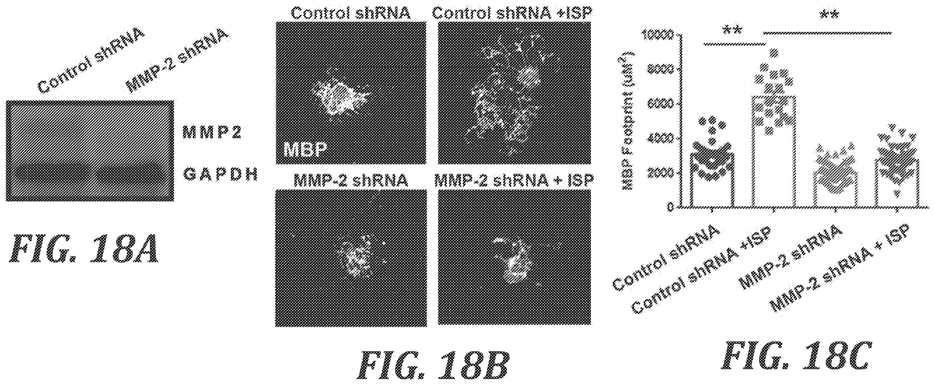

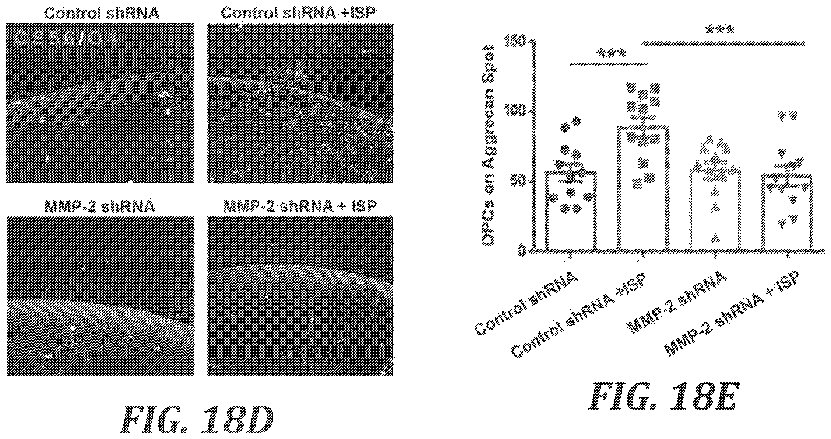

Figs. 18A-17E illustrate that shRNA knock down of MMP-2 decreases OPC maturation and migration on CSPGs to limit remyelination in cerebellar slices. Fig. 18A: OPC cultures infected with control lentiviral scrambled shRNA or lentiviral particles expressing shRNA construct targeting MMP-2 for 48 hours before western blotting to assess MMP-2 knock down compared to GAPDH. Figs. 18B and 18C: Scrambled or shRNA targeting MMP-2 lentiviral-infected OPCs cultured on laminin and low concentration of aggrecan (1 µg/mL) were immunostained with MBP following vehicle or ISP (2.5 µM) treatment for 48 hours. MBP area was quantified. (One-Way ANOVA, Tukey's posthoc test, P=0.013, F(3, 158)=6.677, N=100 cells from 2 repeats). Figs. 18D and 18E: OPCs (O4, red) were infected with lentiviral constructs 48 hours before plating onto spot assays to assess migration across aggrecan (green) with or without ISP (2.5 µM). Number of OPCs crossing aggrecan spot were counted (One-Way ANOVA, Tukey's posthoc test, P=0.0015, F(3, 44)=6.056. N=40 spots from 2 repeats).

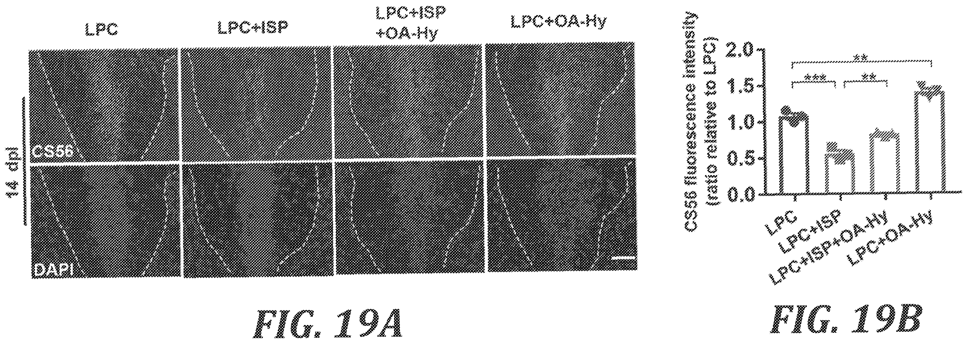

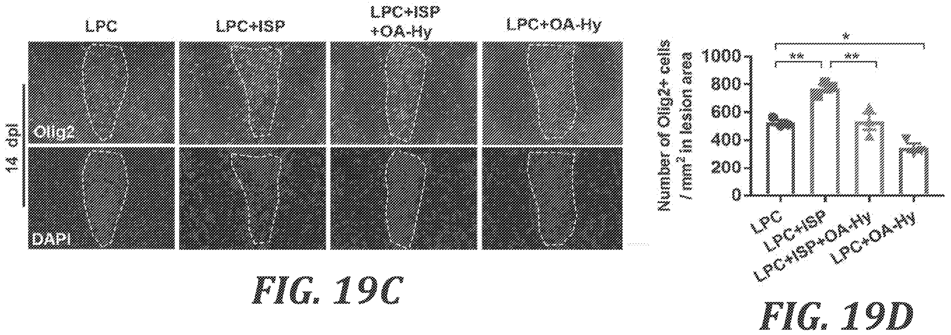

Figs. 19A-19D illustrate that MMP-2 mediates ISP-induced remyelination in LPC-demyelinated mouse model. Fig. 19A: Representative images from immunohistochemistry of CS56 and DAPI from the spinal cords of vehicle, ISP or MMP-2 inhibitor (OA-Hy)-treated mice show MMP-2-mediated CS56 degradation at 14 days post LPC injection. Dashed lines demarcate dorsal white matter of spinal cord. Scare bar= 100 µm. Fig. 19B: Relative quantification of immunofluorescence intensity of CS56 in the spinal cord of vehicle, ISP or MMP-2 inhibitor (OA-Hy)-treated mice at 14dpl (n=3 mice/group, ANOVA F(3,8)=76.08, Tukey's multiple comparison test, *P*_{LPC versus LPC+ISP} < 0.0001, *P*_{LPC+ISP versus LPC+ISP+OA-Hy} = 0.0079, *P*_{LPC versus LPC+OA-HY} = 0.0026). Fig. 19C: Representative images from immunohistochemistry of Olig2 and DAPI from the spinal cords of vehicle, ISP or MMP-2 inhibitor (OA-Hy)-treated mice show MMP-2-mediated OPCs migration at 14 days post LPC injection. Dashed lines demarcate lesion areas. Scare bar= 100 µm. Fig. 19D: Quantification of the number of Olig2+ cells in the spinal cord of vehicle, ISP or MMP-2 inhibitor (OA-Hy)-treated mice at 14dpl (n=3 mice/group, ANOVA F(3,8)=21.58, Tukey's multiple comparison test, *P*_{LPC versus LPC+ISP} = 0.0074, *P*_{LPC+ISP versus LPC+ISP+OA-Hy} = 0.0088, *P*_{LPC versus LPC+OA-HY} = 0.0385).

### DETAILED DESCRIPTION

Unless otherwise defined, scientific and technical terms used herein shall have the meanings that are commonly understood by those of ordinary skill in the art. Further, unless otherwise required by context, singular terms shall include pluralities and plural terms shall include the singular. Generally, nomenclatures utilized in connection with, and techniques of, cell and tissue culture, molecular biology, and protein and oligo- or polynucleotide chemistry and hybridization described herein are those well known and commonly used in the art.

For convenience, certain terms employed in the specification, examples, and appended claims are collected here. Unless defined otherwise, all technical and scientific terms used herein have the same meaning as commonly understood by one of ordinary skill in the art to which this application belongs.

The articles "a" and "an" are used herein to refer to one or to more than one (*i.e.,* to at least one) of the grammatical object of the article. By way of example, "an element" means one element or more than one element.

The terms "comprise," "comprising," "include," "including," "have," and "having" are used in the inclusive, open sense, meaning that additional elements may be included. The terms "such as", *"e.g.",* as used herein are non-limiting and are for illustrative purposes only. "Including" and "including but not limited to" are used interchangeably.

The term "or" as used herein should be understood to mean "and/or", unless the context clearly indicates otherwise.

As used herein, the term "about" or "approximately" refers to a quantity, level, value, number, frequency, percentage, dimension, size, amount, weight or length that varies by as much as 15%, 10%, 9%, 8%, 7%, 6%, 5%, 4%, 3%, 2% or 1% to a reference quantity, level, value, number, frequency, percentage, dimension, size, amount, weight or length. In one embodiment, the term "about" or "approximately" refers a range of quantity, level, value, number, frequency, percentage, dimension, size, amount, weight or length ± 15%, ± 10%, ± 9%, ± 8%, ± 7%, ± 6%, ± 5%, ± 4%, ± 3%, ± 2%, or ± 1% about a reference quantity, level, value, number, frequency, percentage, dimension, size, amount, weight or length.

As used herein, "one or more of a, b, and c" means a, b, c, ab, ac, bc, or abc. The use of "or" herein is the inclusive or.

The term "administering" to a patient includes dispensing, delivering or applying an active compound in a pharmaceutical formulation to a subject by any suitable route for delivery of the active compound to the desired location in the subject (*e.g.,* to thereby contact a desired cell such as a desired neuron), including administration into the cerebrospinal fluid or across the blood- brain barrier, delivery by either the parenteral or oral route, intramuscular injection, subcutaneous or intradermal injection, intravenous injection, buccal administration, transdermal delivery and administration by the rectal, colonic, vaginal, intranasal or respiratory tract route. The agents may, for example, be administered to a comatose, anesthetized or paralyzed subject via an intravenous injection or may be administered intravenously to a pregnant subject to stimulate axonal growth in a fetus. Specific routes of administration may include topical application (such as by eye drops, creams or erodible formulations to be placed under the eyelid, intraocular injection into the aqueous or the vitreous humor, injection into the external layers of the eye, such as via subconjunctival injection or subtenon injection, parenteral administration or via oral routes.

The term "antibody", includes human and animal mAbs, and preparations of polyclonal antibodies, synthetic antibodies, including recombinant antibodies (antisera), chimeric antibodies, including humanized antibodies, anti-idiotopic antibodies and derivatives thereof. A portion or fragment of an antibody refers to a region of an antibody that retains at least part of its ability (binding specificity and affinity) to bind to a specified epitope. The term "epitope" or "antigenic determinant" refers to a site on an antigen to which antibody paratope binds. Epitopes formed from contiguous amino acids are typically retained on exposure to denaturing solvents whereas epitopes formed by tertiary folding are typically lost on treatment with denaturing solvents. An epitope typically includes at least 3, at least 5, or 8 to 10, or about 13 to 15 amino acids in a unique spatial conformation. Methods of determining spatial conformation of epitopes include, for example, x-ray crystallography and 2-dimensional nuclear magnetic resonance.

The terms "chimeric protein" or "fusion protein" refer to a fusion of a first amino acid sequence encoding a polypeptide with a second amino acid sequence defining a domain (*e.g*., polypeptide portion) foreign to and not substantially homologous with the domain of the first polypeptide. A chimeric protein may present a foreign domain, which is found (albeit in a different protein) in an organism, which also expresses the first protein, or it may be an "interspecies", "intergenic", etc. fusion of protein structures expressed by different kinds of organisms.

An "effective amount" of an agent or therapeutic peptide is an amount sufficient to achieve a desired therapeutic or pharmacological effect, such as an amount that is capable of activating the growth of neurons. An effective amount of an agent as defined herein may vary according to factors such as the disease state, age, and weight of the subject, and the ability of the agent to elicit a desired response in the subject. Dosage regimens may be adjusted to provide the optimum therapeutic response. An effective amount is also one in which any toxic or detrimental effects of the active compound are outweighed by the therapeutically beneficial effects.

The term "expression" refers to the process by which nucleic acid is translated into peptides or is transcribed into RNA, which, for example, can be translated into peptides, polypeptides or proteins. If the nucleic acid is derived from genomic DNA, expression may, if an appropriate eukaryotic host cell or organism is selected, include splicing of the mRNA. For heterologous nucleic acid to be expressed in a host cell, it must initially be delivered into the cell and then, once in the cell, ultimately reside in the nucleus.

The term "genetic therapy" and grammatical variants thereof (*e.g.,* "gene therapy"), involves the transfer of heterologous DNA to cells of a mammal, particularly a human, with a disorder or conditions for which therapy or diagnosis is sought. The DNA is introduced into the selected target cells in a manner such that the heterologous DNA is expressed and a therapeutic product encoded thereby is produced. Alternatively, the heterologous DNA may in some manner mediate expression of DNA that encodes the therapeutic product; it may encode a product, such as a peptide or RNA that in some manner mediates, directly or indirectly, expression of a therapeutic product. Genetic therapy may also be used to deliver nucleic acid encoding a gene product to replace a defective gene or supplement a gene product produced by the mammal or the cell in which it is introduced. The heterologous DNA encoding the therapeutic product may be modified prior to introduction into the cells of the afflicted host in order to enhance or otherwise alter the product or expression thereof.

The term "gene" or "recombinant gene" refers to a nucleic acid comprising an open reading frame encoding a polypeptide, including both exon and (optionally) intron sequences.

The term "heterologous nucleic acid sequence" is typically DNA that encodes RNA and proteins that are not normally produced *in vivo* by the cell in which it is expressed or that mediates or encodes mediators that alter expression of endogenous DNA by affecting transcription, translation, or other regulatable biochemical processes. A heterologous nucleic acid sequence may also be referred to as foreign DNA. Any DNA that one of skill in the art would recognize or consider as heterologous or foreign to the cell in which it is expressed is herein encompassed by heterologous DNA. Examples of heterologous DNA include, but are not limited to, DNA that encodes traceable marker proteins, such as a protein that confers drug resistance, DNA that encodes therapeutically effective substances, such as anti-cancer agents, enzymes and hormones, and DNA that encodes other types of proteins, such as antibodies. Antibodies that are encoded by heterologous DNA may be secreted or expressed on the surface of the cell in which the heterologous DNA has been introduced.

The terms "homology" and "identity" are used synonymously throughout and refer to sequence similarity between two peptides or between two nucleic acid molecules. Homology can be determined by comparing a position in each sequence, which may be aligned for purposes of comparison. When a position in the compared sequence is occupied by the same base or amino acid, then the molecules are homologous or identical at that position. A degree of homology or identity between sequences is a function of the number of matching or homologous positions shared by the sequences.

The term "oligodendrocyte progenitor cells" or "OPCs" as used herein refers to a neural progenitor cell capable to generate new oligodendrocyte cells. OPCs can be identified by the expression of a number of surface antigens. For example, the surface antigens known as platelet-derived growth factor-alpha receptor subunit (PDGFRα), NG2 chondroitin sulfate proteoglycan, and ganglioside GD3, are commonly used to identify OPCs.

Immature OPCs are generated in ventral areas of the developing brain from a common glial progenitor. The immature cells actively migrate, proliferate, and populate the central nervous system (CNS) to finally differentiate to premyelinating oligodendrocytes (O4+). OPC differentiation and maturation is characterized by an extension of multiple processes, increase in cell body size and formation of myelin.

The phrases "parenteral administration" and "administered parenterally" as used herein means modes of administration other than enteral and topical administration, usually by injection, and includes, without limitation, intravenous, intramuscular, intraarterial, intrathecal, intraventricular, intracapsular, intraorbital, intracardiac, intradermal, intraperitoneal, transtracheal, subcutaneous, subcuticular, intraarticular, subcapsular, subarachnoid, intraspinal and intrasternal injection and infusion.

The phrases "systemic administration," "administered systemically," "peripheral administration" and "administered peripherally" as used herein mean the administration of a compound, drug or other material other than directly into a target tissue, such that it enters the animal's system and, thus, is subject to metabolism and other like processes, for example, subcutaneous administration.

The term "patient" or "subject" or "animal" or "host" refers to any mammal. The subject may be a human, but can also be a mammal in need of veterinary treatment, *e.g.,* domestic animals (*e.g.,* dogs, cats, and the like), farm animals (*e.g.,* cows, sheep, fowl, pigs, horses, and the like) and laboratory animals (*e.g*., rats, mice, guinea pigs, and the like).

The term "peripheral nervous system (PNS) neurons" includes the neurons which reside or extend outside of the CNS. PNS is intended to include the neurons commonly understood as categorized in the peripheral nervous system, including sensory neurons and motor neurons.

The terms "polynucleotide sequence" and "nucleotide sequence" are also used interchangeably herein.

The terms "peptide" or "polypeptide" are used interchangeably herein and refer to compounds consisting of from about 2 to about 90 amino acid residues, inclusive, wherein the amino group of one amino acid is linked to the carboxyl group of another amino acid by a peptide bond. A peptide can be, for example, derived or removed from a native protein by enzymatic or chemical cleavage, or can be prepared using conventional peptide synthesis techniques (*e.g*., solid phase synthesis) or molecular biology techniques (see Sambrook et al., MOLECULAR CLONING: LAB. MANUAL (Cold Spring Harbor Press, Cold Spring Harbor, NY, 1989)). A "peptide" can comprise any suitable L-and/or D-amino acid, for example, common a-amino acids (*e.g.,* alanine, glycine, valine), non-a-amino acids (*e.g., P-*alanine, 4-aminobutyric acid, 6aminocaproic acid, sarcosine, statine), and unusual amino acids (*e.g*., citrulline, homocitruline, homo serine, norleucine, norvaline, ornithine). The amino, carboxyl and/or other functional groups on a peptide can be free (*e.g*., unmodified) or protected with a suitable protecting group. Suitable protecting groups for amino and carboxyl groups, and means for adding or removing protecting groups are known in the art. See, *e.g.,* Green & Wuts, PROTECTING GROUPS IN ORGANIC SYNTHESIS (John Wiley & Sons, 1991). The functional groups of a peptide can also be derivatized (*e.g*., alkylated) using art-known methods.

Peptides can be synthesized and assembled into libraries comprising a few too many discrete molecular species. Such libraries can be prepared using well-known methods of combinatorial chemistry, and can be screened as described herein or using other suitable methods to determine if the library comprises peptides which can antagonize CSPG-PTPσ interaction. Such peptide antagonists can then be isolated by suitable means.

The term "peptidomimetic", refers to a protein-like molecule designed to mimic a peptide. Peptidomimetics typically arise either from modification of an existing peptide, or by designing similar systems that mimic peptides, such as peptoids and β-peptides. Irrespective of the approach, the altered chemical structure is designed to advantageously adjust the molecular properties such as, stability or biological activity. These modifications involve changes to the peptide that do not occur naturally (such as altered backbones and the incorporation of nonnatural amino acids).

The terms "prevent" or "preventing" refer to reducing the frequency or severity of a disease or condition. The term does not require an absolute preclusion of the disease or condition. Rather, this term includes decreasing the chance for disease occurrence. For example, disclosed but not claimed are methods of reducing the occurrence and/or severity of a root avulsion injury in a subject, comprising administering to the root avulsion injury of the subject a therapeutically effective amount of a composition comprising a therapeutic agent.

A polynucleotide sequence (DNA, RNA) is "operatively linked" to an expression control sequence when the expression control sequence controls and regulates the transcription and translation of that polynucleotide sequence. The term "operatively linked" includes having an appropriate start signal (*e.g.,* ATG) in front of the polynucleotide sequence to be expressed, and maintaining the correct reading frame to permit expression of the polynucleotide sequence under the control of the expression control sequence, and production of the desired polypeptide encoded by the polynucleotide sequence.

The term "recombinant," as used herein, means that a protein is derived from a prokaryotic or eukaryotic expression system.

The term "therapeutically effective" means that the amount of the composition used is of sufficient quantity to ameliorate one or more causes, symptoms, or sequelae of a disease or disorder. Such amelioration only requires a reduction or alteration, not necessarily elimination, of the causes, symptoms, or sequelae of a disease or disorder.

The term "tissue-specific promoter" means a nucleic acid sequence that serves as a promoter, *i.e.,* regulates expression of a selected nucleic acid sequence operably linked to the promoter, and which affects expression of the selected nucleic acid sequence in specific cells of a tissue, such as cells of epithelial cells. The term also covers so-called "leaky" promoters, which regulate expression of a selected nucleic acid primarily in one tissue, but cause expression in other tissues as well. The term "transfection" is used to refer to the uptake of foreign DNA by a cell. A cell has been "transfected" when exogenous DNA has been introduced inside the cell membrane. A number of transfection techniques are generally known in the art. See, *e.g*., Graham et al., Virology 52:456 (1973); Sambrook et al., Molecular Cloning: A Laboratory Manual (1989); Davis et al., Basic Methods in Molecular Biology (1986); Chu et al., Gene 13:197 (1981). Such techniques can be used to introduce one or more exogenous DNA moieties, such as a nucleotide integration vector and other nucleic acid molecules, into suitable host cells. The term captures chemical, electrical, and viral-mediated transfection procedures.

The terms "transcriptional regulatory sequence" is a generic term used in the specification to refer to nucleic acid sequences, such as initiation signals, enhancers, and promoters, which induce or control transcription of protein coding sequences with which they are operably linked. In some examples, transcription of a recombinant gene is under the control of a promoter sequence (or other transcriptional regulatory sequence), which controls the expression of the recombinant gene in a cell-type in which expression is intended. It will also be understood that the recombinant gene can be under the control of transcriptional regulatory sequences which are the same or which are different from those sequences, which control transcription of the naturally occurring form of a protein.

The term "treatment" refers to the medical management of a patient with the intent to cure, ameliorate, stabilize, or prevent a disease, pathological condition, or disorder. This term includes active treatment, that is, treatment directed specifically toward the improvement of a disease, pathological condition, or disorder, and also includes causal treatment, that is, treatment directed toward removal of the cause of the associated disease, pathological condition, or disorder. In addition, this term includes palliative treatment, that is, treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive treatment, that is, treatment employed to supplement another specific therapy directed toward the improvement of the associated disease, pathological condition, or disorder.

The term "vector" refers to a nucleic acid molecule capable of transporting another nucleic acid to which it has been linked. Preferred vectors are those capable of one or more of, autonomous replication and expression of nucleic acids to which they are linked. Vectors capable of directing the expression of genes to which they are operatively linked are referred to herein as "expression vectors".

The term "wild type" (or "WT") refers to the naturally-occurring polynucleotide sequence encoding a protein, or a portion thereof, or protein sequence, or portion thereof, respectively, as it normally exists *in vivo.* As used herein, the term "nucleic acid" refers to polynucleotides, such as deoxyribonucleic acid (DNA), and, where appropriate, ribonucleic acid (RNA). The term should also be understood to include, as equivalents, analogs of either RNA or DNA made from nucleotide analogs, and, as applicable to the embodiment being described, single (sense or antisense) and double-stranded polynucleotides.

The agents for usedescribed herein are considered to be purified and/or isolated prior to their use. Purified materials are typically "substantially pure", meaning that a nucleic acid, polypeptide or fragment thereof, or other molecule has been separated from the components that naturally accompany it. Typically, the polypeptide is substantially pure when it is at least 60%, 70%, 80%, 90%, 95%, or even 99%, by weight, free from the proteins and other organic molecules with which it is associated naturally. For example, a substantially pure polypeptide may be obtained by extraction from a natural source, by expression of a recombinant nucleic acid in a cell that does not normally express that protein, or by chemical synthesis. "Isolated materials" have been removed from their natural location and environment. In the case of an isolated or purified domain or protein fragment, the domain or fragment is substantially free from amino acid sequences that flank the protein in the naturally-occurring sequence. The term "isolated DNA" means DNA has been substantially freed of the genes that flank the given DNA in the naturally occurring genome. Thus, the term "isolated DNA" encompasses, for example, cDNA, cloned genomic DNA, and synthetic DNA.

The terms "portion", "fragment", "variant", "derivative" and "analog", when referring to a polypeptide include any polypeptide that retains at least some biological activity referred to herein (*e.g*., inhibition of an interaction such as binding). Polypeptides as described herein may include portion, fragment, variant, or derivative molecules without limitation, as long as the polypeptide still serves its function. Polypeptides or portions thereof of the present invention may include proteolytic fragments, deletion fragments and in particular, or fragments that more easily reach the site of action when delivered to an animal.

Embodiments described herein generally relate to agents for use in methods for inducing, promoting, and/or modulating oligodendrocyte progenitor cell (OPC) migration, differentiation, proliferation and/or maturation in subjects having or being at risk of a myelin related disorder.

We found that certain types of chondroitin sulfate proteoglycans (CSPGs) released into the extracellular matrix during the formation of glial scarring in response to a central nervous system (CNS) injury can curtail the generation of myelin through binding with cognate receptor protein tyrosine phosphatase σ (PTPσ) on OPCs. In a variety of *in vitro, in situ* and *in vivo* models of multiple sclerosis (MS), proteoglycan deposition during lesion associated scar formation potently inhibited OPC migration, differentiation and the reformation of myelin. We found that a systemic peptide treatment that inhibited PTPσ catalytic activity, signaling, and/or function remarkably enhanced the rate of remyelination in LPS induced lesions and stimulated robust myelin regeneration and functional recovery following chronic demyelination. Moreover, inhibition of PTPσ catalytic activity, signaling, and/or function can enhance up-regulation of the protease MMP-2 secreted by OPCs that, in turn, allows a robust digestion of CSPGs that may halt or slow the progression of MS.

Accordingly, a therapeutic agent that suppresses, inhibits, and/or blocks one or more of catalytic activity, signaling, and/or function of PTPσ is for use in a method that can involve administeration to a subject having or being at risk of a myelin related disorder to induce, promote, and/or modulate OPC migration, differentiation, proliferation and/or maturation.

The activity, signaling, and/or function of PTPσ can be suppressed, inhibited, and/or blocked in several ways including: direct inhibition of the activity of the intracellular domain of the PTPσ (*e.g.,* by using small molecules, peptidomimetics, antibodies, intrabodies, or dominant negative polypeptides); activation of genes and/or proteins that inhibit one or more of, the activity, signaling, and/or function of the intracellular domain of PTPσ (*e.g.,* by increasing the expression or activity of the genes and/or proteins); inhibition of genes and/or proteins that are downstream mediators of the PTPσ (*e.g.,* by blocking the expression and/or activity of the mediator genes and/or proteins); introduction of genes and/or proteins that negatively regulate one or more of, activity, signaling, and/or function of PTPσ (*e.g.,* by using recombinant gene expression vectors, recombinant viral vectors or recombinant polypeptides); or gene replacement with, for instance, a hypomorphic mutant of PTPσ (*e.g.,* by homologous recombination, overexpression using recombinant gene expression or viral vectors, or mutagenesis).

The therapeutic agent that inhibits or reduces one or more of the activity, signaling, and/or function of PTPσ can include an agent that decreases and/or suppresses the activity, signaling, and/or function of PTPσ. Such agents can be delivered intracellularly and once delivered intracellularly promote OPC migration, differentiation, proliferation and/or maturation and enhance myelination or remyelination.

The therapeutic agent that inhibits or reduces one or more of the activity, signaling, and/or function of PTPσ, includes a therapeutic peptide that binds to and/or complexes with the intracellular domain of PTPσ, in particular, the intracellular wedge shaped domain, to inhibit the activity, signaling, and/or function of PTPσ. Accordingly, therapeutic peptides that bind to and/or complex with the intracellular domain of PTPσ of OLs and/or OPCs (OLs/OPCs) can be used to promote cell growth, motility, survival and plasticity of these cells.

The therapeutic agent is a peptide mimetic of the wedge shaped domain (*i.e.,* wedge domain) of the intracellular catalytic domain of PTPσ, such as described, for example, in WO 2013/155103A1. Peptide mimetics of the wedge domain of the PTPσ when expressed in cells (*e.g.,* OLs/OPCs) or conjugated to an intracellular transport moiety can bind to the wedge domain and be used to abolish PTPσ signaling in OLs/OPCs activated with CSPGs to promote cell growth, motility, and survival. Binding of these therapeutic peptides to PTPσ intact wedge domain can potentially: (i) interfere with the ability for PTPσ to interact with target proteins, such as phosphatase targets; (ii) interfere with activity promoting intermolecular interactions between PTPσ and another domain contained in PTPσ, such as the catalytically inactive second phosphatase domain D2; (iii) prevent access of proteins to the active phosphatase site; (iv) out-compete normal interactors of the wedge domain; and/or (v) sterically inhibit phosphatase activity.

The peptide mimetic (*i.e*., therapeutic peptide) has an amino acid sequence that has at least about 65% identity to the amino acid 15 sequence of SEQ ID NO:32 or SEQ ID NO:33 can include, consist essentially, and/or consist of about 10 to about 20 amino acids and have an amino acid sequence that is at least about 65%, at least about 70%, at least about 75%, at least about 80%, at least about 85%, at least about 90%, at least about 95%, or about 100% homologous or identical to an about 10 to about 20 consecutive amino acid portion of the amino acid sequence of the wedge domain of PTPσ. In some embodiments, the about 10 to about 20 consecutive amino acid portion includes consecutive amino acids of N-terminal alpha helix and 4 amino acid turn of the wedge domain.

A peptide (*e.g*., therapeutic peptide) corresponding to or substantially homologous to the wedge domain of PTPσ with a cytosolic-carrier was able to relieve CSPG-mediated inhibition, enhance the rate of remyelination in LPS induced lesions, stimulate robust myelin regeneration and functional recovery following chronic demyelination, and enhance up-regulation of the protease MMP-2 secreted by OPCs that, in turn, allows a robust digestion of CSPGs. This peptide can be given sytemically to a subject to promote myelination or remyelination.

As shown in Table 1, the wedge domain sequence of PTPσ is highly conserved among higher mammals, with only a single amino acid change in humans to mouse and rats (Threonine to Methithione at position 6) preventing 100% homology.

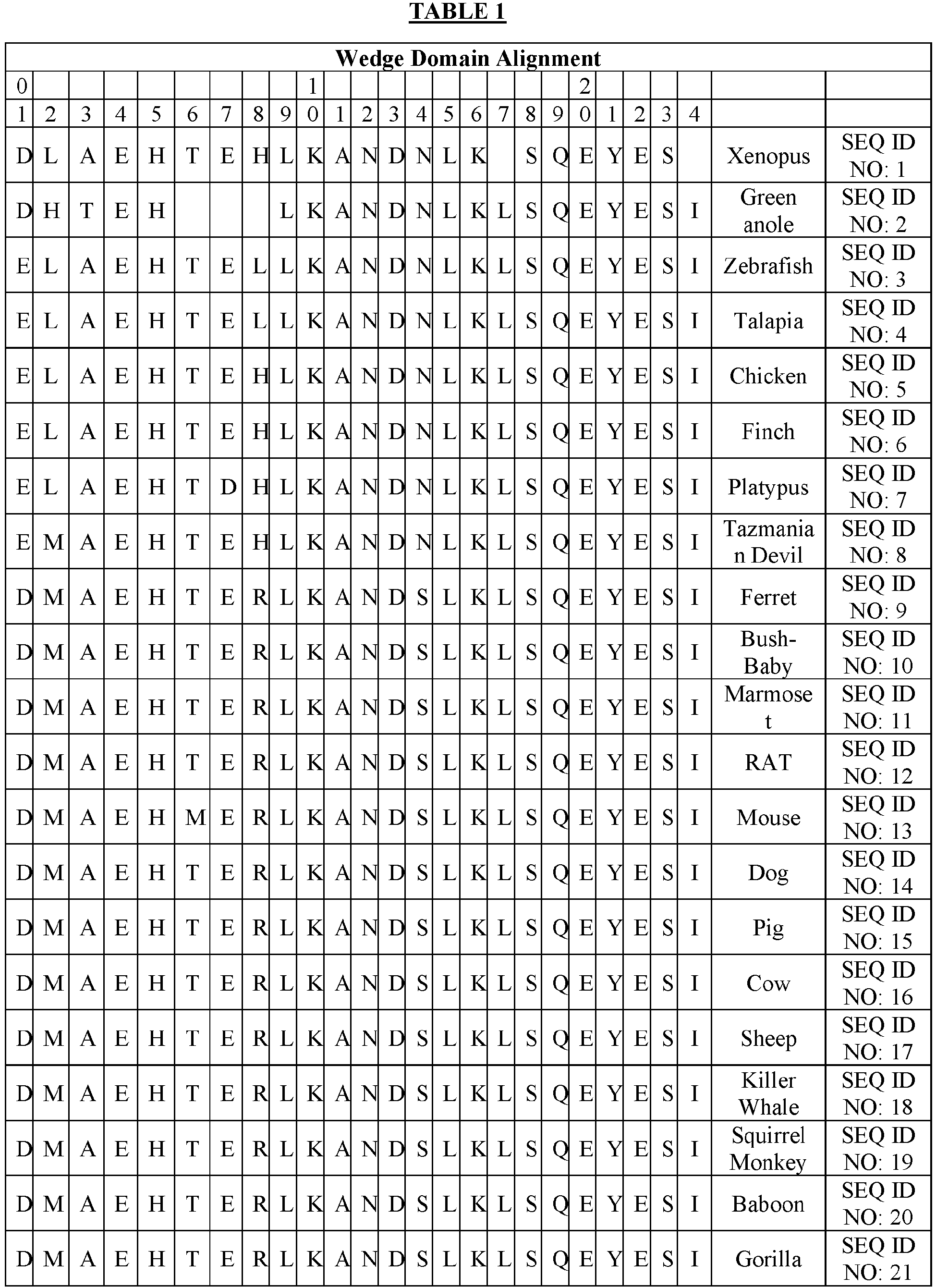

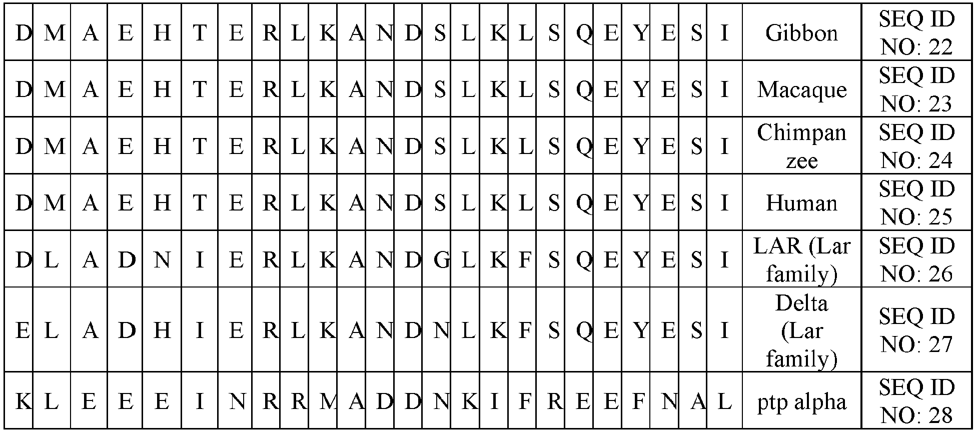

As shown in Table 1, the first alpha helix of the wedge domain of PTPσ includes amino acids 1-10, the turn region includes amino acids 11-14, and the second alpha helix includes amino acids 15-24. For example, the first alpha helix of the wedge domain of human PTPσ has the amino acid sequence of DMAEHTERLK (SEQ ID NO: 29), the turn has the amino acid sequence of ANDS (SEQ ID NO: 30), and the second alpha helix has the amino acid sequence of LKLSQEYESI (SEQ ID NO: 31).

The wedge domain also shares sequence homology with the other members of the LAR family, LAR and PTPσ. It is likely that these amino acids are necessary for the overall structure of the wedge domain. Conserved amino acids include an alanine at position 13, which marks the end of the first alpha helix and the start of the turn, making it likely to be necessary for general wedge size and structure.

Since the general secondary and tertiary structures of the wedge domain remain consistent through most receptor PTPs, several conservative substitutions can be made to a therapeutic peptide targeting the PTPσ wedge domain to obtain similar results. Examples of conservative substitutions include the substitution of one non-polar (hydrophobic) residue, such as isoleucine, valine, leucine or methionine for another, the substitution of one polar (hydrophilic) residue for another, such as between arginine and lysine, between glutamine and asparagine, between glycine and serine, the substitution of one basic residue such as lysine, arginine or histidine for another, and/or the substitution of one acidic residue, such as aspartic acid or glutamic acid for another.

These conservative substitutions can occur in the non-unique domains in either alpha helix or the turn, specifically positions 1-3 and 7-10 in the first alpha helix; 12 and 13 in the turn; and 15, 16, 18-24 in the second alpha helix. These amino acids may be necessary to the overall structure of the wedge domain, but not necessary for specificity of binding of wedge to PTPσ.

The unique amino acids to PTPσ, particularly the amino acids expressed differentially in PTPσ vs LAR, were found to be necessary for specificity of wedge domain binding. These include an EH domain in the first alpha helix position 4 and 5 followed by a threonine or a metathione (rat and mouse substitution) at position 6. In the turn, there is a unique serine at position 14 in all higher mammals. Finally, there is a unique leucine at position 17 in the second alpha helix. The potential roles of these unique amino acids will be discussed below.

The serine residue in the turn at position 14 is of particular interest due to its location in the wedge domain. This amino acid, located in the turn between alpha helixes, is slightly extended from the general secondary and tertiary structure of PTPα, making it available for binding interactions. In addition, serine, due to its hydroxyl group and the polarity it contains, is known to facilitate several homophillic and heterophillic binding events, such as hydrogen binding between adjacent serines. Serines are also known to undergo various modifications, such as phosphorylation, making the likelihood of its necessity for specificity high. It is possible that smaller peptides that focus on the turn in the wedge domain and include the conserved serine may offer greater stability with similar function. Such peptides can be synthesized as loops, with cysteine's on either end to created di-sulfide bonds.

The unique amino acids in the first alpha helix include glutamic acid at position 4, histidine at position 5 and threonine or metathione at position 6. Although the histidine is implicated in the consensus wedge domain, it is not found in LAR, PTPσ, PTPµ or CD45. As all three of these amino acids are either charged or polar, it is likely that either this sequence or one of its components is necessary for PTPσ wedge specificity.

Additionally, the second alpha helix contains a unique leucine at position 17. Leucines have been implicated as the critical adhesive molecules for the three dimensional structure of leucine zippers. In these molecules, which are structurally similar to wedge domains, leucines of opposing alpha helixes, located at approximately 7 intervals, interact with hydrophobic regions of the opposing alpha helix. As there is also a Leucine in the first alpha helix, located at position 9, it is believed that this unique leucine is necessary for the overall three-dimensional structural integrity of the PTPσ wedge.

Accordingly, in some embodiments, the therapeutic peptide can include, consist essentially of, or consist of about 14 to about 20 amino acids and include the amino acid sequence EHX₁ERLKANDSLKL (SEQ ID NO: 32), wherein X₁ is T or M. A therapeutic peptide including SEQ ID NO: 32 can include at least one, at least two, at least three, at least four, or at least five conservative substitutions so that the therapeutic peptide has an amino acid sequence that is at least about 65%, at least about 70%, at least about 75%, at least about 80%, at least about 85%, at least about 90%, or at least about 95% homologous to SEQ ID NO: 32.

In some embodiments, the conservative substitutions can be of amino acid residues 4E, 5R, 6L, 7K, 9N, 10D, 12L, or 13K of SEQ ID NO: 32. By way of example, amino acid residue 4E can be substituted with D or Q, amino acid residue 5R can be substituted with H, L, or K, amino acid residue 6L can be substituted with I, V, or M, amino acid residue 7K can be substituted with R or H, amino acid residue 9N can be substituted with E or D, amino acid residue 10 D can be substituted with E or N, amino acid residue 12L can be substituted with I, V, or M, and/or amino acid residue 13K can be substituted with R or H.

In other embodiments, the therapeutic peptide can include, consist essentially of, or consist of about 14 to about 20 amino acids and include the amino acid sequence DMAEHX₁ERLKANDS (SEQ ID NO: 33), wherein X₁ is T or M. A therapeutic peptide including SEQ ID NO: 33 can include at least one, at least two, at least three, at least four, or at least five conservative substitutions so that the therapeutic peptide has an amino acid sequence that is at least about 65%, at least about 70%, at least about 75%, at least about 80%, at least about 85%, at least about 90%, or at least about 95% homologous to SEQ ID NO: 33.

In some embodiments, the conservative substitutions can be of amino acid residues 7E, 8R, 9L, 10K, 12N, or 13D of SEQ ID NO: 33. By way of example, amino acid residue 7E can be substituted with D or Q, amino acid residue 8R can be substituted with H, L, or K, amino acid residue 9L can be substituted with I, V, or M, amino acid residue 10K can be substituted with R or H, amino acid residue 12N can be substituted with E or D, and amino acid residue 13 D can be substituted with E or N.

The therapeutic peptides described herein can be subject to other various changes, substitutions, insertions, and deletions where such changes provide for certain advantages in its use. In this regard, therapeutic peptides that bind to and/or complex with a wedge domain of PTPσ can correspond to or be substantially homologous with, rather than be identical to, the sequence of a recited polypeptide where one or more changes are made and it retains the ability to inhibits or reduces one or more of the activity, signaling, and/or function of PTPσ function.

The therapeutic polypeptide can be in any of a variety of forms of polypeptide derivatives that include amides, conjugates with proteins, cyclized polypeptides, polymerized polypeptides, analogs, fragments, chemically modified polypeptides and the like derivatives.

It will be appreciated that the conservative substitution can also include the use of a chemically derivatized residue in place of a non-derivatized residue provided that such peptide displays the requisite binding activity.

"Chemical derivative" refers to a subject polypeptide having one or more residues chemically derivatized by reaction of a functional side group. Such derivatized molecules include for example, those molecules in which free amino groups have been derivatized to form amine hydrochlorides, p-toluene sulfonyl groups, carbobenzoxy groups, t-butyloxycarbonyl groups, chloroacetyl groups or formyl groups. Free carboxyl groups may be derivatized to form salts, methyl and ethyl esters or other types of esters or hydrazides. Free hydroxyl groups may be derivatized to form O-acyl or O-alkyl derivatives. The imidazole nitrogen of histidine may be derivatized to form N-im-benzylhistidine. Also included as chemical derivatives are those polypeptides, which contain one or more naturally occurring amino acid derivatives of the twenty standard amino acids. For example: 4-hydroxyproline may be substituted for proline; 5-hydroxylysine may be substituted for lysine; 3-methylhistidine may be substituted for histidine; homoserine may be substituted for serine; and ornithine may be substituted for lysine. Polypeptides described herein may also include any polypeptide having one or more additions and/or deletions or residues relative to the sequence of a polypeptide whose sequence is shown herein, so long as the requisite activity is maintained.

One or more of peptides of the therapeutic peptides described herein can also be modified by natural processes, such as posttranslational processing, and/or by chemical modification techniques, which are known in the art. Modifications may occur in the peptide including the peptide backbone, the amino acid side-chains and the amino or carboxy termini. It will be appreciated that the same type of modification may be present in the same or varying degrees at several sites in a given peptide. Modifications comprise for example, without limitation, acetylation, acylation, addition of acetomidomethyl (Acm) group, ADP-ribosylation, amidation, covalent attachment to fiavin, covalent attachment to a heme moiety, covalent attachment of a nucleotide or nucleotide derivative, covalent attachment of a lipid or lipid derivative, covalent attachment of phosphatidylinositol, cross-linking, cyclization, disulfide bond formation, demethylation, formation of covalent cross-links, formation of cystine, formation of pyroglutamate, formylation, gamma-carboxylation, glycosylation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation and ubiquitination (for reference see, Protein-structure and molecular properties, 2nd Ed., T. E. Creighton, W. H. Freeman and Company, New-York, 1993).

Peptides and/or proteins described herein may also include, for example, biologically active mutants, variants, fragments, chimeras, and analogues; fragments encompass amino acid sequences having truncations of one or more amino acids, wherein the truncation may originate from the amino terminus (N-terminus), carboxy terminus (C-terminus), or from the interior of the protein. Analogues of the invention involve an insertion or a substitution of one or more amino acids. Variants, mutants, fragments, chimeras and analogues may function as inhibitors of the LAR family phosphatases (without being restricted to the present examples).

The therapeutic polypeptides described herein may be prepared by methods known to those skilled in the art. The peptides and/or proteins may be prepared using recombinant DNA. For example, one preparation can include cultivating a host cell (bacterial or eukaryotic) under conditions, which provide for the expression of peptides and/or proteins within the cell.

The purification of the polypeptides may be done by affinity methods, ion exchange chromatography, size exclusion chromatography, hydrophobicity or other purification technique typically used for protein purification. The purification step can be performed under non-denaturating conditions. On the other hand, if a denaturating step is required, the protein may be renatured using techniques known in the art.

In some embodiments, the therapeutic peptides described herein can include additional residues that may be added at either terminus of a polypeptide for the purpose of providing a "linker" by which the polypeptides can be conveniently linked and/or affixed to other polypeptides, proteins, detectable moieties, labels, solid matrices, or carriers.

Amino acid residue linkers are usually at least one residue and can be 40 or more residues, more often 1 to 10 residues. Typical amino acid residues used for linking are glycine, tyrosine, cysteine, lysine, glutamic and aspartic acid, or the like. In addition, a subject polypeptide can differ by the sequence being modified by terminal-NH₂ acylation, *e.g.,* acetylation, or thioglycolic acid amidation, by terminal-carboxylamidation, *e.g.,* with ammonia, methylamine, and the like terminal modifications. Terminal modifications are useful, as is well known, to reduce susceptibility by proteinase digestion, and therefore serve to prolong half life of the polypeptides in solutions, particularly biological fluids where proteases may be present. In this regard, polypeptide cyclization is also a useful terminal modification, and is particularly preferred also because of the stable structures formed by cyclization and in view of the biological activities observed for such cyclic peptides as described herein.

In some embodiments, the linker can be a flexible peptide linker that links the therapeutic peptide to other polypeptides, proteins, and/or molecules, such as detectable moieties, labels, solid matrices, or carriers. A flexible peptide linker can be about 20 or fewer amino acids in length. For example, a peptide linker can contain about 12 or fewer amino acid residues, *e.g.,* 3, 4, 5, 6, 7, 8, 9, 10, 11, and 12. In some cases, a peptide linker comprises two or more of the following amino acids: glycine, serine, alanine, and threonine.

In some embodiments, a therapeutic agent comprising the therapeutic peptides described herein can be provided in the form of a conjugate protein or drug delivery construct includes at least a transport subdomain(s) or moiety(ies) (*i.e.,* transport moieties) that is linked to the therapeutic peptide. The transport moieties can facilitate uptake of the therapeutic polypeptides into a mammalian (*i.e.,* human or animal) tissue or cell (*e.g.,* neural cell). The transport moieties can be covalently linked to the therapeutic polypeptides. The covalent link can include a peptide bond or a labile bond (*e.g.,* a bond readily cleavable or subject to chemical change in the interior target cell environment). Additionally, the transport moieties can be cross-linked (e*.g.,* chemically cross-linked, UV cross-linked) to the therapeutic polypeptide. The transport moieties can also be linked to the therapeutic polypeptide with linking polypeptide described herein.

The transport moieties can be repeated more than once in the therapeutic agent. The repetition of a transport moiety may affect (*e.g.,* increase) the uptake of the peptides and/or proteins by a desired cell. The transport moiety may also be located either at the amino-terminal region of therapeutic peptide or at its carboxy-terminal region or at both regions.

In one embodiment, the transport moiety can include at least one transport peptide sequence that allows the therapeutic polypeptide once linked to the transport moiety to penetrate into the cell by a receptor-independent mechanism. In one example, the transport peptide is a synthetic peptide that contains a Tat-mediated protein delivery sequence and at least one of SEQ ID NOs: 1-25, 32, and 33. These peptides can have, respectively, the amino acid sequences of SEQ ID NOs: 34-60.

Other examples of known transport moieties, subdomains and the like are described in, for example, Canadian patent document No. 2,301,157 (conjugates containing homeodomain of antennapedia) as well as in U.S. Pat. Nos. 5,652,122, 5,670,617, 5,674,980, 5,747,641, and 5,804,604, (conjugates containing amino acids of Tat HIV protein; herpes simplex virus-1 DNA binding protein VP22, a Histidine tag ranging in length from 4 to 30 histidine repeats, or a variation derivative or homologue thereof capable of facilitating uptake of the active cargo moiety by a receptor independent process.

A 16 amino acid region of the third alpha-helix of antennapedia homeodomain has also been shown to enable proteins (made as fusion proteins) to cross cellular membranes (PCT international publication number WO 99/11809 and Canadian application No. 2,301,157. Similarly, HIV Tat protein was shown to be able to cross cellular membranes.

In addition, the transport moiety(ies) can include polypeptides having a basic amino acid rich region covalently linked to an active agent moiety (*e.g.,* intracellular domain-containing fragments inhibitor peptide). As used herein, the term "basic amino acid rich region" relates to a region of a protein with a high content of the basic amino acids such as arginine, histidine, asparagine, glutamine, lysine. A "basic amino acid rich region" may have, for example 15% or more of basic amino acid. In some instance, a "basic amino acid rich region" may have less than 15% of basic amino acids and still function as a transport agent region. In other instances, a basic amino acid region will have 30% or more of basic amino acids.

The transport moiety(ies) may further include a proline rich region. As used herein, the term proline rich region refers to a region of a polypeptide with 5% or more (up to 100%) of proline in its sequence. In some instance, a proline rich region may have between 5% and 15% of prolines. Additionally, a proline rich region refers to a region, of a polypeptide containing more prolines than what is generally observed in naturally occurring proteins (*e.g.,* proteins encoded by the human genome). Proline rich regions of this application can function as a transport agent region.

In one embodiment, the therapeutic peptide described herein can be non-covalently linked to a transduction agent. An example of a non-covalently linked polypeptide transduction agent is the Chariot protein delivery system (See U.S. Patent No. 6,841,535; J Biol Chem 274(35):24941-24946; and Nature Biotec. 19:1173-1176).

In other embodiments, the therapeutic peptides can be expressed in cells being treated using gene therapy to inhibit LAR family signaling or PTPσ signaling. The gene therapy can use a vector including a nucleotide encoding the therapeutic peptides. A "vector" (sometimes referred to as gene delivery or gene transfer "vehicle") refers to a macromolecule or complex of molecules comprising a polynucleotide to be delivered to the cell. The polynucleotide to be delivered may comprise a coding sequence of interest in gene therapy. Vectors include, for example, viral vectors (such as adenoviruses (Ad), adeno-associated viruses (AAV), and retroviruses), liposomes and other lipid-containing complexes, and other macromolecular complexes capable of mediating delivery of a polynucleotide to a target cell.

Vectors can also comprise other components or functionalities that further modulate gene delivery and/or gene expression, or that otherwise provide beneficial properties to the targeted cells. Such other components include, for example, components that influence binding or targeting to cells (including components that mediate cell-type or tissue-specific binding); components that influence uptake of the vector nucleic acid by the cell; components that influence localization of the polynucleotide within the cell after uptake (such as agents mediating nuclear localization); and components that influence expression of the polynucleotide (such as one or more transcriptional regulatory sequences). Such components also might include markers, such as detectable and/or selectable markers that can be used to detect or select for cells that have taken up and are expressing the nucleic acid delivered by the vector. Such components can be provided as a natural feature of the vector (such as the use of certain viral vectors which have components or functionalities mediating binding and uptake), or vectors can be modified to provide such functionalities.

Selectable markers can be positive, negative or bifunctional. Positive selectable markers allow selection for cells carrying the marker, whereas negative selectable markers allow cells carrying the marker to be selectively eliminated. A variety of such marker genes have been described, including bifunctional (*i.e.,* positive/negative) markers (see, *e.g.,* Lupton, S., WO 92/08796, published May 29, 1992; and Lupton, S., WO 94/28143, published Dec. 8, 1994). Such marker genes can provide an added measure of control that can be advantageous in gene therapy contexts. A large variety of such vectors are known in the art and are generally available.

Vectors for use herein but not forming part of the claimed invention include viral vectors, lipid based vectors and other non-viral vectors that are capable of delivering a nucleotide encoding the therapeutic peptides described herein to the target cells. The vector can be a targeted vector, especially a targeted vector that preferentially binds to neurons and. Viral vectors for use in the application but not forming part of the claimed invention can include those that exhibit low toxicity to a target cell and induce production of therapeutically useful quantities of the therapeutic peptide in a cell specific manner.

Examples of viral vectors are those derived from adenovirus (Ad) or adeno-associated virus (AAV). Both human and non-human viral vectors can be used and the recombinant viral vector can be replication-defective in humans. Where the vector is an adenovirus, the vector can comprise a polynucleotide having a promoter operably linked to a gene encoding the therapeutic peptides and is replication-defective in humans.

Other viral vectors that can be used herein but not forming part of the claimed invention include herpes simplex virus
(HSV)-based vectors. HSV vectors deleted of one or more immediate early genes (IE) are advantageous because they are generally non-cytotoxic, persist in a state similar to latency in the target cell, and afford efficient target cell transduction. Recombinant HSV vectors can incorporate approximately 30 kb of heterologous nucleic acid.

Retroviruses, such as C-type retroviruses and lentiviruses, might also be used in the application but do not form part of the claimed invention. For example, retroviral vectors may be based on murine leukemia virus (MLV). See, *e.g.,* Hu and Pathak, Pharmacol. Rev. 52:493-511, 2000 and Fong et al., Crit. Rev. Ther. Drug Carrier Syst. 17:1-60, 2000. MLV-based vectors may contain up to 8 kb of heterologous (therapeutic) DNA in place of the viral genes. The heterologous DNA may include a tissue-specific promoter and a nucleic acid encoding the therapeutic peptide. In methods of delivery to neural cells, it may also encode a ligand to a tissue specific receptor.

Additional retroviral vectors that might be used but do not form part of the claimed invention are replication-defective lentivirus-based vectors, including human immunodeficiency (HIV)-based vectors. See, *e.g.,* Vigna and Naldini, J. Gene Med. 5:308-316, 2000 and Miyoshi et al., J. Virol. 72:8150-8157, 1998. Lentiviral vectors are advantageous in that they are capable of infecting both actively dividing and non-dividing cells.

Lentiviral vectors for use in the application but not forming part of the claimed invention may be derived from human and non-human (including SIV) lentiviruses. Examples of lentiviral vectors include nucleic acid sequences required for vector propagation as well as a tissue-specific promoter operably linked to a therapeutic peptide encoding nucleic acid. These former may include the viral LTRs, a primer binding site, a polypurine tract, att sites, and an encapsidation site.

In some aspects, a lentiviral vector can be employed. Lentiviruses have proven capable of transducing different types of CNS neurons (Azzouz et al., (2002) J Neurosci. 22: 10302-12) and may be used in some embodiments because of their large cloning capacity.

A lentiviral vector may be packaged into any lentiviral capsid. The substitution of one particle protein with another from a different virus is referred to as "pseudotyping". The vector capsid may contain viral envelope proteins from other viruses, including murine leukemia virus (MLV) or vesicular stomatitis virus (VSV). The use of the VSV G-protein yields a high vector titer and results in greater stability of the vector virus particles.

Alphavirus-based vectors, such as those made from semliki forest virus (SFV) and sindbis virus (SIN) might also be used in the application but do not form part of the claimed invention. Use of alphaviruses is described in Lundstrom, K., Intervirology 43:247-257, 2000 and Perri et al., Journal of Virology 74:9802-9807, 2000.

Recombinant, replication-defective alphavirus vectors are advantageous because they are capable of high-level heterologous (therapeutic) gene expression, and can infect a wide target cell range. Alphavirus replicons may be targeted to specific cell types by displaying on their virion surface a functional heterologous ligand or binding domain that would allow selective binding to target cells expressing a cognate binding partner. Alphavirus replicons may establish latency, and therefore long-term heterologous nucleic acid expression in a target cell. The replicons may also exhibit transient heterologous nucleic acid expression in the target cell.

In many of the viral vectors compatible with methods in which the therapeutic agent of the application is for use, more than one promoter can be included in the vector to allow more than one heterologous gene to be expressed by the vector. Further, the vector can comprise a sequence, which encodes a signal peptide or other moiety, which facilitates expression of the therapeutic peptide from the target cell.

To combine advantageous properties of two viral vector systems, hybrid viral vectors may be used to deliver a nucleic acid encoding a therapeutic peptide to a target neuron, cell, or tissue. Standard techniques for the construction of hybrid vectors are well-known to those skilled in the art. Such techniques can be found, for example, in Sambrook, et al., In Molecular Cloning: A laboratory manual. Cold Spring Harbor, N.Y. or any number of laboratory manuals that discuss recombinant DNA technology. Double-stranded AAV genomes in adenoviral capsids containing a combination of AAV and adenoviral ITRs may be used to transduce cells. In another variation, an AAV vector may be placed into a "gutless", "helper-dependent" or "high-capacity" adenoviral vector. Adenovirus/AAV hybrid vectors are discussed in Lieber et al., J. Virol. 73:9314-9324, 1999. Retrovirus/adenovirus hybrid vectors are discussed in Zheng et al., Nature Biotechnol. 18:176-186, 2000. Retroviral genomes contained within an adenovirus may integrate within the target cell genome and effect stable gene expression.

Other nucleotide sequence elements, which facilitate expression of the therapeutic peptide and cloning of the vector are further contemplated. For example, the presence of enhancers upstream of the promoter or terminators downstream of the coding region, for example, can facilitate expression.

In accordance with another non-claimed embodiment, a tissue-specific promoter can be fused to nucleotides encoding the therapeutic peptides described herein. By fusing such tissue specific promoter within the adenoviral construct, transgene expression is limited to a particular tissue. The efficacy of gene expression and degree of specificity provided by tissue specific promoters can be determined, using the recombinant adenoviral system of the present application. Neuron specific promoters, such as the platelet-derived growth factor β-chain (PDGF-β) promoter and vectors, are well known in the art.

In addition to viral vector-based methods, non-viral methods may also be used to introduce a nucleic acid encoding a therapeutic peptide into a target cell. A review of non-viral methods of gene delivery is provided in Nishikawa and Huang, Human Gene Ther. 12:861-870, 2001. An example of a non-viral gene delivery method according to the application employs plasmid DNA to introduce a nucleic acid encoding a therapeutic peptide into a cell. Plasmid-based gene delivery methods are generally known in the art.

Synthetic gene transfer molecules can be designed to form multimolecular aggregates with plasmid DNA. These aggregates can be designed to bind to a target cell. Cationic amphiphiles, including lipopolyamines and cationic lipids, may be used to provide receptor-independent nucleic acid transfer into target cells.

In addition, preformed cationic liposomes or cationic lipids may be mixed with plasmid DNA to generate cell-transfecting complexes. Methods involving cationic lipid formulations are reviewed in Felgner et al., Ann. N.Y. Acad. Sci. 772:126-139, 1995 and Lasic and Templeton, Adv. Drug Delivery Rev. 20:221-266, 1996. For gene delivery, DNA may also be coupled to an amphipathic cationic peptide (Fominaya et al., J. Gene Med. 2:455-464, 2000).

Methods that involve both viral and non-viral based components may be used according to the application but do not form part of the claimed invention. For example, an Epstein Barr virus (EBV)-based plasmid for therapeutic gene delivery is described in Cui et al., Gene Therapy 8:1508-1513, 2001. Additionally, a method involving a DNA/ligand/polycationic adjunct coupled to an adenovirus is described in Curiel, D. T., Nat. Immun. 13:141-164, 1994.

Additionally, the nucleic acid encoding the therapeutic peptides can be introduced into the target cell by transfecting the target cells using electroporation techniques. Electroporation techniques are well known and can be used to facilitate transfection of cells using plasmid DNA.

Vectors that encode the expression of the therapeutic peptides can be delivered *in vivo* to the target cell in the form of an injectable preparation containing pharmaceutically acceptable carrier, such as saline, as necessary. Other pharmaceutical carriers, formulations and dosages can also be used in accordance with the present application.

Where the target cell includes an OL/OPC being treated the vector can be delivered by direct injection into or about the periphery of the OL/OPC at an amount sufficient for the therapeutic peptide to be expressed to a degree, which allows for highly effective therapy. By injecting the vector directly into or about the periphery of the OL/OPC, it is possible to target the vector transfection rather effectively, and to minimize loss of the recombinant vectors. This type of injection enables local transfection of a desired number of cells, especially at a site of injury, thereby maximizing therapeutic efficacy of gene transfer, and minimizing the possibility of an inflammatory response to viral proteins. Other methods of administering the vector to the target cells can be used and will depend on the specific vector employed.

The therapeutic peptide can be expressed for any suitable length of time within the target cell, including transient expression and stable, long-term expression. In one non-claimed aspect of the application, the nucleic acid encoding the therapeutic peptide will be expressed in therapeutic amounts for a defined length of time effective to induce activity and growth of the transfected cells. In another non-claimed aspect of the application, the nucleic acid encoding the therapeutic peptide will be expressed in therapeutic amounts for a defined length of time effective to increase survival rate of OLs/OPCs, enhance OPC migration, differentiation, proliferation and/or maturation, and/or enhance myelination or remyelination.

The therapeutic agents described herein may be modified (*e.g.,* chemically modified). Such modification may be designed to facilitate manipulation or purification of the molecule, to increase solubility of the molecule, to facilitate administration, targeting to the desired location, to increase or decrease half-life. A number of such modifications are known in the art and can be applied by the skilled practitioner.

In the methods of treatment disclosed herein in which the claimed therapeutic agent is for use, a therapeutically effective amount of the therapeutic agent is administered to the subject to treat a myelin related or demyelination related disease or disorder (*e.g.,* MS). In one embodiment, in the method in which the claimed therapeutic agent is for use, a formulation including the therapeutic agent can be administered to the subject in the period from the time of, for example, detection or onset of the myelin related or demyelination related disease or disorder (*e.g.,* MS), to days, weeks, months, and/or years after the detection or onset of the myelin related or demyelination related disease or disorder (*e.g.,* MS).

The therapeutic agents can be delivered to a subject by any suitable route, including, for example, local and/or systemic administration. Systemic administration can include, for example, parenteral administration, such as intramuscular, intravenous, intraarticular, intraarterial, intrathecal, subcutaneous, or intraperitoneal administration. The agent can also be administered orally, transdermally, topically, by inhalation *(e.g.,* intrabronchial, intranasal, oral inhalation or intranasal drops) or rectally. In some embodiments, in the method in which the claimed therapeutic agent is for use, the therapeutic agent can be administered to the subject via intravenous administration using an infusion pump to deliver daily, weekly, or doses of the therapeutic agent.

Desirable features of local administration include achieving effective local concentrations of the therapeutic agent as well as avoiding adverse side effects from systemic administration of the therapeutic agent. In one embodiment, in the method in which the claimed therapeutic agent is for use, the therapeutic agent can be introduced directly into the brain of the subject.

Pharmaceutically acceptable formulations of the therapeutic agent can be suspended in aqueous vehicles and introduced through conventional hypodermic needles or using infusion pumps.

For injection, therapeutic agent can be formulated in liquid solutions, typically in physiologically compatible buffers such as Hank's solution or Ringer's solution. In addition, the therapeutic agent may be formulated in solid form and re-dissolved or suspended immediately prior to use. Lyophilized forms are also included. The injection can be, for example, in the form of a bolus injection or continuous infusion (such as using infusion pumps) of the therapeutic agent.

It will be appreciated that the amount, volume, concentration, and/or dosage of the therapeutic agent that is administered to any one animal or human depends on many factors, including the subject's size, body surface area, age, the particular composition to be administered, sex, time and route of administration, general health, and other drugs being administered concurrently. Specific variations of the above noted amounts, volumes, concentrations, and/or dosages of therapeutic agent can be readily be determined by one skilled in the art using the experimental methods described below.

In some embodiments, in the method in which the claimed therapeutic agent is for use, a therapeutic agent, such as a therapeutic peptide described herein, can be administered locally and/or systemically to a subject in need thereof at a dose or amount of about 0.1 µmol, about 1 µmol, about 5 µmol, about 10 µmol, or more; or about 0.0001 mg/kg, about 0.001 mg/kg, about 0.01 mg/kg, about 0.1 mg/kg, or about 1 mg/kg to about 5 mg/kg or 10 mg/kg of the subject being treated. The therapeutic agent can be administered daily, weekly, biweekly, monthly or less frequently until there is maximal remyelination of the CSPG region.

In another embodiment, in the method in which the claimed therapeutic agent is for use, the therapeutic agent can be administered to a subject systemically by intravenous injection or locally at the site of injury, usually within about 24 hours, about 48 hours, about 100 hours, or about 200 hours or more of when an injury occurs (*e.g.,* within about 6 hours, about 12 hours, or 24 hours, inclusive, of the time of the injury).

In other embodiments, in the method in which the claimed therapeutic agent is for use, a pharmaceutically acceptable formulation used to administer the therapeutic agent(s) can also be formulated to provide sustained delivery of the active compound to a subject. For example, the formulation may deliver the active compound for at least one, two, three, or four weeks, inclusive, following initial administration to the subject. For example, a subject to be treated in accordance with the method described herein in which the claimed therapeutic agent is for use can be treated with the therapeutic agent for at least 30 days (either by repeated administration or by use of a sustained delivery system, or both).

Approaches for sustained delivery include use of a polymeric capsule, a mini pump to deliver the formulation, a biodegradable implant, or implanted transgenic autologous cells (see U.S. Patent No. 6,214,622). Implantable infusion pump systems (*e.g.,* INFUSAID pumps (Towanda, PA)); see Zierski et al., 1988; Kanoff, 1994) and osmotic pumps (sold by Alza Corporation) are available commercially and otherwise known in the art. Another mode of administration is via an implantable, externally programmable infusion pump. Infusion pump systems and reservoir systems are also described in, *e.g.,* U.S. Patents No. 5,368,562 and No. 4,731,058.

Vectors encoding the therapeutic peptides can often be administered less frequently than other types of therapeutics. For example, an effective amount of such a vector can range from about 0.01 mg/kg to about 5 or 10 mg/kg, inclusive; administered daily, weekly, biweekly, monthly or less frequently.

The ability to deliver or express the therapeutic peptides allows for cell activity modulation in a number of different cell types. The therapeutic peptides can be expressed, for example, in an OL/OPC via specific promoters.

The pharmaceutical compositions can be administered to any subject that can experience the beneficial effects of the OPC migration, differentiation, proliferation and/or maturation. Foremost among such animals are humans, although the present invention is not intended to be so limited.

The therapeutic agent can be used in a method of treating a subject by OPC migration, differentiation, proliferation and/or maturation in the subject. The method can include administering to the subject in need thereof a therapeutically effective amount of therapeutic agent described herein. The therapeutically effective amount can include an amount (dose) effective in treating a subject, having, for example, a neurodegenerative disease (*e.g.* multiple sclerosis).

In some embodiments, in the method in which the claimed therapeutic agent is for use, the therapeutic agents described herein may be administered in an amount effective to promote survival of OLs/OPCs in a subject by an increase in the number of surviving OLs/OPCs of at least 5%, 10%, 20%, 25%, 30%, 40%, 50%, 60%, 70%, 75%, 80%, 85%, 90%, 95%, 100%, 110%, 120%, 130%, 140%, 150%, 160%, 170%, 180%, 190%, 200%, 250%, 300%, 350%, 400%, 450%, 500%, 550%, 600%, 650%, 700%, 750%, 800%, 850%, 900%, 950%, or 1000% as compared to the number of surviving OLs/OPCs in an untreated OLs/OPCs or subject.

In some embodiments, in the method in which the claimed therapeutic agent is for use, the therapeutic agent described herein may be administered in an amount effective enhance generation of OLs in the subject's central nervous system by an increase in the amount of OL generation of at least 5%, 10%, 20%, 25%, 30%, 40%, 50%, 60%, 70%, 75%, 80%, 85%, 90%, 95%, 100%, 110%, 120%, 130%, 140%, 150%, 160%, 170%, 180%, 190%, 200%, 250%, 300%, 350%, 400%, 450%, 500%, 550%, 600%, 650%, 700%, 750%, 800%, 850%, 900%, 950%, or 1000% as compared to the amount of OL generation in untreated OPCs or subject.

In some embodiments, in the method in which the claimed therapeutic agent is for use, the therapeutic agent described herein may be administered in an amount effective to induce OPC differentiation in the subject's central nervous system by an increase in the amount of OPC differentiation of at least 5%, 10%, 20%, 25%, 30%, 40%, 50%, 60%, 70%, 75%, 80%, 85%, 90%, 95%, 100%, 110%, 120%, 130%, 140%, 150%, 160%, 170%, 180%, 190%, 200%, 250%, 300%, 350%, 400%, 450%, 500%, 550%, 600%, 650%, 700%, 750%, 800%, 850%, 900%, 950%, or 1000% as compared to the amount of OPC differentiation in untreated OPCs or subject.

In some embodiments, in the method in which the claimed therapeutic agent is for use, the therapeutic agents can be administered to a subject to treat neurodegenerative myelin related disorder. Myelin related disorders can include any disease, condition, or disorder related to demyelination, insufficient myelination and remyelination, or dysmyelination in a subject. A myelin related disorder can arise from a myelination related disorder or demyelination resulting from a variety of neurotoxic insults. "Demyelination" as used herein, refers to the act of demyelinating, or the loss of the myelin sheath insulating the nerves, and is the hallmark of some neurodegenerative autoimmune diseases, including multiple sclerosis, transverse myelitis, chronic inflammatory demyelinating polyneuropathy, leukodystrophies, and Guillain-Barre Syndrome. Leukodystrophies are caused by inherited enzyme deficiencies, which cause abnormal formation, destruction, and/or abnormal turnover of myelin sheaths within the CNS white matter. Both acquired and inherited myelin disorders share a poor prognosis leading to major disability. Thus, some embodiments can include a therapeutic agent for use in methods for the treatment of neurodegenerative autoimmune diseases in a subject.

Myelin related diseases or disorders which may be treated or ameliorated by the methods described herein in which the claimed therapeutic agent is for use can include diseases, disorders or injuries which relate to dysmyelination or demyelination in a subject's CNS or peripheral nervous system. Such diseases include, but are not limited to, diseases and disorders in which the myelin which surrounds the neuron is either absent, incomplete, not formed properly, or is deteriorating. Such disease include, but are not limited to, myelinoclastic disorders, such as multiple sclerosis (MS), Devic's disease, and inflammatory demyelinating diseases, leukodystrophic disorders, such as leukoencephalopathies, leukodystrophies, *e.g*., adrenomyeloneuropathy, cerebrotendineous xanthomatosis, Krabbe disease, Alexander's disease, and Pelizaeus Merzbacher disease (PMD), and demyelinating diseases of the peripheral nervous system, such as Guillian-Barre syndrome and Charcot-Marie-Tooth disease.

In some embodiments, myelin related diseases or disorders which may be treated or ameliorated by the methods described herein in which the claimed therapeutic agent is for use include leukodystrophies. Leukodystrophies are a group of progressive, metabolic, genetic diseases that affect the brain, spinal cord and often the peripheral nerves. Each type of leukodystrophy is caused by a specific gene abnormality that leads to abnormal development or destruction of the myelin sheath of the brain. Each type of leukodystrophy affects a different part of the myelin sheath, leading to a range of neurological problems. Examples of leukodystrophies, which may be treated or ameliorated by the methods described herein in which the claimed therapeutic agent is for use include adult-onset autosomal dominant leukodystrophy (ADLD), Aicardi-Goutieres syndrome, Alexander disease, CADASIL, Canavan disease, CARASIL, cerebrotendionous xanthomatosis, childhood ataxia and cerebral ypomyelination (CACH)/ vanishing white matter disease (VWMD), Fabry disease, fucosidosis, GM1 gangliosidosis, Krabbe disease, L-2-hydroxyglutaric aciduria, megalencephalic leukoencephalopathy with subcortical cysts, metachromatic leukodystrophy, multiple sulfatase deficiency, Pelizaeus-Merzbacher disease (PMD), Pol III-related leukodystrophies, Refsum disease, salla disease (free sialic acid storage disease), Sjogren-Larsson syndrome, X-linked adrenoleukodystrophy, and Zellweger syndrome spectrum disorders.

Myelin related diseases or disorders which may be treated or ameliorated by the methods described herein in which the claimed therapeutic agent is for use can include a disease or disorder characterized by a myelin deficiency. Insufficient myelination in the central nervous system has been implicated in a wide array of neurological disorders. Among these are forms of cerebral palsy in which a congenital deficit in forebrain myelination in children with periventricular leukomalacia, contributes to neurological morbidity (Goldman et al., 2008) Goldman, S. A., Schanz, S., and Windrem, M. S. (2008). Stem cell-based strategies for treating pediatric disorders of myelin. Hum Mol Genet. 17, R76-83. At the other end of the age spectrum, myelin loss and ineffective repair may contribute to the decline in cognitive function associated with senescence (Kohama et al., 2011) Kohama, S. G., Rosene, D. L., and Sherman, L. S. (2011) Age (Dordr). Age-related changes in human and non-human primate white matter: from myelination disturbances to cognitive decline. Therefore, it is contemplated that effective agents and methods of enhancing myelination and/or remyelination may have substantial therapeutic benefits in halting disease progression and restoring function in MS and in a wide array of neurological disorders.

One particular aspect contemplates a therapeutic agent for use in the treatment of multiple sclerosis in a subject. The method in which the claimed therapeutic agent is for use includes administering to the subject a therapeutically effective amount of the therapeutic agent described herein. Multiple sclerosis (MS) is the most common demyelinating disease. In multiple sclerosis, the body's failure to repair myelin is thought to lead to nerve damage, causing multiple sclerosis associated symptoms and increasing disability. The demyelination observed in MS is not always permanent and remyelination has been documented in early stages of the disease. It is contemplated that methods described herein in which the claimed therapeutic agent is for use can promote OPC differentiation in a subject, therefore leading to endogenous remyelination.

Another particular aspect contemplates a therapeutic agent for use in the treatment of a genetic myelin disorder which results from the loss of myelin (demyelination) in a subject. The method in which the claimed therapeutic agent is for use includes administering to the subject a therapeutically effective amount of the therapeutic agents described herein. In certain embodiments, the genetic myelin disorder is a leukodystrophy such as, but not limited to Pelizaeus Merzbacher Disease (PMD)

Another strategy for treating a subject suffering from a neurodegenerative disease or disorder is to administer a therapeutically effective amount of the therapeutic agent described herein along with a therapeutically effective amount of additional oligodendrocyte differentiation and/or proliferation inducing agent(s) and/or anti-neurodegenerative disease agent. Examples of anti-neurodegenerative disease agents include L-dopa, cholinesterase inhibitors, anticholinergics, dopamine agonists, steroids, and immunomodulators including interferons, monoclonal antibodies, and glatiramer acetate.

Therefore, in some embodiments, in the method in which the claimed therapeutic agent is for use, the therapeutic agent described herein can be administered as part of a combination therapy with adjunctive therapies for treating neurodegenerative and myelin related disorders.

The phrase "combination therapy" embraces the administration of the therapeutic agent, which inhibits or reduces one or more of the activity, signaling, and/or function of PTPσ, and an additional therapeutic agent as part of a specific treatment regimen intended to provide a beneficial effect from the co-action of these therapeutic agents. When administered as a combination, the therapeutic agent, which inhibits or reduces one or more of the activity, signaling, and/or function of PTPσ, and the additional therapeutic agent can be formulated as separate compositions. Administration of these therapeutic agents in combination typically is carried out over a defined time period (usually minutes, hours, days or weeks depending upon the combination selected).

"Combination therapy" is intended to embrace administration of these therapeutic agents in a sequential manner, that is, wherein each therapeutic agent is administered at a different time, as well as administration of these therapeutic agents, or at least two of the therapeutic agents, in a substantially simultaneous manner. Substantially simultaneous administration can be accomplished, for example, by administering to the subject a single capsule having a fixed ratio of each therapeutic agent or in multiple, single capsules for each of the therapeutic agents. Sequential or substantially simultaneous administration of each therapeutic agent can be effected by any appropriate route including, but not limited to, oral routes, intravenous routes, intramuscular routes, and direct absorption through mucous membrane tissues. The therapeutic agents can be administered by the same route or by different routes. For example, a first therapeutic agent of the combination selected may be administered by intravenous injection while the other therapeutic agents of the combination may be administered orally. Alternatively, for example, all therapeutic agents may be administered orally or all therapeutic agents may be administered by intravenous injection. The sequence in which the therapeutic agents are administered is not narrowly critical. "Combination therapy" also can embrace the administration of the therapeutic agents as described above in further combination with other biologically active ingredients (such as, but not limited to, a second and different therapeutic agent) and non-drug therapies (*e.g.,* surgery).

In another embodiment, in the method in which the claimed therapeutic agent is for use, the additional therapeutic agent administered in a combination therapy with the therapeutic agent, which inhibits or reduces one or more of the activity, signaling, and/or function of PTPσ, described herein, can include at least one anti-neurodegenerative agent such as but not limited to, an immunotherapeutic agent.

An immunotherapeutic agent for use in the methods in which the claimed therapeutic agent is for use can include therapies which target the immune component of the disease and/or the acute inflammatory response evidenced during an acute attack in remitting-relapsing multiple sclerosis. Examples include, but are not limited to immunomodulators such as interferon beta-la and beta-lb (Avonex and Betaseron respectively), natalizumab (Copaxone) natalizumab (Tysabri), glatiramer acetate (Copaxone) or mitoxantrone. In other embodiments, in the methods in which the claimed therapeutic agent is for use, the therapeutic agents can be administered to a subject that does not have, and/or is not suspected of having, a myelin related disorder in order to enhance or promote a myelin dependent process. In some embodiments, in the methods in which the claimed therapeutic agent is for use, the therapeutic agents described herein can be administered to a subject to promote myelination of CNS neurons in order to enhance cognition, which is known to be a myelin dependent process, in cognitive healthy subjects. In certain embodiments, in the methods in which the claimed therapeutic agent is for use, the therapeutic agents described herein can be administered in combination with cognitive enhancing (nootropic) agents. Examples of agents include any drugs, supplements, or other substances that improve cognitive function, particularly executive functions, memory, creativity, or motivation, in healthy individuals. Non limiting examples include racetams (*e.g.,* piracetam, oxiracetam, and aniracetam), nutraceuticals *(e.g.,* bacopa monnieri, panax ginseng, ginko biloba, and GABA), stimulants (*e.g.,* amphetamine pharmaceuticals, methylphenidate, eugeroics, xanthines, and nicotine), L-Theanine, Tolcapone, Levodopa, Atomoxetine, and Desipramine.

The following is a discussion of a specific and illustrative embodiment of the present disclosure. In this discussion we show that intracellular sigma peptide (ISP) treatment promotes functional recovery in a variety of demyelinating models. ISP treatment overcomes the CSPG barrier to promote remyelination and functional recovery revealing the critical role of receptor protein tyrosine phosphatases (RPTPs) in the pathobiology of multiple sclerosis (MS). We also show a novel mechanism by which ISP modulation of PTPσ dramatically increases protease activity in the OPCs themselves. In turn, enhanced enzyme release selectively degrades CSPGs which further helps to augment their migration into and differentiation within previously scarred territories.

### Example

### Materials and Methods

### Animals

All animal care and animal procedures were approved by the Institutional Animal Care and Use Committee of Case Western Reserve University School of Medicine. Wild-type C57BL/6 mice were purchased from the Jackson Laboratory (Stock No. 000664) and housed at Animal Research Center of Case Western Reserve University, Mice were maintained with a 12-h light/dark cycle. Both male and female mice were included in this study.

### Experimental autoimmune encephalomyelitis (EAE) model

For induction of EAE, C57BL6/J female mice at 10-week-old of age were immunized with MOG₃₅₋₅₅ together with complete Freund's adjuvant emulsion (Hooke Laboratories, MOG₃₅₋₅₅ EAE Induction kit, EK-2110) according to the manufacturers' instruction. Using the EAE Induction Kit result in 98% successful disease induction. All EAE animals were monitored daily and scored using a clinical scale from 0 to 5 (0: no abnormality; 1: limp tail; 2: limp tail and hind legs weakness; 3: limp tail and complete paralysis of hind legs; 4, hind leg and partial front leg paralysis; 5: moribund. Once EAE mice scored for 1 or 3, they were randomly recruited into treatment group and vehicle group. For treatment group, 5-7 mice were given daily intraperitoneal injections of ISP (20 µg/day), or 5% DMSO in saline, 100 µl) for Vehicle group. Experiments were blinded and animals were scored daily. For ISP Onset: ISP treatment was given at onset of sickness scored by clinical scale. For ISP Peak: ISP treatment was given at peak of sickness scored by clinical scale

### Lysolecithin (LPC)-induced focal demyelination in mice

12-week-old C57BL/6 male mice were anaesthetized using isoflurane and a laminectomy was performed. 1.5 µl of 1% LPC was infused into the dorsal column between T11 and T12 spinal cord at a rate of 0.25 µl/min. The needle was removed after a delay of 5 min to minimize back flow and the lesion closed. Started at 24h post-surgery, mice were treated daily with ISP (20 µg/day) or vehicle (5% DMSO in saline, 100 µl) by subcutaneous injection near the injury site. The mice were euthanized at day 7, 14 and 21 after the laminectomy separately. Spinal cords were dissected for further western blot, histology and ultrastructural analysis. Control animals received an equivalent injection of saline, and tissues were collected according to the same paradigm. For the second injection of the MMP-2 inhibitor OA-Hy (10 µg/1.5 µl, 444244, Calbiochem), lentiviral particles expressing shRNA targeting mouse MMP-2 (1 µl, LPP-MSH027657-LVRU6GP, GeneCopoeia) or 0.9% saline, animals were anesthetized at 1 d (for lentiviral particles) or 4 d (for OA-Hy) after LPC lesion and OA-Hy or lentiviral particles was delivered to the same area using the above paradigm. Animals were allowed to recover and sacrificed at 14 days post lesion (dpl) or 18 dpl. Lesion sizes were determined by staining of serial sections with eriochrome cyanine staining.

### LPC-induced demyelination in mouse cerebellar slice cultures

We carried out the cerebellar slice culture method as described previously (Zhang, H., et al. Central nervous system remyelination in culture - A tool for multiple sclerosis research. Experimental Neurology 230, 138-148 (2011)). Briefly, 300 µm-thick cerebellar slices were cut from P10-12 mouse cerebellum using a Leica vibrating microtome (Leica, VT1000S) and cultured in medium containing 50% basal medium eagle medium, 25% Heat-inactivated horse serum, 25% Hank's solution, 2.5% glucose, 1% glutamine and penicillin-streptomycin. After 4 days *in vitro* (DIV), 0.5 mg/ml LPC were added for 17-18h to induce demyelination. Slices were then incubated with 2.5 µM ISP for 8 days. For GM6001 experiments, 25 µM GM6001 (Tocris) and/or 2.5 µM ISP or SISP was incubated for 9 days. Remyelination was examined by semi-quantitative Western blot of MBP and immunofluorescence stain of MBP and NeuF200.

### Immunostaining of cultured slices

Slices were fixed with 4% PFA, delipidated, and washed three times in PBS, blocked in PBS containing 0.1% Triton X-100 and 5% normal goat serum and incubated with anti-MBP (SMI-99P, Covance, 1:300), and anti-NeuF200 (N4142, Sigma, 1:250) antibodies overnight at 4°C. The slices were then washed in PBS and incubated in Alexa Fluor-conjugated secondary antibodies (1:500, invitrogen) for 2h. Slices were mounted in Vectashield mounting medium with DAPI (Vector Laboratories) and analyzed using Leica DFC500 fluorescence microscope.

### Purified mouse Oligodendrocyte Progenitor Cell (OPC) cultures

OPCs were prepared from newborn C57BL/6 mice as described previously (Luo, F., et al. The Activators of Cyclin-Dependent Kinase 5 p35 and p39 Are Essential for Oligodendrocyte Maturation, Process Formation, and Myelination. J. Neurosci. 36, 3024-3037 (2016).). Cell culture plates were pre-coated with IgM (10 µg/ml, Millipore) in 50 mM Tris-HCl and followed primary mouse antibody A2B5. Dissociated cells were incubated in the pre-coated culture dishes for 30 min at 37°C and then non-adherent cells were gently removed. A2B5+ OPCs were released by 0.05% trypsin in DMEM at a purify of ~96%. Purified OPC cells were expanded in DMEM/F12 medium supplemented with N2, 20ng/ml PDGF, 20ng/mlFGF, 5ng/mlNT-3, 10ng/ml CNTF, Glutamine (200 mM).

### Conditioned media (CM) protease activity assay

Around 1x10⁶ OPCs were plated per well on PLL, 1 µg/mL laminin, and 2 µg/mL aggrecan coated 6-well plates and treated with vehicle, 2.5 µM ISP or SISP for 2 days at 37°C. CM was collected, cell-strained, and placed on ice until assayed. ThermoFisher Protease Assay Kit (E66383, EnzChek, ThermoFisher) was used to assay protease activity. 1x of the EnzChek mixture was mixed 1:1 with the CM from each group and incubated at RT O/N with gentle shaking. 3 replicates were performed for each sample. Samples were analyzed using a spectrophotometer at 502/528nm excitation and emission to assess cleaved and fluorescing casein. Fluorescence units reported have been blanked with EnzChek and cell culture media mixture.

### CSPG gradient crossing assay and gradient quantification

CSPG gradients were prepared as described previously (Tom, V. J., et al. Studies on the Development and Behavior of the Dystrophic Growth Cone, the Hallmark of Regeneration Failure, in an In vitro Model of the Glial Scar and after Spinal Cord Injury.). 24-well glass coverslip were coated with poly-L-lysine and nitrocellulose, and a mixture of 700 µg/mL aggrecan (A1960 Sigma) and 10 µg/mL laminin (11243217001 Sigma) spotted on the coated coverslip. After drying, coated coverslips were then incubated with laminin at 37°C for 3h. Purified OPCs were plated at a density of 10,000/coverslip and cultured in DMEM/F12 medium containing with N2, PDGF (20ng/ml), FGF (20ng/ml), NT-3 (5ng/ml), CNTF (10ng/ml), Glutamine (200 mM). Coverslips were stained with CS-56 (C8035, Sigma, 1:500) and O4 antibodies (Hybridoma Core Cleveland Clinic, 1:10). O4-positive cells crossing the aggrecan border were counted for each spot. For CSPG gradient quantification, 1x10⁶ OPCs were plated per well on PLL, 1 µg/mL laminin, and 2 µg/mL aggrecan coated 6-well plates. OPCs treated with vehicle, 2.5 µM ISP, or 2.5 µM SISP for 2 days at 37°C. CM was harvested and incubated with freshly made spots. Spots were incubated with CM for 2 days at 37°C then stained with CS-56 and laminin (L9393, Sigma, 1:1000) antibodies and consistently imaged. Using ImageJ software (NIH), pixel intensities of the CS-56 or laminin spot rims were quantified using the same ROI. For cultured spinal cord explant, the spinal cords of cervical and thoracic of P1 mouse pups were chopped into 1-2 mm tissue pieces and transferred to the coverslip. Explants were cultured in DMEM/F12 medium with 15% FBS (Hyclone), 10ng/ml PDGF (Sigma) and N2 supplement (Invitrogen). For ISP treatment, 2.5uM ISP was added to the media at the time of plating. 25 µM GM6001 (2983, Tocris), 100nM MMP-2 inhibitor (444244, Calbiochem), and/or 2.5 µM ISP or SISP was used.

### Peptide Sequences

Peptides were purchased from GenScript or CS-Bio in 1mg lyophylized quantities that were diluted to 2.5mM in dH₂0 and aliquoted at -20°C when ready for use as previously described (Lang, B. T. et al. Modulation of the proteoglycan receptor PTPsigma promotes recovery after spinal cord injury. Nature 518, 404-408 (2015)).
Intracellular Sigma Peptide (ISP):
   GRKKRRQRRRCDMAEHMERLKANDSLKLSQEYESI (SEQ ID NO: 61)
Scrambled ISP (SISP):
   GRKKRRQRRRCIREDDSLMLYALAQEKKESNMHES (SEQ ID NO: 62)

### Western blot analysis of aggrecan and laminin

CM was harvested from 1x10⁶ OPCs incubated on PLL, 1 µg/mL laminin, and 2 µg/mL aggrecan coated 6-well plates. OPCs were treated with vehicle, 2.5 µM ISP, or 2.5 µM SISP in conjunction with 10 µg/mL Exo1 (ab120292, Abcam), or 25 µM GM6001 (364205, Calbiochem) for 2 days at 37°C. 100 µL CM of each cell-strained group was incubated with 20 µg/mL aggrecan and/or 10 µg/mL laminin for 2 hours in 1mL Eppendorf tubes at 37°C. As a positive control, OPC media was incubated with aggrecan and incubated in the same fashion. Western blots were then performed as described below with incubation against CS-56 and/or laminin antibodies.

### Western blot analysis of OPC lysate or CM

To assess MMP-2 or 10 in OPC CM or lysates, OPCs were incubated on precoated PLL, aggrecan, and laminin as described above. OPCs were treated with vehicle, 2.5 µM ISP or SISP for 2 days at 37°C. CM was then harvested and concentrated using Milipore Ultracel YM-C centrifugal filter units at max speed (Eppendorf Centrifuge 5415D) for 30 minutes at 4°C. 50 µg of concentrated CM was loaded into each lane. OPC lysates were assessed using western blot techniques described below with 20 µg protein loaded from each group.

### Western blot analysis

Tissue samples or cerebellar slices or purified OPC cells were homogenized with RIPA lysis buffer and protein concentration was determined by Pierce BCA protein assay kit according to the manufacture instruction (Thermo Fisher). Then, equal amounts of protein were loaded to 15% SDS-PAGE gels, and electrophoretically transferred to PVDF membranes (Millipore). The membranes were blocked in 0.1% TPBS buffer with 5% non-fat milk for 1h at room temperature and probed with indicated primary antibodies overnight at 4°C and followed by secondary antibody conjugated to horseradish peroxidase (HRP). The following primary antibodies were used: MBP (SMI-99P, Covance, 1: 1000), CS-56 (C8035, Sigma, 1:1000), Laminin (L9393, Sigma, 1:1000), GAPDH (AF5718, R and D Systems, 1: 1000), MMP-2 (AF1488, R and D Systems, 1: 1000), MMP-10 (MAB910, R and D Systems, 1:1000), and β-actin (sc-47778, Santa Cruz, 1:1000). Enhanced chemiluminescence was performed with a West Pico Kit (Thermo Fisher) and detected by FluorChem E system (ProteinSimple, USA). The density of bands was quantified using ImageJ software (NIH).

### Gelatin Zymography

CM was collected from 1x10⁶ OPCs incubated on PLL, 1 µg/mL laminin, and 2 µg/mL aggrecan coated 6-well plates. OPCs were treated with vehicle, 2.5 µM ISP or SISP for 2 days at 37°C. CM was concentrated using Milipore Ultracel YM-3 centrifugal filter units as previously described. 40 µg undenatured protein from concentrated CM or 25 ng of recombinant MMP-2 (PF023, Milipore) and MMP-9 (PF140, Milipore) was loaded with 1x laemmli buffer (1610747, Bio-Rad) onto 10% gelatin zymograms (1611167, Bio-Rad) and ran at 100mV for 1.5 hours on ice. Zymograms were then gently shaken with 1x renaturing buffer (1610765, Bio-Rad) for 30 minutes at RT then incubated with 1x developing buffer (1610766, Bio-Rad) O/N at 37°C. Developed Zymograms were then gently washed with dH₂0 and incubated O/N with 0.1% Coomassie Blue dye (27816, Sigma). Following washes with destaining buffer (40% MeOH, 10% Acetic Acid), Zymograms were imaged and the amount of gelatin degradation was assessed using ImageJ in a method described in the western blot section.

### Protease Array Screen

OPCs were cultured on 6-well plates for 4div with vehicle control or 2.5 µM ISP treatment. Conditioned media was collected from each group and cell-strained before incubation with blot array provided in R & D Systems Protease Array Kit (ARY025). Instructions from kit were followed with overnight incubation of collected media at 4°C. Control and ISP blots were developed together with the same exposure time and pixel intensities of array was assess using ImageJ (NIH).

### Luxol Fast Blue (LFB) myelin staining and quantification

LFB staining was performed according to the manufacturer's instruction (#26681, Electron Microscopy Sciences). For spinal cord sections, 20 µm coronal sections were incubated in LFB solution in 56°C overnight and then rinsed sequentially with 95% alcohol and distilled water. The sections were then in 0.1% lithium carbonate solution and followed dehydration with a series of gradated ethanol, cleared with Histoclear and mounted. A set of serial matched sections were imaged and analyzed. Images (5 to 6 sections/animal) were captured under light microscope. The demyelinated areas (lack of LFB staining) were quantified using ImageJ software. For EAE sections, demyelinated areas were measured and represented as a percentage of total area of spinal cord. For sections of LPC model, lesion volumes were calculated by the lesion area from serial sections throughout the entire lesion based on the equation for volume of cylinder (V=lesion area x length of lesion).

### Immunocytochemistry

For MMP-2/O4 staining, OPCs were plated onto 24-well coverslips that were precoated with PLL, 1 µg/mL laminin, and 2 µg/mL aggrecan and incubated with vehicle or 2.5 µM ISP for 2 days at 37°C. Cultured OPC or OL cells were fixed in 4% PFA and followed blocking in PBST solution (10% normal goat serum and 0.2% Triton -X100 in PBS). Diluted primary antibodies were incubated with samples overnight at 4°C and followed by appropriate secondary antibody goat anti-mouse or anti-rabbit IgM or IgG conjugated with Alexa Fluor 488 or 594 (1:500, Invitrogen). The following primary antibodies were used: PDGFRα(XX), O4 (Hybridoma Core, Cleveland Clinic), MBP (SMI-99P, Covance, 1:300), MMP-2 (R and D Systems, 1:500), and CS-56 (C8035, Sigma, 1:250). Cells were mounted with Vecta Shield mounting medium with DAPI (Vector Laboratories).

### TUNEL, Ki-67 immunocytochemistry and quantification

To assess proliferation, OPCs were plated on 24-well coverslips that were precoated with PLL, 1 µg/mL laminin, and 2 µg/mL aggrecan. OPCs were treated with vehicle, 2.5 µM ISP or SISP immediately upon plating for 2 days at 37°C. Coverslips were fixed and stained using the same method described in the immunocytochemistry section with Ki-67 (550609, BD Pharmingen, 1:500) and O4 (1:10). Coverslips were imaged and counted. To assess apoptosis, OPCs were cultured in the same fashion and incubated with vehicle or 1 µg/mL LPC for 2 hours at 2 days following vehicle, 2.5 µM ISP or SISP incubation. Coverslips were then fixed with PFA and methanol then stained using APO-BrdU TUNEL assay kit (A23210, ThermoFisher) and guidelines with an O/N incubation of the primary antibody at RT. Coverslips were additionally costained with DAPI (D9542, Sigma, 1:10,000) then imaged and counted.

### Immunohistochemistry

Mice were anesthetized with avertin and perfusion with PBS and 4% paraformaldehyde (PFA). Brain or spinal cord were dissected and post-fixed in 4%PFA overnight at 4°C and equilibrated in 20% sucrose. 20 µm-thick sections were pretreated with Reveal Decloaker Solution (RV1000M, Biocare Medical) for antigen retrieval according to the manufacturer's instructions. After blocking, sections were incubated with primary antibodies overnight at 4°C and followed by appropriate secondary antibodies conjugated with Alexa fluorescence 488 or 594. The following primary antibodies were used: MBP (SMI-99P, Covance, 1:300), NeuF200 (N4142, Sigma, 1:250), CS-56 (C8035, Sigma, 1:250), CAT301 (MAB5284, Millipore, 1:250), versican (AB1032, Millipore, 1:250), Iba1 (019-19741, WAKO, 1:250), GFAP (MAB360, Millipore, 1:250), Olig2 (AB9610, Millipore, 1:250), CC1 (OP80, Millipore, 1:250). For each staining, at least 3 individual animals/group were examined and images were captured with a Leica DFC500 fluorescence microscope. Staining was quantified using Image J software (US National Instisutes of Health, USA). Fluorescence intensity was calculated as percentages of the mean value of the naive controls.

### Tissue preparation for electron microscopy (EM) analysis

For ultrastructural analyses of myelination, anesthetized animals were perfused with 2% glutaraldehyde/4% paraformaldehyde in 0.1 M sodium carcodylate buffer, PH7.4 (Electron Microscopy Sciences). Lesioned areas of the LPC or EAE-induced spinal cords from ISP-treated or control animals were dissected and post-fixed in 1% OsO4 for 2hrs. Coronal sections (500 µm) of spinal cord or brain containing corpus callosum were prepared (Leica, Vibratome), dehydrated, stained with saturated uranyl acetate and embedded in a Poly/Bed812 resin (Polysciences Inc.). The 1 µm-thick sections were cut and stained with toluidine blue, and matched areas were selected for EM analysis. For ultrastructure analysis, ultrathin sections (0.1 µm) were cut and visualized using an electron microscope (JEOL100CX) at 80kV. G-ratios were calculated from at least 50-100 randomly selected myelinated axons by measuring the myelin thickness of the inner and outer diameter of the myelin sheath.

### shRNA knock down of MMP-2

MMP-2 knock down was mediated through lentiviral particles expressing shRNA targeting mouse MMP2 driven by the U6 promoter (LPP-MSH027657-LVRU6GP, GeneCopoeia). Lentviral particles for shRNA scrambled constructs (LPP-CSHCTR001-LVRU6GP-100-C, GeneCopoeia) were used as the corresponding controls. OPC cultures were infected for at least 48 hours before experiments at the multiplicity of infection (MOI) of 1. Constructs were validated using western blots analysis of infected OPC cultures for MMP-2 (AB191677, Sigma, 1:500).

### Statistical analysis

All data analyses were performed using GraphPad Prism 6.00. Data are shown as mean ± SEM. p<0.05 is deemed statistically significant. Statistical analysis was performed by two-tailed unpaired Student's t tests, one-way or two-way ANOVA with post-hoc analysis by Tukey's multiple comparison test, Dunnett's multiple comparison test, or Sidak's multiple comparison test. Quantifications were performed in a blinded fashion. No statistical tests were used to predetermine sample sizes, but our sample sizes are similar to those generally employed in the field. Data distribution was assumed to be normal, but this was not formally tested. All experiments were performed at least three times independently.

### Results

### Increased CSPGs and receptor PTPσ expression in lesions of EAE and LPC demyelinating MS mouse models

We characterized CSPG expression in demyelinating lesions of MOG₃₅₋₅₅-induced chronic progressive EAE and LPC-induced acute focal demyelination. Demyelinated EAE and LPC lesions in the white matter of the spinal cord were visualized with Luxol Fast Blue (LFB) myelin staining (Figs. 9A-9D). As expected, LFB staining decreased in the lesions of both models. Immunostaining of sections of spinal cord tissue revealed upregulated CSPG expression in demyelinating lesions of EAE- and LPC-afflicted animals compared to vehicle controls (Figs. 9A-9D). Furthermore, CSPG upregulation progressively increased in the EAE-lesioned spinal cord from 28 to 41 days after immunization (Fig. 9A). Tissue sections collected from animals at 7 and 14 days post-LPC injection in the dorsal spinal cord (Figs. 9C and 9D) similarly showed increased production of CSPGs in demyelinating lesions. Increased CSPGs in focally demyelinated areas led us to hypothesize that CSPGs negatively influence OPCs in lesion sites through PTPσ signaling, which may ultimately affect their ability to remyelinate the cord.

CSPGs are known to signal through the receptors PTPσ, LAR, and the Nogo receptors 1 and 3. We used a previously published RNA-sequencing transcriptome database of mouse cerebral cortex to search for the gene expressions of PTPRS (PTPσ), PTPRF (LAR), PTPRD (PTPδ), and RTN4R (Nogo receptors) during OPC development. PTPRS gene transcripts (FPKM) were the most abundant type of CSPG receptors in developing OPCs (Fig. 10B). Immunostained OPCs/OLs cells derived from wild type mouse pup brains (postnatal day 1-2) revealed that PTPσ was expressed in the somata and processes of immature Olig2⁺ and mature CC1⁺ or myelin basic protein (MBP)⁺ cells (Figs. 10A and 10C). Western blot analyses also indicated an upregulation of PTPσ in the lesioned spinal cord of EAE- or LPC-induced demyelinating models at day 28 (EAE) and day 7 (LPC) post injections (Fig. 10D). In EAE-induced animals, double-immunostaining was also performed with antibodies against PTPσ and the OPC marker, Olig2, to reveal increased PTPσ co-labeled with Olig2⁺ OPCs in demyelinating lesions (Fig. 10E). These findings suggest that PTPσ is expressed and upregulated in cells of oligodendrocyte lineages following EAE or LPC induced disease, and that this receptor presents a tractable target to study the effects of CSPGs and/or receptor manipulations in MS models.

### Modulation of PTPσ with ISP promotes functional recovery and remyelination in an EAE animal model

We next tested Intracellular Sigma Peptide (ISP) in an EAE mouse model, which recapitulates chronic progressive demyelination disease processes. Following MOG₃₅₋₅₅ immunization, animals received intraperitoneal ISP injections (20µg/mouse, daily) for 41 days at the beginning (EAE ISP Onset) or the peak of sickness (EAE ISP Peak) determined by clinical scoring (Fig. 1A). The control group was injected with 5% DMSO vehicle in parallel. Functional recovery was initially observed in the Onset group after ~10-12 days of ISP administration (i.e. day 23 post immunization). ISP improved clinical scores from 3.5-4 (severe paralysis) to 2-1.5 (limp tail and hind limb weakness). After 20-22 days of ISP treatment (~33 days post immunization), several animals in the Onset group recovered with clinical scores improving to 0.5-1 (limp tail) (Figs. 1B and 1C). In contrast, control animals remained severely paralyzed with scores remaining around 3.5-4. EAE ISP Peak animals also improved significantly with ISP treatment; however, ISP given at the onset of disease allowed for better hind limb recovery (Figs. 1B and 1C). These improvements were also closely correlated with histological improvements. Lesion sizes were especially reduced in Onset treated animals as indicated by LFB myelin staining (Figs. 1D and 1E). Conversely, MBP immunostaining was denser in animals treated with ISP for 41 days compared with control EAE animals (Fig. 1F). Western blotting of MBP protein isoforms also showed restoration of MBP expression in ISP-treated mice (Fig. 1G). Ultrastructural analyses revealed increased myelinated/remyelinated axons in the EAE-lesioned spinal cord in the ISP-treated mice compared to controls (Fig. 1H). Quantitative analysis confirmed an increase of myelinated/remyelinated axons in the ISP-treated group (Fig. 1I) and the G-ratio, which indicates myelination thickness by normalizing the diameter of myelination by axon diameter, was lower in the ISP-treated group compared to the vehicle-treated group (Fig. 1J). Importantly, our results suggest that ISP acted to enhance myelin regeneration rather than prevent demyelination especially since demyelination baselines (LFB) at 18 days following EAE induction were not significantly different between the two groups at this early stage of disease progression (Figs. 12E and 12F).

### ISP treatment is associated with decreased CSPG expression in demyelinated lesions over time as well as an altered inflammatory response in EAE

In addition to observations that CSPGs markedly decreased following ISP treatment of EAE-induced animals (Fig. 4A, Cat301) we also found altered macrophage dynamics in the same animals. To begin, macrophages (Iba1) appeared to colocalize with aggrecan (Cat301) especially in the white matter (Fig. 4A), which may be due to activated macrophages depositing or, more likely, phagocytosing aggrecan, which they are not known to produce. We additionally observed decreased Iba1 immunostaining as well as decreased amounts of aggrecan within Iba1⁺ macrophages in ISP-treated EAE animals compared to controls (Fig. 4A). We performed further quantification of Iba1 and GFAP at 41 days following EAE induction and saw significant decreases in both microglia/macrophages and reactive astrocytes respectively following ISP treatment (Figs. 4A and 4B). A recent reported that modulation of LAR family receptor phosphatases with synthetic peptides, including ISP, skewed microglia/macrophages towards an M2 polarization following spinal cord injury. Indeed, immunostaining for markers identifying M1 (iNOS) and M2 (Arginase-1) macrophage polarization revealed supporting evidence that ISP treatment modulates the inflammatory environment in EAE animals (Figs. 4C and 4D). Of note, while microglia/macrophages seem to produce PTPσ after injury, reactive astrocytes do not.

### ISP enhances the rate of myelin repair in LPC-induced demyelination

We next asked whether ISP modulation of PTPσ-CSPG interactions has similar effects in acute focal demyelination induced by LPC injected into the dorsal column white matter of young adult C57BL6/J mice treated either with ISP (20µg/day, subcutaneous) or control vehicle starting at 1-day post LPC injection. After ISP treatment, LPC-induced lesion volumes were significantly reduced at 14 and 21-days post lesion (dpl) compared with vehicle-treated animals as shown by LFB myelin staining (Figs. 2A and 2B). As indicated in Figs. 2A-2G, vehicle-treated animals had an average lesion volume of 1.508±0.069 mm³, 1.035±0.06mm³ and 0.738±0.027mm³ after 7, 14 or 21dpl, respectively. In contrast, ISP-treated animals showed reduced lesion volume from an average of 1.535±0.058mm³ at 7dpl to 0.613±0.043mm³ at 14dpl (Fig. 2B). By 21dpl, we found extensive lesion repair and reduced lesion volumes in ISP-treated mice (1.535±0.058mm³ to 0.2±0.041mm³) (Fig. 2B). Immunostaining consistently indicated increased MBP expression in LPC-lesions of ISP-treated mice compared with vehicle-treated mice after 14 and 21dpl (Fig. 2C). Quantitative western blot analysis confirmed increased expression of MBP in LPC-lesioned animals after ISP treatment at 14dpl (Fig. 2D). Finally, ultrastructural analysis confirmed the number of remyelinated axons in ISP-treated mice at 14dpl compared to controls (Figs. 2E and 2F). Consistent with these results, quantitative analyses of the G-ratio between ISP- and vehicle-treated mice revealed increased thickness of myelin sheaths in ISP-treated mice at 14dpl (Fig. 2G). These experiments showed that ISP treatment accelerated the rate of myelin repair *in vivo.*

To better visualize the ability of ISP to impact OPCs and subsequent remyelination, we utilized a well-established *ex vivo* model of myelinating mouse cerebellar slice culture derived from postnatal day 8-10 pups treated with 0.1% LPC for 17-18 hours to induce demyelination. We found that naive slice cultures developed abundant myelinated axons as shown by MBP and neurofilament (NF200) colocalization (Fig. 3A, Con). LPC treatment, however, caused profuse demyelination with the production of punctate and disorganized myelin (Fig. 3A, 1 day *in vitro* (div)). After 8div, the demyelinated phenotype was still prominent and remyelination was delayed in treated LPC slices compared with vehicle (Fig. 3A, LPC+Veh, 8div). In contrast, LPC-demyelinated slices treated with ISP for 8div showed increased remyelination (Fig. 3A, LPC+ISP, 8div) compared to vehicle-treatment. Although increased MBP expression was seen in vehicle-treated slices by 14div after LPC treatment, the expression of MBP was still disorganized and failed to colocalize well with axons (Fig. 3A, LPC+Veh, 14div). However, ISP treatment for 14div resulted in abundant remyelinated axons (Figs. 3A and 3B, LPC+ISP, 14div), which was confirmed with western blot analysis and quantification (Fig. 3C). These slice culture experiments confirm that following LPC treatment, ISP enhances the rate of remyelination perhaps by influencing OPCs directly or possibly microglia/macrophages as well.

Interestingly, this ISP enhanced rate of remyelination was correlated with a decrease in aggrecan presence (Cat301) by 8div in our cerebellar slice cultures (Figs. 11A and 11B), although aggrecan expression was similar between ISP and vehicle groups at 4div (Figs. 11A and 11B). By 14div, we observed simultaneous remyelination through MBP staining and decreased CSPG expression in both groups although ISP-treated slices had enhanced MBP expression and greater CSPG decrease.

To further investigate whether CSPGs are affected by ISP treatment, we double immunostained for MBP and CSPGs in LPC-injected animals. Control LPC-lesioned animals showed upregulated levels of GAG-CSPGs (CS56) and aggrecan (Cat301) that was inversely correlated with decreased expression of MBP at 14dpl (Fig. 4B). In contrast, ISP-treated animals showed quicker reduction of CSPGs (CS56 and Cat301) and enhanced myelin expression compared to controls (Figs. 4B and 4D). It is important to note that while myelin repair does normally occur in LPC-lesioned animals, CSPG degradation is much slower than that which occurs after peptide treatment (Supp. Figs. 3A and 3B). Thus, we found that ISP treatment not only enhanced the rate of myelin repair, but was associated with more rapidly decreased CSPG expression over time.

In LPC-injected animals, versican immunostaining was strongest in the penumbra of the lesion where reactive astrocytes (GFAP⁺) were found (Fig. 4C). This pattern of CSPG deposition confirms recently reported findings of increased versican secretion by reactive astrocytes. We also examined inflammatory cells and astrocytes in locally demyelinated LPC lesions and altered the timing of ISP treatment to begin our investigation of the mechanism(s) underlying decreased CSPG expression following ISP treatment. Immediately (rather than a 1 day delay) upon LPC injection, mice received ISP (20 µg day/mouse, subcutaneous) for 7 days. Staining of the lesion epicenter revealed no change in the amount of activated microglia (Iba1), reactive astrocytes (GFAP), or MBP myelin protein expression between ISP-treated animals and control groups at this early stage (Figs. 4C and 4D). This suggests that the extent of LPC-induced injury was initially similar between ISP and control groups. Again, aggrecan staining was colocalized with Iba1⁺ macrophages (Fig. 4C). However, CSPG expression (CS56, Cat301) was significantly reduced after ISP treatment, suggesting that ISP may be involved in the enhanced degradation of CSPGs in demyelinating lesions.

### CSPG reduction by ISP enhances OPC survival, differentiation, and migration

ISP-induced CSPG disinhibition could result in enhanced remyelination by regulating OPC proliferation, survival, differentiation, or migration. To distinguish between these possibilities, we began with quantification of proliferating OPCs by immunostaining with Olig2 and Ki67 antibodies in the LPC lesion at 7dpl. We found that the percentage of Olig2⁺ OPCs was significantly increased in the lesions of ISP-treated mice compared to vehicle-treated mice (Figs. 13A and 13B), but found no differences in Olig2⁺/Ki67⁺ cells between the groups (Fig. 13B). To further examine the effects on OPC proliferation by ISP, we peptide treated cultured OPCs for 2div on a low concentration of laminin (1 µg/mL) and aggrecan (2 µg/mL) and found no significant differences in proliferation rates between treated and control groups (Figs. 13C and 13D). To investigate whether apoptosis of OPCs was affected by CSPGs, we performed TUNEL staining of OPCs also cultured on low concentrations of laminin and aggrecan and found that ISP treatment decreased the percentage of apoptotic cells (Figs. 13E and 13F). ISP also decreased OPC death when similarly cultured OPCs were challenged with LPC (1 µg/mL, 2 hours) (Figs. 13E and 13F).

To examine the effects of ISP treatment on OPC differentiation, we quantified the number of differentiated CC1⁺ oligodendrocytes in both EAE animals treated with ISP (41dp1) and in LPC-injected animals (14dpl). The percentage of CC1⁺ oligodendrocytes was significantly enhanced in lesions of ISP-treated mice compared to controls (Figs. 14A-14D). ISP-enhanced OPC maturation was also confirmed *in vitro* with immunopurified OPCs (P1-2 WT mice) cultured on aggrecan and laminin pre-coated coverslips. Immunostaining of early OPCs (O4⁺) and mature OLs (MBP⁺) showed that CSPGs reduced the progressive maturation of OPCs as seen through reduced process lengths of O4- and MBP-expressing cells grown on CSPGs compared to OPCs grown on non-CSPG control substrates (Figs. 14E and 14F). Process outgrowth and maturation of OPCs grown on CSPGs were largely rescued with ISP treatment (quantified by analyzing MBP⁺ footprints of cells grown on CSPG with or without ISP treatment) (Figs. 14E and 14G). These findings indicate that ISP may be enhancing survival and differentiation, instead of proliferation, of OPCs in demyelinated lesions.

ISP may also be promoting the migration of OPCs into the lesion site where they can survive and subsequently differentiate into their myelinating forms. To explore CSPG/receptor effects on OPC migration, we utilized spinal cord explants derived from P2 WT pups grown on our CSPG gradient spot assay that has been previously used as a potent *in vitro* model of the inhibitory gradient distribution of CSPGs found in glial scars. ISP-treated early (PDGFRα⁺) and pre-mature (O4⁺) OPCs derived from the explant were able to cross the CSPG-enriched outer-rim of the gradient spot. In control explants, few cells were able to migrate across this inhibitory territory (Figs. 5A-5C). Thus, in addition to relieving CSPG-related apoptosis and maturational defects, ISP may also be promoting the migration of OPCs into the lesion site where they can survive and subsequently differentiate into their mature myelinating forms. These observations taken together with the reduction of CSPGs in both slice culture (Figs. 11A and 11B) and *in vivo* models of MS (Figs. 4A-4D) after ISP treatment lead us to hypothesize that targeting PTPσ through ISP induces increased secretion or activation of endogenous proteases.

### ISP treatment enhances protease-dependent enzymatic digestion of CSPGs

In addition to observations of reduced CSPG expression in ISP-treated *ex vivo* and *in vivo* demyelination models, we noticed that ISP-treated PDGFRα⁺ OPCs left "shadows" of possibly digested GAG-CSPG areas where they infiltrated the aggrecan rim of our spot assays (Fig. 5D, arrows). The entire outer proteoglycan rim was also reduced in diameter in the presence of ISP (Fig. 5D, compare rim widths). To begin investigating whether protease activity was occurring, we returned to our spot assay to better characterize putative aggrecan degradation. Conditioned media (CM) was collected from immunopurified OPCs treated with vehicle, ISP, or SISP (scrambled ISP) and plated onto freshly made spots. ISP-treated OPC CM significantly reduced CS56 expression compared to vehicle or scrambled peptide controls as well as a no cell control (Figs. 16A-16C). Interestingly, the laminin portion of the spot was completely spared as visualized by immunostaining (Figs. 16D and 16E). We also confirmed these results with western blot analyses of OPC CM incubated with aggrecan (20µg/mL) and laminin (10µg/mL) collected from OPCs treated with vehicle, ISP, or SISP present (Figs. 5E-5G).

To independently characterize ISP induction of OPC protease activity, we performed a general enzyme activity assay (EnzChek Kit) based on quenched casein fluorescence and found that ISP treatment of OPCs, indeed, increased protease activity (fluorescence A.U.) compared to vehicle and SISP controls (Figs. 5H and 5I). Furthermore, ISP increased aggrecan digestion in a dose-dependent manner as visualized through western blot analysis of ISP treatment of OPC CM incubated with aggrecan (Figs. 16G and 16H).

### ISP increases protease activity and in particular MMP-2 secretion to enhance OPC migration and remyelination

To begin identifying which critical proteases ISP may be regulating, we incubated vehicle or ISP-treated OPC CM with a protease array blot. We found an increased signal for various enzymes belonging to several classes of proteases (e.g., ADAMTS, Kallikreins, Cathepsins, MMPs) in the ISP-treatment group that are potentially (if produced in sufficient amounts) capable of digesting CSPGs (Fig. 15). Interestingly, three laminin degrading proteases such as Cathepsins L and V and MMP-10 were reduced, suggesting some level of specificity in the regulation of the enzyme cascade that is linked with PTPσ modulation. This result may help explain why we have observed unchanged laminin expression in our ISP-treated *in vitro* assays (Figs. 5E, 5G, 16D, and 16E). To confirm the results from our protease array, we performed western blot analyses of multiple upregulated proteases including MMP-2, 9, and Cathepsin B in ISP or control treated OPC CM and found that MMP-2 was readily detectable and clearly enhanced after ISP treatment (Figs. 6C and 6D). Staining of cultured OPCs showed that MMP-2 is expressed in O4⁺ OPCs within their processes and appears to intensify after peptide treatment (Fig. 6H).

To confirm that OPC CM-derived MMP-2 activity is enhanced by ISP treatment, we performed gelatin zymography and found that MMP-2 gelatin-degrading activity was significantly increased upon ISP treatment over controls (Figs. 6A and 6B). MMP-9 activity was barely visible by gelatin zymography (Fig. 6A), and undetected by western blot analysis (data not shown). We also blotted for MMP-10, a protease that degrades fibronectin, laminin, and elastin, and found that it is secreted in far lower amounts than MMP-2 (Fig. 6C). Vehicle, ISP, and SISP-treated OPC cultures seem to secrete MMP-10 in equally low amounts (Figs. 6C and 6E), suggesting that enhanced MMP-2 secretion by ISP may be specific to PTPσ modulation. In addition to ISP-treated OPC CM, OPC lysates were also analyzed by western blot and showed a decrease in MMP-2 expression normalized over GAPDH loading control suggesting that MMP-2 secretion may be enhanced by ISP (Fig. 6F). To test this, we incubated aggrecan with OPC CM treated with an exocytosis inhibitor, Exo1 (10µg/mL), in conjunction with ISP. At sufficient concentrations, Exo1 has been observed to reversibly inhibit exocytosis through its inhibition of the Arf GTPase. We found that Exo1 partially rescued aggrecan GAG digestion (Fig. 6I and 6J). We also performed the same experiment with a broad MMP inhibitor, GM6001 (25 µM), and a specific MMP-2 inhibitor (OA-Hy, Calbiochem, 100nM) with ISP and found that GAG digestion was partially rescued in both cases indicating that ISP-induced CSPG degradation may very well be perpetrated by the metalloprotease family and MMP-2 predominantly (Figs. 6I and 6J). GM6001 and an MMP-2 inhibitor additionally rescued CS56 spot degradation (Figs. 17A-17D).

We returned to the spot assay to test whether MMP inhibition decreases OPC migration across the CSPG rim. Treatment of OPCs with GM6001 (25 µM) and the specific MMP-2 inhibitor (OA-Hy, 100nM) effectively halted OPC entry into the CSPG-rich area even in the presence of ISP (Figs. 7A and 7B). This suggests that ISP-induction of enhanced OPC migration may be dependent on MMPs. Finally, to test the functional necessity of MMPs on remyelination, we treated LPC-demyelinated cerebellar slices with GM6001 or the specific MMP-2 inhibitor in conjunction with ISP. Colocalization of MBP and neurofilament⁺ axons was, indeed, decreased with GM6001 and the MMP-2 inhibitor treatment despite the presence of ISP (Figs. 7C and 7D). Furthermore, 2div inhibition of MMP-2 in LPC-challenged (1 µg/mL, 2 hours) OPCs cultured on low concentrations of aggrecan and laminin ablated the survival-promoting effects of ISP as assessed through TUNEL staining (Fig. 17E). The MMP-2 inhibitor, however, did not seem to increase apoptosis compared to vehicle control on aggrecan/laminin alone (Fig. 17E). Specific Inhibition of MMP-2 additionally negated gains in MBP footprints of mature oligodendrocytes grown on low concentrations of aggrecan/laminin (Figs. 17F and 17G).

To further elucidate the necessity of MMP-2 activity following ISP treatment in enhancing remyelination, we utilized a lentiviral particle-delivered shRNA construct. We first validated this shRNA approach using lentiviral delivery as well as western analysis to knock down MMP-2 in OPC cultures infected for 48 hours (Fig. 18A). shRNA knock down of MMP-2 was able to reduce the area of extended MBP⁺ processes of OLs cultured on aggrecan *in vitro* (Figs. 18B and 18C) and the ability of OPCs to migrate past a high aggrecan barrier (Figs. 18D and 18E) despite ISP treatment. As expected, shRNA knock down of MMP-2 also attenuated ISP-induced remyelination in cerebellar slice cultures (Figs. 7E and 7F).

We next characterized MMP-2 expression *in vivo* using immunohistochemistry and found that while the dorsal column of the naive spinal cord expressed a baseline of some MMP-2 protein, LPC injection into the same area somewhat increased MMP-2 expression at 14dpl (Figs. 8A and 8B). However, ISP treatment markedly enhanced MMP-2 expression in the LPC-injected site, which was also confirmed with western blot analysis of the affected spinal cord areas (Fig. 8C). Immunostaining of ISP-treated LPC demyelinated cords showed MMP-2 colocalizing with Olig2-identified OPCs (Fig. 8D), but also with Iba1-labeled immune cells (Fig. 8E). To explore further the necessity of MMP-2 activity in ISP-enhanced remyelination, we returned to the LPC model and analyzed lesion volume following myelin staining at 18dpl (Figs. 8F and 8G) with an MMP-2 inhibitor (OA-Hy) or shRNA construct targeting MMP-2 delivered with lentiviral particles. Interestingly, pharmacological inhibition of MMP-2 increased the lesion volume over vehicle control suggesting that baseline levels of MMP-2 may be facilitating slow remyelination in this model. The addition of MMP-2 pharmacological inhibition or MMP-2 shRNA-mediated knock down attenuated the enhanced remyelinating effects of ISP which correlated with a concomitant increase in CS56 immunoreactivity (Figs. 19A and 19B), and decreased accumulation of Olig2⁺ OPCs in the lesion epicenter (Figs. 19C and 19D). Together, these results indicate the importance of PTPσ-regulated MMP-2 secretion by OPCs, but also possibly microglia, to assist them in their migration and ability to remyelinate despite high CSPG deposition after focal demyelinating injury.

### Discussion

We have elucidated a critical role for the CSPG receptor PTPσ following its modulation in the restoration of OPC homeostasis in a variety of MS models where proteoglycan deposition during lesion associated scar formation potently inhibits OPC migration, differentiation, and remyelination. As described herein, targeting PTPσ with a systemically delivered peptide enhances the rate of myelin repair in LPC-induced lesions and stimulates robust myelin regeneration and functional recovery following chronic demyelinating EAE. These data show a novel linkage between PTPσ modulation with altered immune polarization and enhanced protease activity. This underscores the important role that CSPGs play following CNS demyelinating diseases and identifies a strategy that can target MS lesions broadly throughout the neuraxis to relieve CSPG mediated inhibition.

The downstream mechanisms following peptide modulation of the receptor PTPσ that allow cells to overcome CSPG inhibition are largely unknown. Protease activity is heavily regulated at several levels including transcription, translation, secretion, localization, activation, and inhibition to prevent unfettered, potentially damaging activity. Left uncontrolled, proteases are able to degrade a broad range of proteins with potentially disastrous outcomes as seen in "protease storms" following a variety of CNS injuries. In the relapsing phase of MS, nonspecific protease up-regulation associated with rampant inflammation and myelin degeneration have been well characterized. However, the beneficial effects of finely regulated protease secretion following injury to promote tissue repair are becoming more appreciated. Here, we present a novel finding linking PTPσ modulation with enhanced MMP-2 protease activity by OPCs, which aids in their digestion through CSPG-laden demyelinated plaque that envelops the MS-like lesion. Through *in vitro, ex vivo* and *in vivo* assays, we have (in part) identified the necessity of MMP-2 activity through PTPσ modulation not only for OPC migration but also for improved OPC survival, maturation, and remyelination. MMP-2 upregulation has been previously identified to allow stem cells to invade CSPG-containing regions of the glial scar and improve remyelination of peripheral axons by Schwann cells in culture. We observed laminin sparing and concomitant CSPG degradation with ISP treatment, which may be one mechanism by which OPCs are able to infiltrate into and survive otherwise CSPG-dense demyelinated lesions. This highlights the precise regulation of proteases by OPCs, and possibly immune cells, through PTPσ to promote controlled CSPG degradation.

In addition, our study confirms this modulation of macrophages in a peptide treated EAE model of MS where we observed decreases in CSPG load as well as the destructive M1 macrophage phenotype marker iNOS and increases in M2 associated Arginase-1.

Our study suggests that ISP may be markedly enhancing microglial phagocytic capacity. By modulating the cognate receptor of CSPGs, OPCs and microglia may be working together to more rapidly clear inhibitory CSPGs and other cellular remnants. Thus, in altering the pro-inflammatory environment toward an M2 state and inducing focal protease activity by selective cells, ISP may provide additional CSPG disinhibition, culminating in myelin regeneration.

## Claims

1. A therapeutic agent for use in a method of enhancing oligodendrocyte progenitor cell (OPC) migration, differentiation, proliferation and/or maturation in a subject in need thereof, the therapeutic agent comprising a therapeutic peptide, the therapeutic peptide having an amino acid sequence that has at least about 65% identity to the amino acid sequence of SEQ ID NO:32 or SEQ ID NO:33, wherein the subject has or is at risk of a myelin related disorder.

2. The therapeutic agent for use of claim 1, wherein the amino acid sequence has at least about 70%, at least about 75%, at least about 80%, at least about 85%, at least about 90%, or at least about 95% identity to the amino acid sequence of SEQ ID NO:32 or SEQ ID NO:33.

3. The therapeutic agent for use of claim 2, wherein the therapeutic peptide comprises a conservative substitution of at least one of residue 4, 5, 6, 7, 9, 10, 12, or 13 of SEQ ID NO: 32.

4. The therapeutic agent for use of claim 3, wherein the amino acid residue 4E is substituted with D or Q, the amino acid residue 5R is substituted with H, L, or K, the amino acid residue 6L is substituted with I, V, or M, the amino acid residue 7K is substituted with R or H, the amino acid residue 9N is substituted with E or D, the amino acid residue 10D is substituted with E or N, the amino acid residue 12L is substituted with I, V, or M, and/or the amino acid residue 13K is substituted with R or H.

5. The therapeutic agent for use of claim 2, wherein the therapeutic peptide comprises a conservative substitution of at least one of residue 7, 8, 9, 10, 12, or 13 of SEQ ID NO:33.

6. The therapeutic agent for use of claim 5, wherein the amino acid residue 7E is substituted with D or Q, the amino acid residue 8R is substituted with H, L, or K, the amino acid residue 9L is substituted with I, V, or M, the amino acid residue 10K is substituted with R or H, the amino acid residue 12N is substituted with E or D, and/or the amino acid residue 13D is substituted with E or N.

7. The therapeutic agent for use of claim 1, the therapeutic peptide comprising an amino acid sequence selected from the group consisting of SEQ ID NOs:1-25, 32 and 33.

8. The therapeutic agent for use of any one of claims 1-7, wherein the therapeutic agent comprises a transport moiety that is linked to the therapeutic peptide and facilitates uptake of the therapeutic peptide by the OPC.

9. The therapeutic agent for use of claim 8, wherein the transport moiety is an HIV Tat transport moiety.

10. The therapeutic agent for use of claim 8 or claim 9, wherein the transport moiety is linked to the therapeutic peptide by a peptide linker

11. The therapeutic agent for use of any one of claims 8 to 10, wherein the therapeutic agent comprises an amino acid sequence selected from the group consisting of SEQ ID NOs:34-60.

12. The therapeutic agent for use of any one of claims 1-11, wherein the therapeutic agent is formulated for systemic administration to the subject being treated.

13. The therapeutic agent for use of claim 1, wherein the myelin related disorder comprises at least one of myelinoclastic disorders, leukodystrophic disorders or demyelinating diseases of the peripheral nervous system.

14. The therapeutic agent for use of claim 13, wherein the myelin related disorder is multiple sclerosis.

## Patentansprüche

1. Ein therapeutisches Mittel zur Verwendung in einem Verfahren zur Steigerung der Migration, Differenzierung, Proliferation und/oder Reifung von Oligodendrozyten-Vorläuferzellen (OPC) bei einem Subjekt, das dies benötigt, wobei das therapeutische Mittel ein therapeutisches Peptid umfasst, wobei das therapeutische Peptid eine Aminosäuresequenz aufweist, die mindestens etwa 65 % Identität mit der Aminosäuresequenz von SEQ ID NO:32 oder SEQ ID NO:33 aufweist, wobei das Subjekt an einer Myelin-bedingten Störung leidet oder das Risiko einer Myelin-bedingten Störung besteht.

2. Das therapeutische Mittel zur Verwendung nach Anspruch 1, wobei die Aminosäuresequenz mindestens etwa 70 %, mindestens etwa 75 %, mindestens etwa 80 %, mindestens etwa 85 %, mindestens etwa 90 % oder mindestens etwa 95 % Identität mit der Aminosäuresequenz von SEQ ID NO:32 oder SEQ ID NO:33 aufweist.

3. Das therapeutische Mittel zur Verwendung nach Anspruch 2, wobei das therapeutische Peptid eine konservative Substitution von mindestens einem der Reste 4, 5, 6, 7, 9, 10, 12 oder 13 von SEQ ID NO: 32 umfasst.

4. Das therapeutische Mittel zur Verwendung nach Anspruch 3, wobei der Aminosäurerest 4E durch D oder Q substituiert ist, der Aminosäurerest 5R durch H, L oder K substituiert ist, der Aminosäurerest 6L durch I, V oder M substituiert ist, der Aminosäurerest 7K durch R oder H substituiert ist, der Aminosäurerest 9N durch E oder D substituiert ist, der Aminosäurerest 10D durch E oder N substituiert ist, der Aminosäurerest 12L durch I, V oder M substituiert ist und/oder der Aminosäurerest 13K durch R oder H substituiert ist.

5. Das therapeutische Mittel zur Verwendung nach Anspruch 2, wobei das therapeutische Peptid eine konservative Substitution von mindestens einem der Reste 7, 8, 9, 10, 12 oder 13 von SEQ ID NO:33 umfasst.

6. Das therapeutische Mittel zur Verwendung nach Anspruch 5, wobei der Aminosäurerest 7E durch D oder Q substituiert ist, der Aminosäurerest 8R durch H, L oder K substituiert ist, der Aminosäurerest 9L durch I, V oder M substituiert ist, der Aminosäurerest 10K durch R oder H substituiert ist, der Aminosäurerest 12N durch E oder D substituiert ist und/oder der Aminosäurerest 13D durch E oder N substituiert ist.

7. Das therapeutische Mittel zur Verwendung nach Anspruch 1, wobei das therapeutische Peptid eine Aminosäuresequenz umfasst, die aus der Gruppe ausgewählt ist, die aus den SEQ ID NOs: 1-25, 32 und 33 besteht.

8. Das therapeutische Mittel zur Verwendung nach einem der Ansprüche 1 bis 7, wobei das therapeutische Mittel eine Transporteinheit umfasst, die mit dem therapeutischen Peptid verknüpft ist und die Aufnahme des therapeutischen Peptids durch die OPC erleichtert.

9. Das therapeutische Mittel zur Verwendung nach Anspruch 8, wobei die Transporteinheit eine HIV-Tat-Transporteinheit ist.

10. Das therapeutische Mittel zur Verwendung nach Anspruch 8 oder Anspruch 9, wobei die Transporteinheit durch einen Peptidlinker mit dem therapeutischen Peptid verknüpft ist.

11. Das therapeutische Mittel zur Verwendung nach einem der Ansprüche 8 bis 10, wobei das therapeutische Mittel eine Aminosäuresequenz umfasst, die aus der Gruppe ausgewählt ist, die aus den SEQ ID NOs: 34-60 besteht.

12. Das therapeutische Mittel zur Verwendung nach einem der Ansprüche 1 bis 11, wobei das therapeutische Mittel zur systemischen Verabreichung an das zu behandelnde Subjekt formuliert ist.

13. Das therapeutische Mittel zur Verwendung nach Anspruch 1, wobei die Myelinbedingte Störung mindestens eine der folgenden umfasst: myelinoklastische Störungen, leukodystrophische Störungen oder demyelinisierende Erkrankungen des peripheren Nervensystems.

14. Das therapeutische Mittel zur Verwendung nach Anspruch 13, wobei die Myelinbedingte Störung Multiple Sklerose ist.

## Revendications

1. Agent thérapeutique pour une utilisation dans un procédé d'amélioration de la migration, de la différenciation, de la prolifération et/ou de la maturation de cellules progénitrices d'oligodendrocytes (OPC) chez un sujet en ayant besoin, l'agent thérapeutique comprenant un peptide thérapeutique, le peptide thérapeutique ayant une séquence d'acides aminés présentant au moins environ 65 % d'identité avec la séquence d'acides aminés de SEQ ID NO : 32 ou SEQ ID NO : 33, le sujet étant atteint d'un trouble lié à la myéline ou étant à risque de développer un tel trouble.

2. Agent thérapeutique pour une utilisation selon la revendication 1, dans lequel la séquence d'acides aminés présente au moins environ 70 %, au moins environ 75 %, au moins environ 80 %, au moins environ 85 %, au moins environ 90 %, ou au moins environ 95 % d'identité avec la séquence d'acides aminés de SEQ ID NO : 32 ou SEQ ID NO : 33.

3. Agent thérapeutique pour une utilisation selon la revendication 2, dans lequel le peptide thérapeutique comprend une substitution conservatrice d'au moins l'un des résidus 4, 5, 6, 7, 9, 10, 12 ou 13 de SEQ ID NO : 32.

4. Agent thérapeutique pour une utilisation selon la revendication 3, dans lequel le résidu d'acide aminé 4E est substitué par D ou Q, le résidu d'acide aminé 5R est substitué par H, L ou K, le résidu d'acide aminé 6L est substitué par I, V ou M, le résidu d'acide aminé 7K est substitué par R ou H, le résidu d'acide aminé 9N est substitué par E ou D, le résidu d'acide aminé 10D est substitué par E ou N, le résidu d'acide aminé 12L est substitué par I, V ou M et/ou le résidu d'acide aminé 13K est substitué par R ou H.

5. Agent thérapeutique pour une utilisation selon la revendication 2, dans lequel le peptide thérapeutique comprend une substitution conservatrice d'au moins l'un des résidus 7, 8, 9, 10, 12 ou 13 de SEQ ID NO : 33.

6. Agent thérapeutique pour une utilisation selon la revendication 5, dans lequel le résidu d'acide aminé 7E est substitué par D ou Q, le résidu d'acide aminé 8R est substitué par H, L, ou K, le résidu d'acide aminé 9L est substitué par I, V, ou M, le résidu d'acide aminé 10K est substitué par R ou H, le résidu d'acide aminé 12N est substitué par E ou D et/ou le résidu d'acide aminé 13D est substitué par E ou N.

7. Agent thérapeutique pour une utilisation selon la revendication 1, le peptide thérapeutique comprenant une séquence d'acides aminés sélectionnée dans le groupe constituant en SEQ ID NO : 1-25, 32 et 33.

8. Agent thérapeutique pour une utilisation selon l'une des revendications 1 à 7, dans lequel l'agent thérapeutique comprend une fraction de transport qui est liée au peptide thérapeutique et facilite l'absorption du peptide thérapeutique par l'OPC.

9. Agent thérapeutique pour une utilisation selon la revendication 8, dans lequel la fraction de transport est une fraction de transport de Tat du VIH.

10. Agent thérapeutique pour une utilisation selon la revendication 8 ou la revendication 9, dans lequel la fraction de transport est liée au peptide thérapeutique par un liant peptidique

11. Agent thérapeutique pour une utilisation selon l'une des revendications 8 à 10, dans lequel l'agent thérapeutique comprend une séquence d'acides aminés sélectionnée dans le groupe consistant en SEQ ID NO : 34-60.

12. Agent thérapeutique pour une utilisation selon l'une des revendications 1 à 11, dans lequel l'agent thérapeutique est formulé pour une administration systémique au sujet traité.

13. Agent thérapeutique pour une utilisation selon la revendication 1, dans lequel le trouble lié à la myéline comprend au moins un élément parmi des troubles myélinoclastiques, des troubles leucodystrophiques ou des maladies démyélinisantes du système nerveux périphérique.

14. Agent thérapeutique pour une utilisation selon la revendication 13, dans lequel le trouble lié à la myéline est la sclérose en plaques.
